# EUROPEAN PATENT APPLICATION

(11) **EP 4 610 262 A1**
(43) Date of publication of application: **03.09.2025**
(21) Application number: 23881767.0
(22) Date of filing: 23.10.2023
(51) Int. Cl.: C07D 487/08, C07D 513/08, C07D 515/08, C07D 513/18, C07D 498/08, A61K 31/529, A61P 35/00

(54) **MACROCYCLIC COMPOUND AS CDK9 INHIBITOR AND USE THEREOF**

(30) Priority: 24.10.2022 CN 202211315138
(71) Applicant: Artivila Biopharma, Wuxi, Jiangsu 214000 (CN)
(72) Inventor: NIU, Deqiang, Wuxi, Jiangsu 214000 (CN); ZHU, Zhendong, Wuxi, Jiangsu 214000 (CN); FAN, Ming, Wuxi, Jiangsu 214000 (CN)
(74) Representative: Boult Wade Tennant LLP
(86) International application number: PCT/CN2023/125860
(87) International publication number: WO 2024/088189

(57) **Abstract**

The present disclosure provides a compound represented by formula (I), a pharmaceutical composition thereof, and a use thereof in treating and/or preventing diseases or disorders related to CDK9 kinase activity.

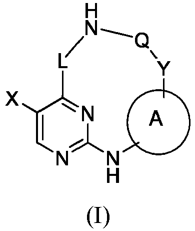

## Description

### CROSS REFERENCE TO RELATED APPLICATIONS

This application claims the priority of Chinese Patent Application No. 202211315138.1, filed with the China National Intellectual Property Administration on October 24, 2022, and titled with "MACROCYCLIC COMPOUND AS CDK9 INHIBITOR AND USE THEREOF", which is hereby incorporated by reference in its entirety.

### FIELD

The present application relates to compounds for use in inhibiting CDK9 kinase activity, and use thereof in treating and/or preventing a disease or condition associated with dysregulated CDK9 kinase activity.

### BACKGROUND

Protein kinases are enzyme proteins widely present inside cells and on the cell surface. So far, nearly 600 protein kinases have been discovered and identified, which belong to a structurally related protein family, and the known members are related to almost all cell signal transduction activities. The catalytic function of protein kinase is to transfer the γ-phosphate group in the ATP molecule to specific threonine, serine or tyrosine group of the target protein, so that the conformation of the target protein changes, resulting in the change of the target protein functions from a static state to an activated state. According to the specificity of the target amino acids of protein kinases, protein kinases are divided into two major categories: serine/threonine protein kinases and tyrosine protein kinases.

The signal transduction and regulation involving protein kinases play an extremely important role in the normal function of cells and organs, including cell growth, differentiation, proliferation, angiogenesis, apoptosis, cytoskeletal arrangement, regulation of metabolic reactions, membrane transport and cell movement etc. In addition, the non-catalytic functions of protein kinases also play an indispensable role, including allosteric effects, subcellular targeting, protein complex scaffolds, competitive protein interactions and DNA binding. On the other hand, when gene mutations or protein kinase overexpression occur, dysregulated protein kinases can lead to a variety of pathological changes, including cancer, inflammation, autoimmune diseases, cardiovascular and neurological diseases. Therefore, protein kinases have become one of the most important targets in drug development today, and the success of protein kinase inhibitors in clinical treatment in recent years has further demonstrated the feasibility of this strategy and revealed the good prospects of using protein kinases as therapeutic targets.

Cyclin-dependent kinases (CDKs), belonging to serine/threonine kinases, are protein kinases that regulate cell cycle and gene transcription. At different stages of the cell cycle, CDKs phosphorylate specific cyclins to regulate cell activities. Dysregulation of CDKs has a significant impact on the normal cell state and is often identified as a carcinogenic factor. CDK9 is one of the most important CDKs, which can cooperate with four cyclins, including cyclin T1, cyclin K, cyclin T2a and cyclin T2b. CDK9 binds to cyclins to form a heterodimer of positive transcription elongation factor b (P-TEFb). P-TEFb, a core molecule for transcription elongation, phosphorylates the C-terminal domain (CTD) of RNA polymerase II, allowing transcription to extend from the start site. Dysregulation of CDK9 kinase activity can lead to a variety of diseases, including highly proliferative diseases (such as cancer), viral infectious diseases, or cardiovascular diseases. CDK9 inhibitors can inhibit the transcription process. Since the transcription process is dysregulated in many cancers, CDK9 inhibitors can be used to treat cancers, including solid tumors and hematological malignant tumors, such as acute myeloid leukemia (AML), breast cancer, prostate cancer, hepatocellular carcinoma, pancreatic cancer and other solid tumors.

Given the key role of CDK9 in disease, downregulating CDK9 provides an excellent opportunity for the development of targeted therapies for cancer and other diseases. Although many small molecule CDK inhibitors have been reported, most of them lack selectivity for specific CDKs, and thus show low efficacy and high adverse event rates in clinical practice. Therefore, the development of CDK9-specific targeted inhibitors is crucial for the treatment of human diseases.

### SUMMARY

After long-term research, the inventors unexpectedly found a group of compounds with significant inhibitory effect on CDK9 activity, which show highly efficient and selective inhibitory effect on CDK9 kinase, and can be used to treat or prevent diseases related to CDK9 activity.

In particular, the present disclosure provides a compound represented by formula (I) as an inhibitor of CDK9 kinase activity: 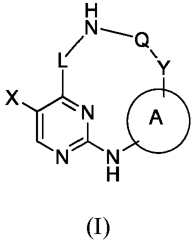
or a mesomer, a racemate, an enantiomer, a diastereoisomer or a pharmaceutically acceptable salt thereof,
wherein,
X is selected from the group consisting of H, C₁-C₃ alkyl, halogen, CF₃, carbamoyl, cyano and C₁-C₃ alkoxycarbonyl;
L is selected from the group consisting of a single bond (i.e., a direct connection by a covalent bond), C₆-C₁₀ aryl, 5-12 membered heteroaryl, C₃-C₈ cycloalkyl and 3-8 membered heterocyclyl, wherein the C₆-C₁₀ aryl, 5-12 membered heteroaryl, C₃-C₈ cycloalkyl and 3-8 membered heterocyclyl are optionally further substituted by one or more R¹;
ring A is selected from the group consisting of C₆-C₁₀ aryl, 5-12 membered heteroaryl, C₃-C₈ cycloalkyl and 3-8 membered heterocyclyl, wherein the C₆-C₁₀ aryl, 5-12 membered heteroaryl, C₃-C₈ cycloalkyl and 3-8 membered heterocyclyl are optionally further substituted by one or more R²;
Y is selected from the group consisting of a single bond and -CH₂NR³-, wherein R³ is selected from the group consisting of H, C₁-C₃ alkyl, C₁-C₃ alkyl acyl and Boc;
wherein at least one of L and Y is a single bond;
Q is selected from the group consisting of C₄-C₅ alkylene and C₄-C₅ alkenylene, wherein one -CH₂- unit in the above groups is optionally replaced by an O atom, a S atom or -NR³-; preferably, Q is selected from the group consisting of: 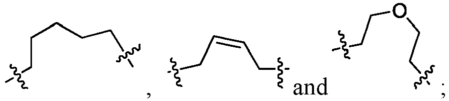
R¹ and R² are each independently selected from the group consisting of hydrogen, halogen, amino, C₁-C₃ alkylamino, cyano, oxo, hydroxyl, C₁-C₃ alkyl, C₁-C₃ alkoxy, 3-8 membered heterocyclyl and 3-8 membered heterocyclyl substituted by C₁-C₃ alkyl.

In a preferred embodiment, the compound represented by formula (I) or the mesomer, racemate, enantiomer, diastereomer or pharmaceutically acceptable salt thereof according to the present disclosure is a compound represented by formula (II) or a mesomer, a racemate, an enantiomer, a diastereomer or a pharmaceutically acceptable salt thereof: 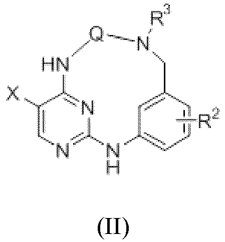 wherein,
X, Q, R² and R³ are as defined above.

In another preferred embodiment, the compound represented by formula (I) or the mesomer, racemate, enantiomer, diastereomer or pharmaceutically acceptable salt thereof according to the present disclosure is a compound represented by formula (III) or a mesomer, a racemate, an enantiomer, a diastereomer or a pharmaceutically acceptable salt thereof: 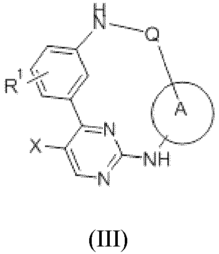 wherein,
X, Q, A and R¹ are as defined above.

In another preferred embodiment, in the compound represented by formula (III) according to the present disclosure, ring A is selected from the group consisting of a benzene ring and a pyrazole ring.

Typical compounds of the present disclosure include but are not limited to the following compounds: 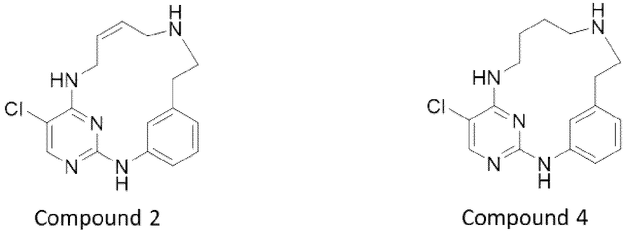 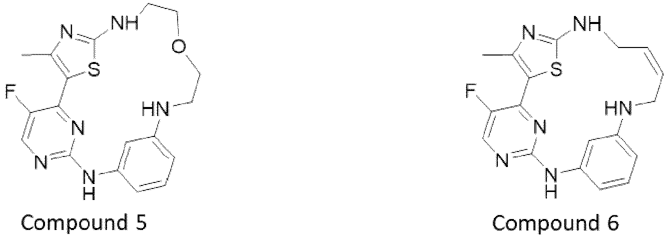 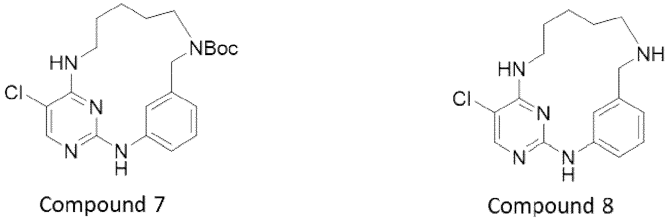 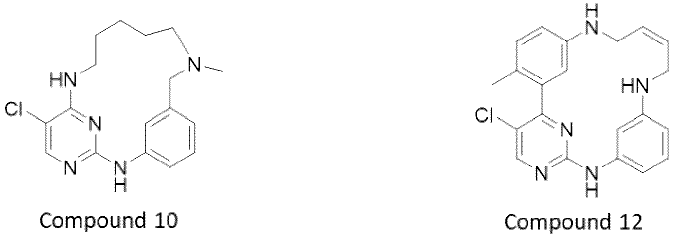 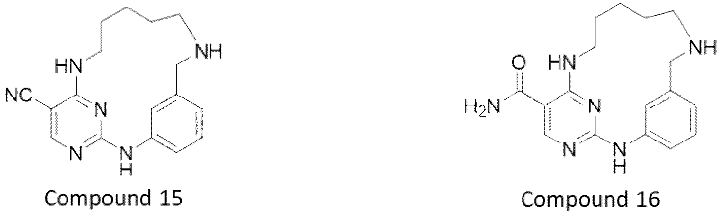 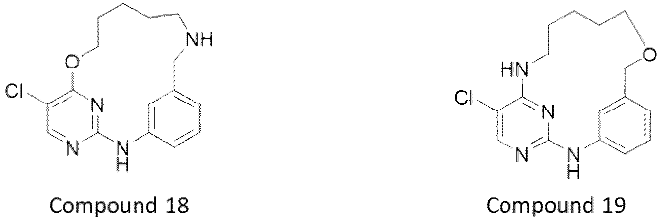 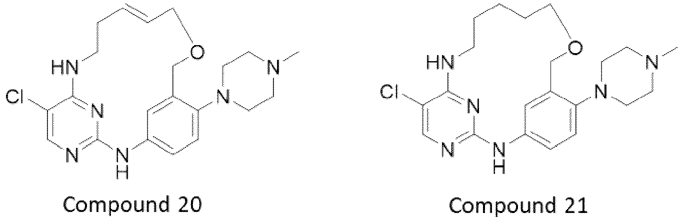 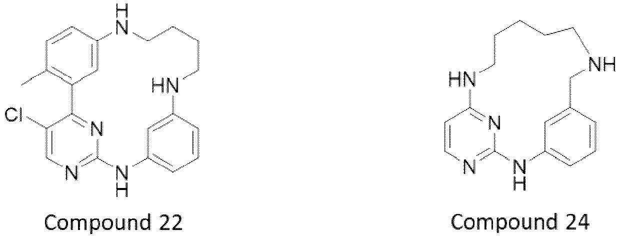 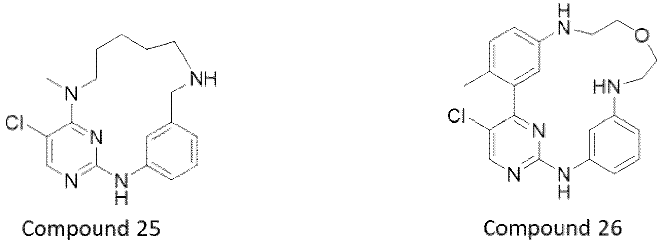 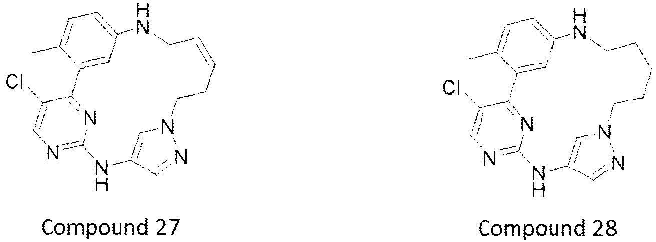 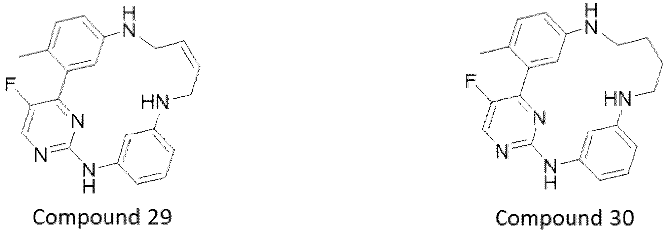 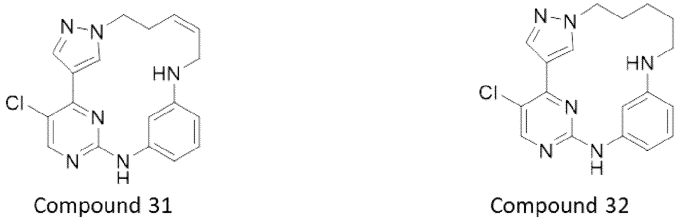 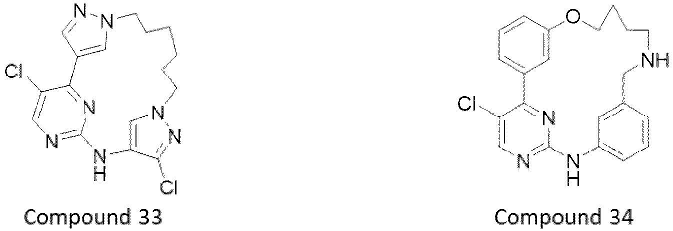 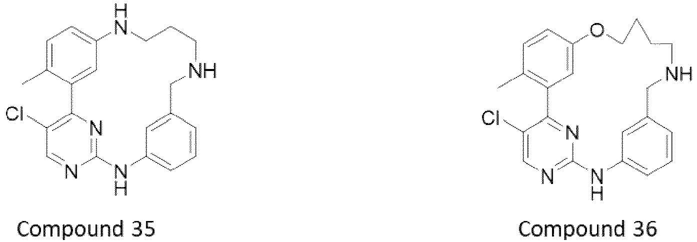 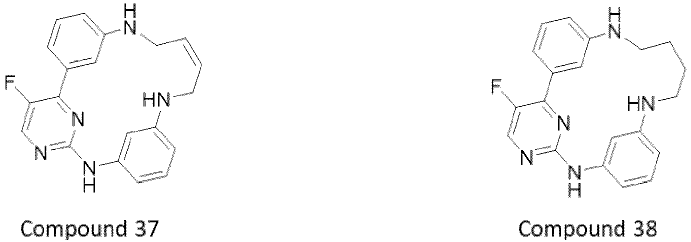 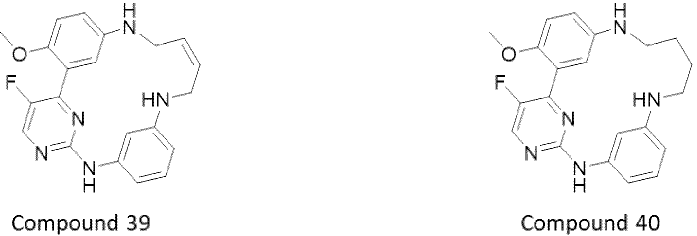 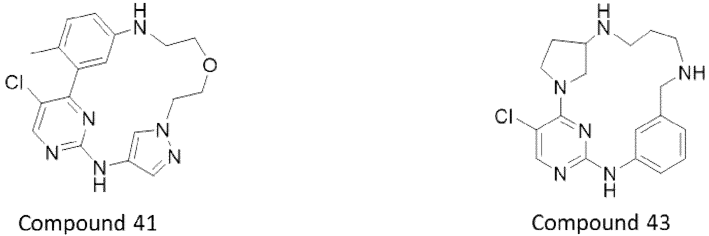 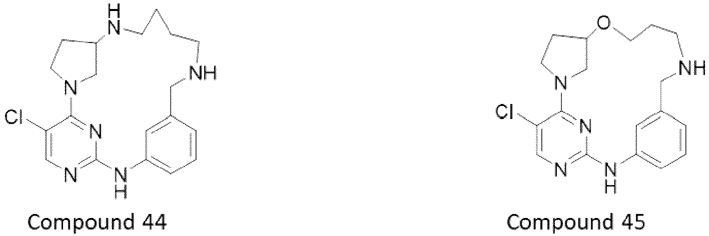 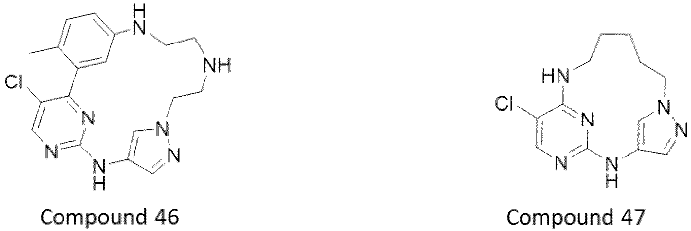 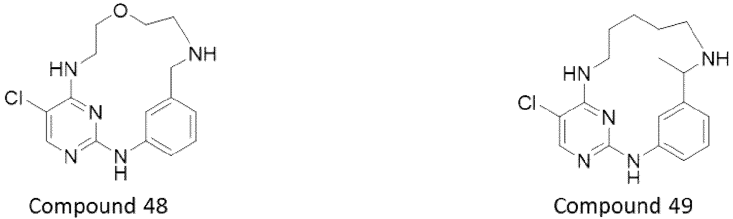 or a mesomer, a racemate, an enantiomer, a diastereomer or a pharmaceutically acceptable salt thereof.

The present disclosure further provides a method for producing the compounds represented by formula (I), (II) and (III) according to the present disclosure or the mesomers, racemates, enantiomers, diastereomers or pharmaceutically acceptable salts thereof, comprising steps of: 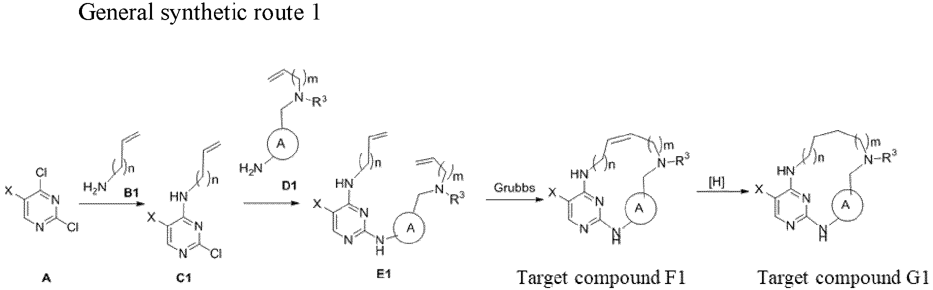 wherein X, R³, and A are as defined above, and n and m are each 1 or 2.

Compound A and compound B1 undergo a nucleophilic substitution reaction to obtain compound C1; compound C1 and compound D1 undergo a Buchwald-Hartwig coupling reaction under the catalysis of palladium to obtain compound E1; compound E1 undergoes an intramolecular olefin ring-closing metathesis (RCM) reaction in the presence of a Grubbs catalyst and cyclization to obtain the target compound F1; and the double bond in compound F1 is reduced by hydrogenation to generate the target compound G1. 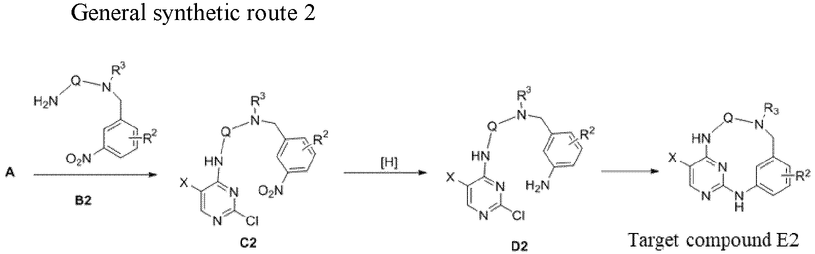 wherein X, Q, R² and R³ are as defined above.

Compound A and compound B2 undergo a nucleophilic substitution reaction to obtain compound C2; the nitro group in compound C2 is reduced to amino group in the presence of a reducing agent to obtain compound D2; and compound D2 undergoes an intramolecular C-N coupling reaction under the catalysis of palladium and cyclization to obtain the target compound E2. 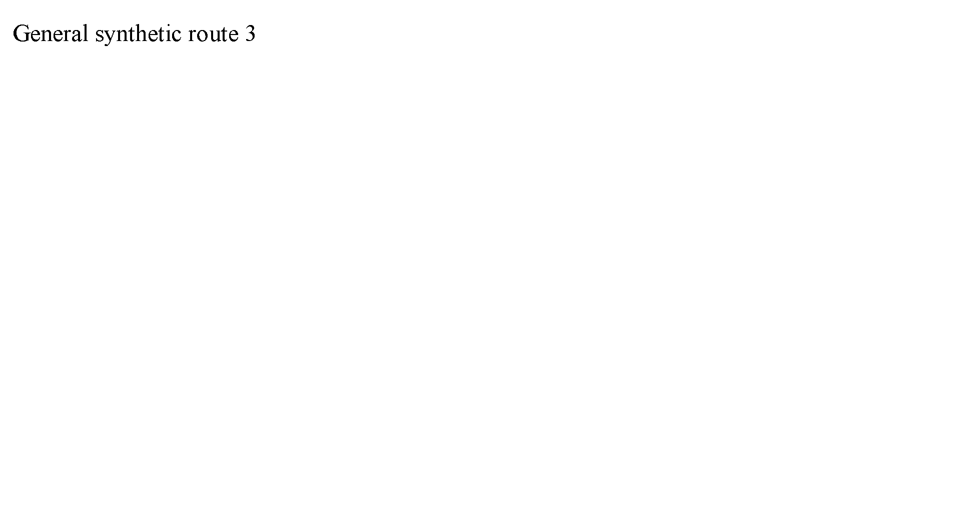 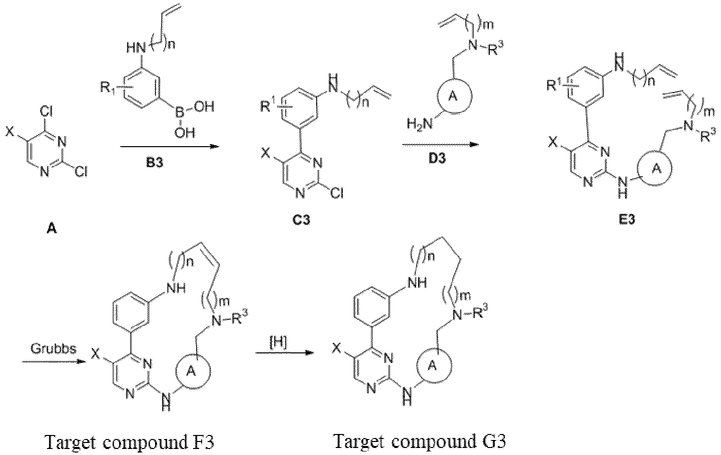 wherein, X, R¹, R³ and A are as defined above, and n and m are each 1 or 2.

Compound A and compound B3 undergo a Suzuki coupling reaction to obtain compound C3; compound C3 and compound D3 undergo a Buchwald-Hartwig coupling reaction under the catalysis of palladium to obtain compound E3; compound E3 undergoes an intramolecular olefin ring-closing metathesis (RCM) reaction in the presence of a Grubbs catalyst and cyclization to obtain the target compound F3; and compound F3 is reduced by hydrogenation to generate a single bond to obtain the target compound G3. 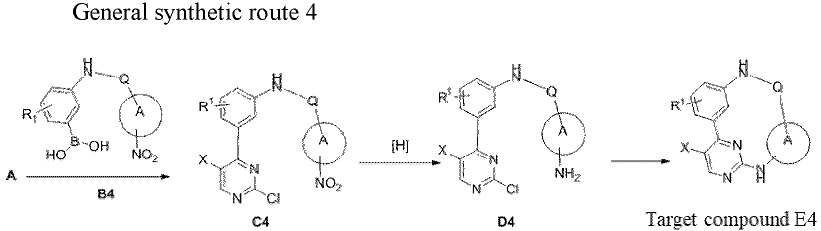 wherein, X, R¹, Q and A are as defined above.

Compound A and compound B4 undergo a Suzuki coupling reaction to obtain compound C4; the nitro group in compound C4 is reduced to an amino group in the presence of a reducing agent to obtain compound D4; and compound D4 undergoes an intramolecular C-N coupling reaction and cyclization to obtain the target compound E4.

### Description of terms

Unless otherwise stated, the terms used in the specification and claims have the following meanings.

**In** the present disclosure, when referring to a "compound" having a specific structural formula, it generally further includes the pharmaceutically acceptable salts, stereoisomers, diastereomers, enantiomers, racemic mixtures and isotopic derivatives thereof.

It is well known to those skilled in the art that in addition to salts of compounds, solvates and hydrates of compounds are alternative forms of the compounds, which can be converted into the compounds under certain conditions. Therefore, when referring to a compound in the present disclosure, it generally further includes the solvates and hydrates thereof.

The "pharmaceutically acceptable salt" used herein referred to a salt of the compound of the present disclosure, which is suitable for contacting with human and mammalian tissues within the scope of reasonable medical judgment without undue toxicity, irritation, allergic reaction, etc., and has appropriate biological activity, thereby having a reasonable benefit/risk ratio. The salt can be produced in situ during the final separation and purification of the compound of the present disclosure, or produced separately by reacting a free base or a free acid with a suitable reagent. For example, a free base can react with a suitable acid. Examples of pharmaceutically acceptable acid addition salts are salts formed by an amino group (amine group) with an inorganic acid (e.g., hydrochloric acid, hydrobromic acid, phosphoric acid, sulfuric acid and perchloric acid) or an organic acid (e.g., acetic acid, oxalic acid, maleic acid, tartaric acid, citric acid, succinic acid or malonic acid), or by using other methods known in the art such as ion exchange. The pharmaceutically acceptable salts of the present disclosure can be produced by conventional methods, for example, by dissolving the compound of the present disclosure in an organic solvent miscible with water (e.g., methanol, ethanol, acetone, and acetonitrile), adding an excessive amount of an aqueous solution of an organic acid or an inorganic acid thereto, thereby precipitating the salt from the resulting mixture, removing the solvent and the remaining free acid therefrom, and then isolating the precipitated salt. Other pharmaceutically acceptable salts include sodium alginate, ascorbate, benzenesulfonate, adipate, camphorsulfonate, aspartate, benzoate, bisulfate, borate, butyrate, camphorate, citrate, dodecylsulfate, ethanesulfonate, formate, fumarate, glucoheptonate, glycerophosphate, gluconate, heptanoate, hexanoate, hydroiodide, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate, etc.

In the specification and claims, a given chemical formula or name shall encompass all stereoisomers, optical isomers and racemates of the above isomers. Unless otherwise indicated, all chiral (enantiomers and diastereoisomers) and racemic forms are within the scope of the present disclosure. Many geometric isomers of C=C double bonds, C=N double bonds, ring systems, etc. may also present in the compounds, and all of the above stable isomers are encompassed by the present disclosure. The present disclosure describes cis- and trans- (or E- and Z-) geometric isomers of the compounds of the present disclosure, which may be separated into mixtures of isomers or into separated isomeric forms.

The compounds of the present disclosure may be isolated in optically active or racemic forms. All methods for producing the compounds of the present disclosure and intermediates produced therein are considered part of the present disclosure. When enantiomeric or diastereomeric products are produced, they may be separated by conventional methods (e.g., by chromatography or fractional crystallization). It should be understood that all tautomeric forms that may exist are included in the present disclosure. The compounds used in the present disclosure may be obtained commercially when they are known compounds in the prior art.

The term "alkyl" referred to a branched or straight-chain saturated aliphatic hydrocarbon group having a specified number of carbon atoms. The alkyl in the present disclosure is preferably C₁-C₁₂ alkyl, C₁-C₁₀ alkyl, C₁-C₈ alkyl, more preferably C₁-C₆ alkyl, particularly preferably C₁-C₄ alkyl, especially C₁-C₃ alkyl. For example, "C₁-C₆ alkyl" means an alkyl having 1 to 6 carbon atoms. Examples of alkyl include, but are not limited to, methyl, ethyl, propyl (e.g., n-propyl and isopropyl), butyl (e.g., n-butyl, isobutyl, tert-butyl) and pentyl (e.g., n-pentyl, isopentyl, neopentyl). The alkyl may be substituted or unsubstituted. In the case that the alkyl is substituted, any available connection site may be substituted by a substituent, and the substituent is preferably one or more independently selected from the group consisting of alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkyloxy, heterocycloalkyloxy, cycloalkylthio, heterocycloalkylthio, oxo, carboxyl or carboxylate. For the C₁-C₁₂ alkyl in the present disclosure, 1, 2, 3 or 4 -CH₂- units therein may be optionally replaced by O atoms, S atoms or -NH-.

The term "alkoxy" referred to -O-(alkyl) or -O-(unsubstituted cycloalkyl). For example, "C₁-C₆ alkoxy" referred to C₁, C₂, C₃, C₄, C₅, and C₆ alkoxy. Preferred alkoxy is C₁-C₁₀ alkoxy, preferably C₁-C₈ alkoxy, more preferably C₁-C₆ alkoxy, particularly preferably C₁-C₄ alkoxy, especially C₁-C₃ alkoxy. Examples of alkoxy include, but are not limited to, methoxy, ethoxy, propoxy (e.g., n-propoxy and isopropoxy), tert-butoxy, cyclopropyloxy, cyclobutyloxy, cyclopentyloxy and cyclohexyloxy. Alkoxy may be optionally substituted or unsubstituted. In the case that the alkoxy is substituted by a substituent, the substituent is preferably one or more independently selected from the group consisting of alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkyloxy, heterocycloalkyloxy, cycloalkylthio, heterocycloalkylthio, carboxyl or carboxylate group. Similarly, "alkylthio" referred to alkyl having a specified number of carbon atoms as defined above connected by a sulfur bridge; for example, methyl-S- and ethyl-S-. Similarly, preferred alkylthio is C₁-C₁₀ alkylthio, C₁-C₈ alkylthio, more preferably C₁-C₆ alkylthio, particularly preferably C₁-C₄ alkylthio, especially C₁-C₃ alkylthio.

The term "halo" or "halogen" includes fluorine, chlorine, bromine and iodine. In the present disclosure, one or more halogens can be independently selected from the group consisting of fluorine, chlorine, bromine and iodine.

The term "haloalkyl" referred to a branched or straight-chain saturated aliphatic hydrocarbon group having a specified number of carbon atoms and substituted with one or more halogens. Examples of haloalkyl include, but are not limited to, fluoromethyl, difluoromethyl, trifluoromethyl, trichloromethyl, pentafluoroethyl, pentachloroethyl, 2,2,2-trifluoroethyl, heptafluoropropyl and heptachloropropyl. Preferred haloalkyl includes halo(C₁-C₆ alkyl) and halo(C₁-C₄ alkyl).

The term "oxo" or "carbonyl" referred to an organic functional group (C=O or C(O)) formed by connecting carbon atom and oxygen atom through a double bond.

The term "benzyl" referred to -CH₂-phenyl or "Bn".

The term "hydroxyl" referred to -OH group.

The term "amino" referred to -NH₂.

The term "cyano" referred to -CN.

The term "nitro" referred to -NO₂.

The term "carboxyl" referred to -C(O)OH.

The term "mercapto" referred to -SH.

The term "ester" or "carboxylate" referred to -C(O)O-(alkyl) or -C(O)O(cycloalkyl), wherein alkyl and cycloalkyl are as defined above.

The term "acyl" referred to a compound containing a group of -C(O)R, wherein R is alkyl, cycloalkyl, heterocyclyl, aryl, or heteroaryl.

The term "cycloalkyl" referred to a saturated or partially unsaturated monocyclic or polycyclic hydrocarbon substituent, wherein the ring of cycloalkyl contains 3 to 20 carbon atoms, and the cycloalkyl of the present disclosure is preferably C₃-C₈ cycloalkyl or C₃-C₆ cycloalkyl. Monocyclic cycloalkyl includes, but is not limited to, cyclopropyl, cyclobutyl, cyclopentyl, cyclopentenyl, cyclohexyl, cyclohexenyl, cyclohexadienyl, cycloheptyl, cycloheptatrienyl, and cyclooctyl, and polycyclic cycloalkyl includes, but is not limited to, spirocyclic, fused, and bridged cycloalkyl, such as norbornyl.

The ring of cycloalkyl may be fused to a ring of aryl, heteroaryl, or heterocyclyl, wherein the ring attached to the parent structure is cycloalkyl, non-limiting examples of which include, but are not limited to:

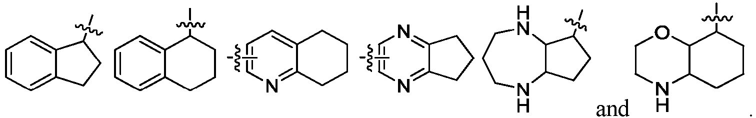

The cycloalkyl may be optionally substituted or unsubstituted. In the case that the cycloalkyl is substituted by a substituent, the substituent is preferably one or more independently selected from the group consisting of alkyl, alkenyl, alkynyl, alkoxy, hydroxyalkyl, alkylthio, alkylamino, halogen, thiol, hydroxyl, nitro, cyano, cycloalkyl, heterocycloalkyl, aryl, aryloxy, heteroaryl, cycloalkyloxy, heterocycloalkyloxy, cycloalkylthio, heterocycloalkylthio, oxo, carboxyl, or carboxylate.

The term "heterocyclyl" referred to a saturated or partially unsaturated monocyclic or polycyclic hydrocarbon group containing 3 to 20 ring atoms, wherein one or more of the ring atoms is a heteroatom selected from the group consisting of N, O and S (N and S atoms may be optionally oxidized), but the ring part of -O-O-, -O-S- or -S-S- is excluded, and the remaining ring atoms are carbon. Preferably, heterocyclyl contains 3 to 12 ring atoms, of which 1 to 4 are heteroatoms; most preferably, heterocyclyl contains 3 to 8 ring atoms, of which 1 to 3 are heteroatoms; most preferably, heterocyclyl contains 5 to 7 ring atoms, of which 1 to 2 or 1 to 3 are heteroatoms. Examples of monocyclic heterocyclyl include, but are not limited to, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl and tetrahydropyranyl, and polycyclic heterocyclyl includes, but is not limited to, spirocyclic, fused and bridged heterocyclyls.

The ring of a heterocyclyl may be fused to a ring of aryl, heteroaryl or cycloalkyl, wherein the ring attached to the parent structure is heterocyclyl, non-limiting examples of which include but are not limited to:

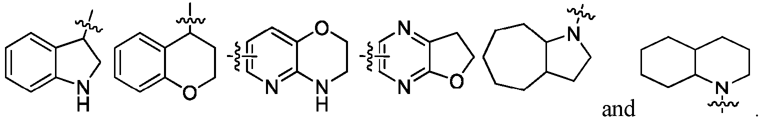

The heterocyclyl may be optionally substituted or unsubstituted. In the case that the heterocyclyl is substituted by a substituent, the substituent is preferably one or more independently selected from the group consisting of alkyl, alkenyl, alkynyl, alkoxy, hydroxyalkyl, alkylthio, alkylamino, halogen, thiol, hydroxyl, nitro, cyano, cycloalkyl, heterocycloalkyl, aryl, aryloxy, heteroaryl, cycloalkyloxy, heterocycloalkyloxy, cycloalkylthio, heterocycloalkylthio, oxo, carboxyl or carboxylate group.

The term "aryl" referred to a monocyclic, bicyclic or tricyclic ring system having a conjugated π electron system with a total of 6 to 14 ring atoms, preferably 6 to 10 carbon atoms, wherein at least one ring in the system is aromatic and each ring in the system contains 3 to 7 ring atoms. In certain embodiments of the present disclosure, "aryl" referred to an aromatic ring system, including but not limited to phenyl, naphthyl, biphenyl, indanyl, 1-naphthyl, 2-naphthyl and tetrahydronaphthyl. The aryl of the present disclosure is preferably C₆-C₁₀ aryl. The aryl may be substituted or unsubstituted. In the case that the aryl is substituted by a substituent, the substituent is preferably one or more independently selected from the group consisting of alkyl, alkenyl, alkynyl, alkoxy, hydroxyalkyl, alkylthio, alkylamino, halogen, thiol, hydroxyl, nitro, cyano, cycloalkyl, heterocycloalkyl, aryl, aryloxy, heteroaryl, cycloalkyloxy, heterocycloalkyloxy, cycloalkylthio, heterocycloalkylthio, carboxyl or carboxylate group.

The term "heteroaryl" referred to a stable 3-, 4-, 5-, 6-, or 7-membered aromatic monocyclic ring or a 7-, 8-, 9-, 10-, 11-, or 12-membered aromatic bicyclic ring or aromatic polycyclic heterocycle, which is completely unsaturated or partially unsaturated, and contains carbon atoms and 1, 2, 3, or 4 heteroatoms independently selected from the group consisting of N, O, and S (N and S atoms may be optionally oxidized). The nitrogen atom is substituted or unsubstituted (i.e., N or NR, wherein R is H or, if defined, another substituent). The heterocycle may be connected to its side group at any heteroatom or carbon atom that results in a stable structure. If the resulting compound is stable, the heterocyclyl of the present disclosure may be substituted at a carbon or nitrogen atom. The nitrogen in the heterocycle may be optionally quaternized. Preferably, in the case that the total number of S and O atoms in the heterocycle exceeds 1, these heteroatoms are not adjacent to each other. Preferably, the total number of S and O atoms in the heterocycle is not greater than 1. When the term "heterocycle" is used, it is intended to include heteroaryl. Examples of heteroaryl include, but are not limited to, acridinyl, imidazolyl, furanyl, thienyl, oxazolyl, thiazolyl, benzotriazolyl, benzotetrazolyl, benzisoxazolyl, benzisothiazolyl, indolyl, indolizinyl, indazolyl, pyrimidinyl, phenazinyl, piperazinyl, piperidinyl, purinyl, pyranyl, pyrazinyl, pyrrolyl and quinolinyl. The term "heteroaryl" may further include biaryl structures formed by the above-defined "aryl", "heterocyclyl" or "cycloalkyl" and monocyclic "heteroaryl", such as but not limited to "-phenylbipyridyl-", "-phenylbipyrimidyl-", "-pyridylbinaphthyl-", "-pyrimidylbinaphthyl-" and "-pyridylbipyrimidyl-", wherein the present disclosure further includes spirocyclic, fused and bridged ring compounds containing, for example, the above-mentioned heterocycles.

"Optional" or "optionally" as used herein means that the event or circumstance described subsequently may but need not occur, and the description includes occasions where the event or circumstance occurs or does not occur. For example, "a heterocyclyl optionally substituted by alkyl" means that the alkyl may but need not be present, and the description includes the case where the heterocyclyl is substituted by an alkyl and the case where the heterocyclyl is not substituted by an alkyl.

"Substitution" or "substituted" as used herein referred to one or more hydrogen atoms in a group, preferably up to 5, more preferably 1 to 3 hydrogen atoms, being replaced independently by a corresponding number of substituents, provided that normal valence is maintained and the substitution results in a stable compound. It goes without saying that the substituents are only in their possible chemical positions, and those skilled in the art can determine (by experiment or theory) possible or impossible substitutions without making undue effort. For example, an amino or hydroxyl with free hydrogen may be unstable when connected with a carbon atom with an unsaturated (such as olefin) bond.

"Pharmaceutical composition" referred to a mixture comprising one or more compounds of the present disclosure or the mesomers, racemates, enantiomers, diastereomers or pharmaceutically acceptable salts thereof, and optionally comprising additional components such as physiologically/pharmaceutically acceptable carriers and/or excipients. A purpose of a pharmaceutical composition is to facilitate the administration to an organism and the absorption of active ingredients, thus exerting biological activity.

### DETAILED DESCRIPTION

In order to better illustrate the technical means and effects of the present disclosure, the present disclosure is further illustrated in conjunction with non-limiting examples. The examples of the present disclosure, including the descriptions provided in the examples, are intended to illustrate the embodiments of the present disclosure and are not intended to limit the scope of any claims. According to the present disclosure, those skilled in the art will understand that many changes can be made to the disclosed specific embodiments without departing from the spirit and scope of the present disclosure and still obtain the same or similar results.

Unless otherwise stated, all materials/reagents were obtained from commercial suppliers and used without further purification. The structures of the compounds in the following examples were characterized and determined by nuclear magnetic resonance (NMR) and/or mass spectrometry (MS).

The ¹H NMR spectra were recorded at room temperature on a Bruker Avance 400 MHz spectrometer, and the detection solvents were deuterated dimethyl sulfoxide (DMSO-*d*₆), deuterated chloroform (CDCl₃), deuterated methanol (CD₃OD) or deuterated water (D₂O). Chemical shift values (*δ*) are expressed in ppm, with tetramethylsilane (TMS) or residual solvent peak as internal standard, coupling constant (J) is expressed in Hertz (Hz), and the abbreviations of the multiplicity of peaks in ¹H NMR spectra are as follows: s (singlet), d (doublet), t (triplet), q (quartet), qn (quintet), m (multiplet), br (broad peak).

The instrument used for liquid chromatography-mass spectrometry (LC-MS) was Shimadzu LCMS-2020, and the instrument used for preparative high performance liquid chromatography (Prep-HPLC) was Bonna-Agela FLEXA FL-H100G. The thin layer chromatography silica gel plate model used for thin layer chromatography (TLC) was Yantai Huanghai HSGF254 thin layer chromatography silica gel plate, with a size for reaction monitoring of 2.5 × 8 cm and a coating thickness of 0.2 ± 0.03 mm, and a size for separation and purification of 20 × 20 cm and a coating thickness of 0.4-0.5 mm. The carrier for the silica gel column chromatography method was Qingdao Ocean Silica Gel of 100-200 mesh or 200-300 mesh.

Unless otherwise defined, all professional and scientific terms used herein have the same meaning as those familiar to those skilled in the art. In addition, any methods and materials similar or equivalent to the recorded content can be applied to the method of the present disclosure.

### Example 1: Synthesis route of compound 1 and compound 2

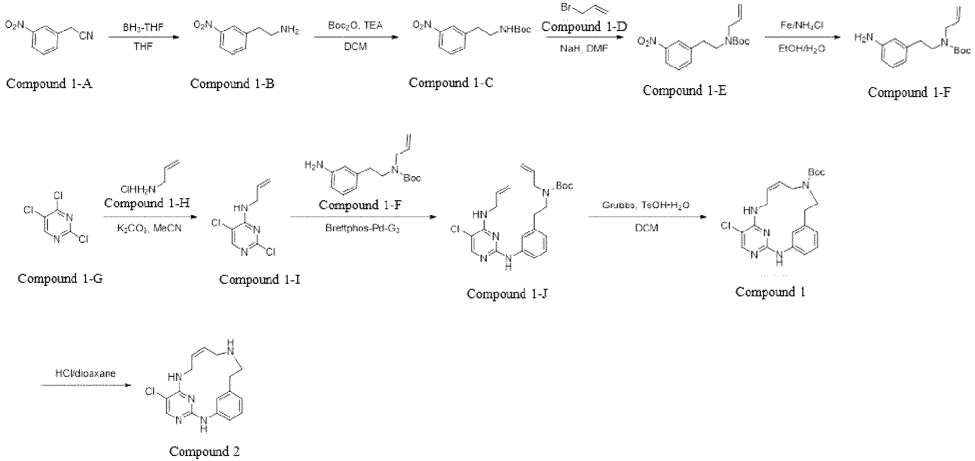

### Step 1: Compound 1-B 2-(3-nitrophenyl)ethyl-1-amine

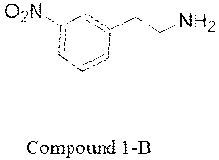

A solution of borane in tetrahydrofuran (24.6 mL, 1M) was added to a solution of **compound 1-A** 2-(3-nitrophenyl)acetonitrile (2.0 g, 12.33 mmol) in tetrahydrofuran (20 mL). The reaction solution was stirred at 70°C for 3 hours. The reaction was monitored by TLC to be completed. Methanol (30 mL) was added to the reaction solution and stirred at 70°C for 1 hour. The reaction solution was concentrated under reduced pressure to obtain a yellow oily crude **compound 1-B** 2-(3-nitrophenyl)ethyl-1-amine (2.05 g), which was used directly in the next step without further purification.

### Step 2: Compound 1-C Tert-butyl (3-nitrophenylethyl) carbamate

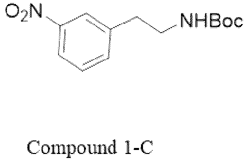

Triethylamine (2.50 g, 24.67 mmol) and di-tert-butyl dicarbonate (2.96 g, 13.57 mmol) were added to a solution of **compound 1-B** 2-(3-nitrophenyl) ethan-1-amine (2.05 g, 12.34 mmol) in dichloromethane (30 mL). The reaction solution was stirred at 20 °C for 12 hours. The reaction was monitored by TLC to be completed. The reaction solution was diluted with water and extracted with dichloromethane (50 mLx2). The organic layers were combined, washed with saturated brine (100 mL), dried over anhydrous sodium sulfate, filtered, and concentrated under reduced pressure. The crude product was separated and purified by silica gel column chromatography (eluted with a petroleum ether solution containing 20% ethyl acetate) to obtain a yellow oil **compound 1-C** tert-butyl (3-nitrophenylethyl) carbamate (2.54 g).

LC_MS: (ES⁺): m/z 279.1 [M+H] ⁺.

### Step 3: Compound 1-E tert-butyl allyl(3-nitrophenylethyl)carbamate

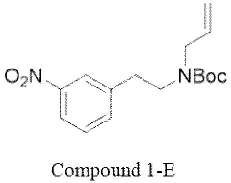

Sodium hydride (180.2 mg, 4.51 mmol, mass fraction 60%) was added to a solution of **compound 1-C** (3-nitrophenylethyl) carbamate (1.0 g, 3.76 mmol) in DMF (30 mL) at 0 °C, stirred for 30 minutes, and added with **compound 1-D** 3-bromoprop-1-ene (499.7 mg, 4.13 mmol). The reaction solution was stirred at 20 °C for 12 hours. The reaction was monitored by LCMS to be completed. The reaction solution was poured into a saturated ammonium chloride solution (30 mL) and extracted with ethyl acetate (30 mLx2). The organic layers were combined, washed with saturated brine (30 mL), dried over anhydrous sodium sulfate, filtered, and concentrated under reduced pressure. The obtained crude product was separated and purified by silica gel column chromatography (eluted with petroleum ether solution containing 10%-20% ethyl acetate) to obtain a yellow oily **compound 1-E** tert-butyl allyl(3-nitrophenylethyl)carbamate (828 mg).

LC_MS: (ES⁺): m/z 307.1 [M+H] ⁺.

### Step 4: Compound 1-F tert-butyl allyl(3-aminophenylethyl)carbamate

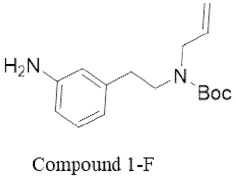

Iron powder (755 mg, 13.51 mmol) and ammonium chloride (723 mg, 13.51 mmol) were added to a solution of **compound 1-E** tert-butyl allyl(3-nitrophenylethyl)carbamate (828 mg, 2.70 mmol) in ethanol (10 mL)/water (2 mL). The reaction solution was stirred at 80 °C for 12 hours. The reaction was monitored by TLC to be completed. The reaction solution was filtered, concentrated, diluted with water (20 mL), and extracted with ethyl acetate (20 mLx2). The organic layers were combined, washed with saturated brine (30 mL), dried over anhydrous sodium sulfate, filtered, and concentrated under reduced pressure. The obtained crude product was separated and purified by silica gel column chromatography (eluted with a petroleum ether solution containing 20% ethyl acetate) to obtain a yellow oily **compound 1-F** tert-butyl allyl(3-aminophenylethyl)carbamate (710 mg).

### Step 5: Compound 1-I N-allyl-2,5-dichloropyrimidin-4-amine

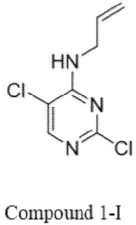

Potassium carbonate (2.26 g, 16.36 mmol) was added to a solution of **compound 1-G** 2,4,5-trichloropyrimidine (1.0 g, 5.45 mmol) and **compound 1-H** prop-2-en-1-amine hydrochloride (535.6 mg, 5.72mmol) in acetonitrile (20 mL). The reaction solution was stirred at 80 °C for 3 hours. The reaction was monitored by TLC to be completed. The reaction solution was diluted with water (30 mL) and extracted with ethyl acetate (30 mLx2). The organic layers were combined, washed with saturated brine (50 mL), dried over anhydrous sodium sulfate, filtered, and concentrated under reduced pressure. The residue was separated and purified by silica gel column chromatography (eluted with petroleum ether solution containing 10% ethyl acetate) to obtain a white solid **compound 1-I** N-allyl-2,5-dichloropyrimidin-4-amine (1.129 g).

LC_MS: (ES⁺): m/z 204.0 [M+H] ⁺.

### Step 6: Compound 1-J tert-butyl allyl(3-((4-(allylamino)-5-chloropyrimidin-2-yl)amino)phenethyl)carbamate

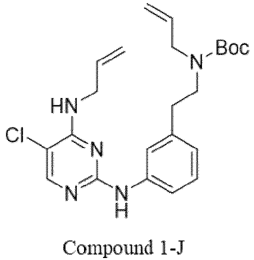

Cesium carbonate (2.40 g, 7.35 mmol) and BrettPhos-Pd-G3 (111 mg, 122.5 µmol) were added to a solution of compound 1-I N-allyl-2,5-dichloropyrimidin-4-amine (500 mg, 2.45 mmol) and compound 1-F tert-butyl allyl (3-aminophenethyl)carbamate (677.2 mg, 2.45 mmol) in 1,4-dioxane (15 mL). The reaction solution was stirred at 100 °C under nitrogen atmosphere for 12 hours. The reaction was monitored by TLC to be completed. The reaction solution was filtered, diluted with water (30 mL), and extracted with ethyl acetate (30 mLx2). The organic layers were combined, washed with saturated brine (50 mL), dried over anhydrous sodium sulfate, filtered, and concentrated under reduced pressure. The residue was separated and purified by silica gel column chromatography (eluted with a petroleum ether solution containing 25% ethyl acetate) to obtain a yellow oily **compound 1-J** tert-butyl allyl(3-((4-(allylamino)-5-chloropyrimidin-2-yl)amino)phenethyl)carbamate (830 mg).

LC_MS: (ES⁺): m/z 444.2 [M+H] ⁺.

### Step 7: Compound 1 tert-butyl (Z)-1⁵-chloro-2,6,11-triaza-1(2,4)-pyrimidina-3(1,3)-benzenacycloundecaphan-8-ene-6-carboxyla te

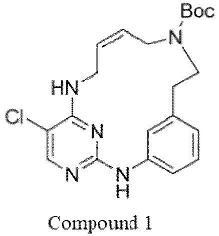

Grubb's (143.9 mg, 169.5 mmol) and TsOH•H₂O (224.9 mg, 1.18 mmol) were added to a solution of **compound 1-J** tert-butyl allyl (3-((4-(allylamino)-5-chloropyrimidin-2-yl)amino)phenethyl)carbamate (500 mg, 1.13 mmol) in dichloromethane (30 mL). The reaction solution was stirred at 45 °C for 12 hours. The reaction was monitored by TLC to be completed. The reaction solution was added with saturated sodium bicarbonate solution (20 mL). The organic layer was collected, washed with saturated brine (30 mL), dried over anhydrous sodium sulfate, filtered, and concentrated under reduced pressure. The residue was separated and purified by silica gel column chromatography (eluted with a petroleum ether solution containing 30%-50% ethyl acetate) to obtain a brown solid **compound 1** tert-butyl (Z)-1⁵-chloro-2,6,11-triaza-1(2,4)-pyrimidina-3(1,3)-benzenacycloundecaphan-8-ene-6-carboxyla te (490 mg).

LC_MS: (ES⁺): m/z 416.2 [M+H] ⁺.

### Step 8: Compound 2 (Z)-1⁵-chloro-2,6,11-triaza-1(2,4)-pyrimidina-3(1,3)-benzenacycloundecaphan-8-ene

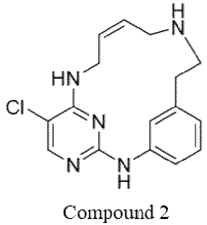

A solution of **compound 1** tert-butyl (Z)-1⁵-chloro-2,6,11-triaza-1(2,4)-pyrimidina-3(1,3)-benzenacycloundecaphan-8-ene-6-carboxyla te (10 mg, 24.0 µmol) in hydrogen chloride/1,4-dioxane (1 mL) was stirred at 25 °C for 2 hours. The reaction was monitored by LCMS to be completed. The reaction solution was concentrated under reduced pressure to obtain a brown solid **compound 2** (Z)-1⁵-chloro-2,6,11-triaza-1(2,4)-pyrimidina-3(1,3)-benzenacycloundecaphan-8-ene (5.7 mg).

LC_MS: (ES⁺): m/z 316.1 [M+H] ⁺.

### Example 2: Synthesis route of compound 3 and compound 4

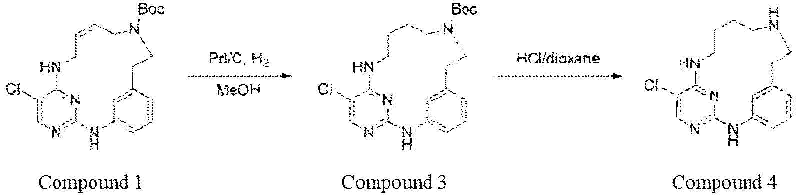

### Step 1: Compound 3 tert-butyl-1⁵-chloro-2,6,11-triaza-1(2,4)-pyrimidina-3(1.3)-benzenacycloundecaphan-6-carboxyla te

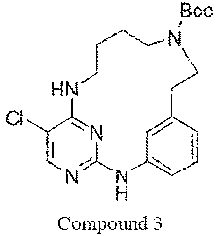

Palladium/carbon (50 mg) was added to a solution of **compound 1** tert-butyl (Z)-1⁵-chloro-2,6,11-triaza-1(2,4)-pyrimidina-3(1,3)-benzenacycloundecaphan-8-ene-6-carboxyla te (100 mg, 24.04 µmol) in methanol. The reaction solution was stirred at 20 °C under a hydrogen balloon atmosphere for 2 hours. The reaction was monitored by LCMS to be completed. The reaction solution was filtered through celite, and the residue was separated and purified by Pre-TLC (eluted with a petroleum ether solution containing 50% ethyl acetate) to obtain a brown solid **compound 3** tert-butyl-1⁵-chloro-2,6,11-triaza-1(2,4)-pyrimidina-3(1.3)-benzenacycloundecaphan-6-carboxyla te (26.1 mg).

LC_MS: (ES⁺): m/z 418.2 [M+H] ⁺.

### Step 2: Compound 4 1⁵ -chloro-2,6,11-triaza-1(2,4)-pyrimidina-3(1.3)-benzenacyclodecaphane

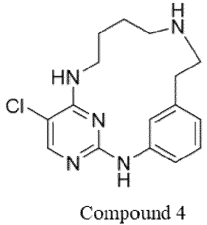

A solution of **compound 3** tert-butyl-1⁵-chloro-2,6,11-triaza-1(2,4)-pyrimidina-3(1.3)-benzenacycloundecaphan-6-carboxyla te (20 mg, 47.9 µmol) in hydrogen chloride/1,4-dioxane (1 mL) was stirred at 25°C for 2 hours. The reaction was monitored by LCMS to be completed. The reaction solution was concentrated under reduced pressure to obtain a brown solid **compound 4** 1⁵-chloro-2,6,11-triaza-1(2,4)-pyrimidina-3(1.3)-benzocycloundecaphane (10.5 mg).

LC_MS: (ES⁺): m/z 318.1 [M+H] ⁺.

### Example 3: Synthesis route of compound 5

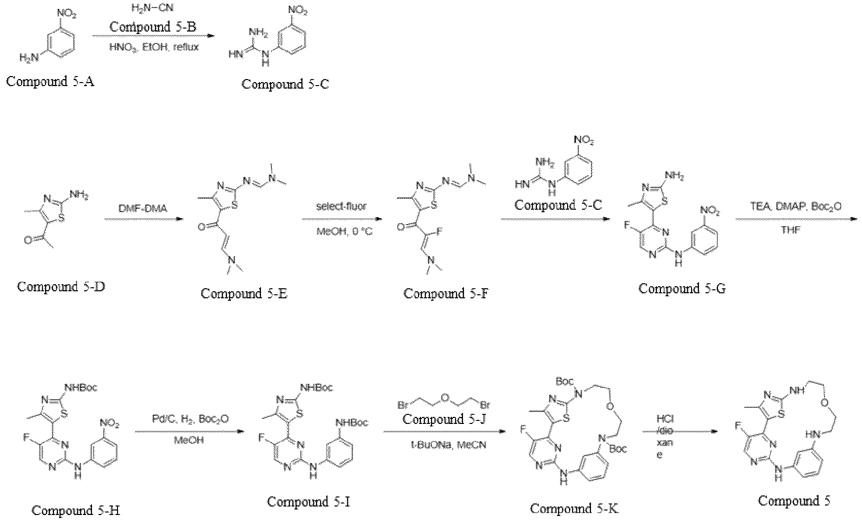

### Step 1: Compound 5-C 1-(3-nitrophenyl)guanidine

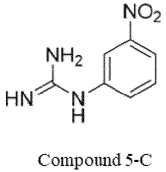

**Compound 5-B** cyanamide (3.50 g, 83.26 mmol) and 70% nitric acid (6.52 g, 72.40 mmol) were added to a solution of **compound 5-A** 3-nitroaniline (10 g, 72.4 mmol) in ethanol (50 mL) .The reaction solution was stirred at 80 °C under a nitrogen atmosphere for 12 hours. The reaction was monitored by LCMS to be completed. The reaction solution was concentrated, the residue was triturated with ethanol/methyl tert-butyl ether = 1:1 (50 mL) and filtered, and the filter cake was collected to obtain a yellow solid **compound 5-C** 1-(3-nitrophenyl)guanidine (6.33 g). The obtained crude product was used directly in the next step without further purification.

LC_MS: (ES⁺): m/z 181.0 [M+H] ⁺.

### Step 2: Compound 5-E (E)-N'-(5-((E)-3-(dimethylamino)acryloyl)-4-methylthiazol-2-yl)-N,N-dimethylformimide

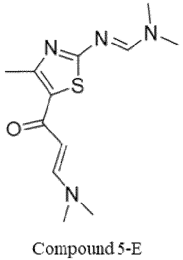

A solution of **compound 5-D** 1-(2-amino-4-methylthiazol-5-yl)ethan-1-one (5.0 g, 32.01 mmol) in 1,1-dimethoxy-N,N-dimethylmethanamine (30 mL) was stirred at 120 °C for 12 hours. The reaction was monitored by LCMS to be completed. The residue was triturated with ethyl acetate/petroleum ether = 2:1 (50 mL) and filtered, and the filter cake was collected to obtain an orange solid **compound 5-E** (E)-N'-(5-((E)-3-(dimethylamino)acryloyl)-4-methylthiazol-2-yl)-N,N-dimethylformimide (4.47 g). The crude product was used directly in the next step without further purification. LC_MS: (ES⁺): m/z 267.1 [M+H] ⁺.

### Step 3: Compound 5-F (E)-N'-(5-((Z)-3-(dimethylamino)-2-fluoroacryloyl)-4-methylthiazol-2-yl)-N,N-dimethylformimi de

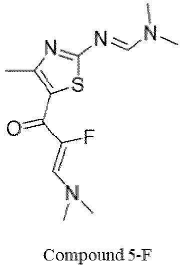

1-Chloromethyl-4-fluoro-1,4-diazabicyclo[2.2.2]octane di(tetrafluoroborate) salt (8.92 g, 25.173 mmol) was added to a solution of **compound 5-E** (E)-N'-(5-((E)-3-(dimethylamino)acryloyl)-4-methylthiazol-2-yl)-N,N-dimethylformimide (4.47 g, 16.782 mmol) in methanol (50 mL) at 0 °C, and the reaction solution was stirred at 0 °C for 2 hours. The reaction was monitored by LCMS to be completed. The reaction solution was filtered and concentrated, and the residue was separated and purified by silica gel column chromatography (eluted with a dichloromethane solution containing 6.6% methanol) to obtain a crude yellow oily **compound 5-F** (E)-N'-(5-((Z)-3-(dimethylamino)-2-fluoroacryloyl)-4-methylthiazol-2-yl)-N,N-dimethylformimi de (3.9 g).

LC_MS: (ES⁺): m/z 285.1 [M+H] ⁺.

### Step 4: Compound 5-H 5-(5-fluoro-2-((3-nitrophenyl)amino)pyrimidin-4-yl)-4-methylthiazol-2-amine

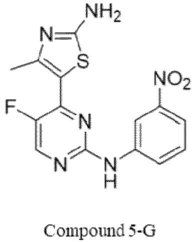

Sodium hydroxide (549 mg, 13.72 mmol) was added to a solution of **compound 5-F** (E)-N'-(5-((Z)-3-(dimethylamino)-2-fluoroacryloyl)-4-methylthiazol-2-yl)-N,N-dimethylformimi de (3.9 g, 13.72 mmol) and **compound 5-C** 1-(3-nitrophenyl)guanidine (4.94 g, 27.43 mmol) in ethylene glycol monomethyl ether (50 mL). The reaction solution was stirred at 125 °C for 12 hours. The reaction was monitored by LCMS to be completed. The reaction solution was concentrated, diluted with water (50 mL), and extracted with ethyl acetate (50 mLx2). The organic layers were combined, washed with saturated brine (30 mL), dried over anhydrous sodium sulfate, filtered, and concentrated. The residue was separated and purified by silica gel column chromatography (eluted with a petroleum ether solution containing 50% - 66% ethyl acetate) to obtain a yellow solid **compound 5-G** 5-(5-fluoro-2-((3-nitrophenyl)amino)pyrimidin-4-yl)-4-methylthiazol-2-amine (1.12 g).

LC_MS: (ES⁺): m/z 347.0 [M+H] ⁺.

### Step 5: Compound 5-H tert-butyl (5-(5-fluoro-2-((3-nitrophenyl)amino)pyrimidin-4-yl)-4-methylthiazol-2-yl)carbamate

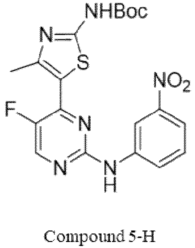

Triethylamine (981.7 mg, 9.7 mmol) and 4-dimethylaminopyridine (39.5 mg, 323.38 µmol) were added to a solution of **compound 5-G** 5-(5-fluoro-2-((3-nitrophenyl)amino)pyrimidin-4-yl)-4-methylthiazol-2-amine (1.12 g, 3.23 mmol) and di-tert-butyl dicarbonate (776.4 mg, 3.56 mmol) in tetrahydrofuran (20 mL). The reaction solution was stirred at 20 °C for 12 hours. The reaction was monitored by LCMS to be completed. The reaction solution was concentrated, and the residue was separated and purified by silica gel column chromatography (eluted with a petroleum ether solution containing 50% ethyl acetate) to obtain a yellow solid **compound 5-H** tert-butyl (5-(5-fluoro-2-((3-nitrophenyl)amino)pyrimidin-4-yl)-4-methylthiazol-2-yl)carbamate (1.275 g).

LC_MS: (ES⁺): m/z 447.1 [M+H] ⁺.

### Step 6: Compound 5-I tert-butyl (5-(2-((3-((tert-butoxycarbonyl)amino)phenyl)amino)-5-fluoropyrimidin-4-yl)-4-methylthiazol-2-yl)carbamate

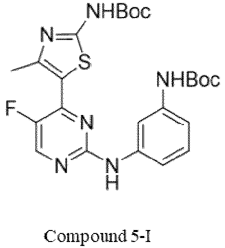

Di-tert-butyl dicarbonate (623.3 mg, 2.86 mmol) and palladium/carbon (150 mg) were added to a solution of **compound 5-H** tert-butyl (5-(5-fluoro-2-((3-nitrophenyl)amino)pyrimidin-4-yl)-4-methylthiazol-2-yl)carbamate (1.275 g, 2.86 mmol) in methanol (15 mL)/tetrahydrofuran (15 mL). The reaction solution was stirred at 20 °C under a hydrogen balloon atmosphere for 120 hours. The reaction was monitored by LCMS to be completed. The reaction solution was filtered through celite, the filtrate was concentrated, and the residue was separated and purified by silica gel column chromatography (eluted with a petroleum ether solution containing 30%-50% ethyl acetate) to obtain a yellow solid **compound 5-I** tert-butyl (5-(2-((3-((tert-butoxycarbonyl)amino)phenyl)amino)-5-fluoropyrimidin-4-yl)-4-methylthiazol-2-yl)carbamate (500 mg).

LC_MS: (ES⁺): m/z 517.2 [M+H] ⁺.

### Step 7: Compound 5-K di-tert-butyl 2⁵-fluoro-1⁴-methyl-8-oxa-3,5,11-triaza-1(5,2)-thiazol-2(4,2)-pyrimidina-4(1,3)-benzenacyclound ecaphan-5,11-dicarboxylate

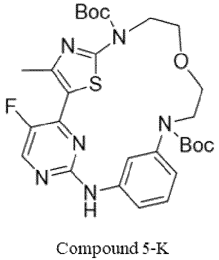

Sodium tert-butoxide (7.4 mg, 77.43 µmol) and **compound 5-J** 1-bromo-2-(2-bromoethoxy)ethane (9.0 mg, 38.72 µmol) were added to a solution of compound **5-I** tert-butyl (5-(2-((3-((tert-butoxycarbonyl)amino)phenyl)amino)-5-fluoropyrimidin-4-yl)-4-methylthiazol-2-yl)carbamate (20 mg, 38.72 µmol) in acetonitrile (5 mL). The reaction solution was stirred at 60 °C for 12 hours. The reaction was monitored by LCMS to be completed. The reaction solution was diluted with water (10 mL) and extracted with ethyl acetate (10 mLx2). The organic layers were combined, washed with saturated brine (20 mL), dried over anhydrous sodium sulfate, filtered, and concentrated. The residue was separated and purified by Pre-TLC (eluted with a petroleum ether solution containing 33% ethyl acetate) to obtain a yellow solid **compound 5-K** di-tert-butyl 2⁵-fluoro-1⁴-methyl-8-oxa-3,5,11-triaza-1(5,2)-thiazol-2(4,2)-pyrimidina-4(1,3)-benzenacyclound ecaphan-5,11-dicarboxylate (3 mg).

LC_MS: (ES⁺): m/z 587.2 [M+H] ⁺.

### Step 8: Compound 5 2⁵-fluoro-1⁴-methyl-8-oxa-3,5,11-triaza-1(5,2)-thiazol-2(4,2)-pyrimidina-4(1,3)-benzenacyclodec aphane

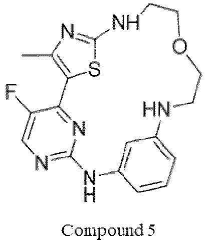

A solution of **compound 5-K** di-tert-butyl 2⁵-fluoro-14-methyl-8-oxa-3,5,11-triaza-1(5,2)-thiazol-2(4,2)-pyrimidina-4(1,3)-benzenacycloun decaphan-5,11-dicarboxylate (3 mg, 5.11 µmol) in hydrogen chloride/1,4-dioxane (2 mL) was stirred at 25 °C for 1 hour. The reaction was monitored by LCMS to be completed. The reaction solution was concentrated under reduced pressure, and the pH value was adjusted to 7 with aqueous ammonia. The residue was separated and purified by Pre-TLC (eluted with a dichloromethane solution containing 10% methanol) to obtain a yellow solid **compound 5** 2⁵-fluoro-1⁴-methyl-8-oxa-3,5,11-triaza-1(5,2)-thiazol-2(4,2)-pyrimidina-4(1,3)-benzenacyclound ecaphane (0.3 mg).

LC_MS: (ES⁺): m/z 387.1 [M+H] ⁺.

### Example 4: Synthesis route of compound 6

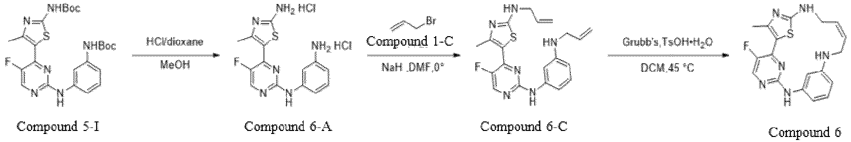

### Step 1: Compound 6-A Nitrogen¹-(4-(2-amino-4-methylthiazol-5-yl)-5-fluoropyrimidin-2-yl)benzene-1,3-diamine dihydrochloride

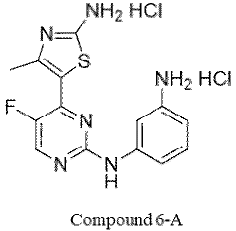

A hydrochloric acid/dioxane (1 ml) solution was added to a solution of **compound 5-I** (20 mg, 0.0387 mmol) in methanol (1 mL), and the reaction solution was stirred at 45°C for 12 hours. The reaction was monitored by TLC to be completed. The reaction solution was concentrated to obtain a yellow solid **compound 6-A** nitrogen¹-(4-(2-amino-4-methylthiazol-5-yl)-5-fluoropyrimidin-2-yl)benzene-1,3-diamine dihydrochloride (19 mg).

LC_MS: (ES⁺): m/z 317.1 [M+Na] ⁺

### Step 2: Compound 6-C Nitrogen ¹-allyl-nitrogen ³-(4-(2-(allylamino)-4-methylthiazol-5-yl)-5-fluoropyrimidin-2-yl)benzene-1,3-diamine

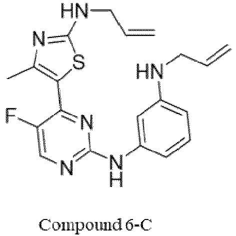

Sodium hydride (3.9 mg, 0.0976 mmol) was added to a solution of **compound 6-A** nitrogen¹-(4-(2-amino-4-methylthiazol-5-yl)-5-fluoropyrimidin-2-yl)benzene-1,3-diamine dihydrochloride (19 mg, 0.0488 mmol) in N,N-dimethylformamide (5 ml) at 0°C, and **compound 6-B** 3-bromoprop-1-ene (71.8 mg, 0.0976 mmol) was slowly added dropwise. The reaction solution was stirred at 0°C for 5 hours. The reaction was monitored by TLC to be completed. The reaction solution was poured into a saturated ammonium chloride solution and extracted with ethyl acetate. The organic layers were combined, washed with saturated brine, dried over anhydrous sodium sulfate, and concentrated. The residue was separated and purified by Pre-TLC (eluted with dichloromethane containing 4.76% methanol) to obtain a yellow solid **compound 6-C** nitrogen¹-allyl-nitrogen³-(4-(2-(allylamino)-4-methylthiazol-5-yl)-5-fluoropyrimidin-2-yl)benzen e-1,3-diamine (8 mg).

LC_MS: (ES⁺): m/z 397.1 [M+Na] ⁺

### Step 3: Compound 6 (Z)-2⁵-fluoro-1⁴-methyl-3,5,10-triaza-1(5,2)-thiazolin-2(4,2)-pyrimidina-4(1,3)-benzenacyclodec aphan-7-ene

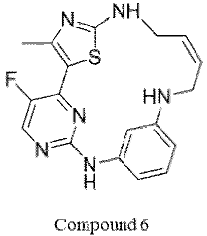

p-Toluenesulfonic acid monohydrate (4.01 mg, 0.0211 mmol) and Grubb's (5.13 mg, 0.006 mmol) were added to a solution of **compound 6-C** nitrogen¹-allyl-nitrogen³-(4-(2-(allylamino)-4-methylthiazol-5-yl)-5-fluoropyrimidin-2-yl)benzen e-1,3-diamine (8 mg, 0.0201 mmol) in dichloromethane (5 ml). The reaction solution was stirred at 42°C under nitrogen atmosphere for 12 hours. The reaction was monitored by TLC to be completed. The reaction solution was diluted with water and extracted with dichloromethane. The organic layers were combined, dried over anhydrous sodium sulfate, and concentrated. The residue was separated and purified by Pre-TLC (eluted with dichloromethane containing 4.76% methanol) to obtain a brown solid **compound 6** (Z)-2⁵-fluoro-1⁴-methyl-3,5,10-triaza-1(5,2)-thiazolin-2(4,2)-pyrimidina-4(1,3)-benzenacyclodec aphan-7-ene (1.3 mg).

LC _ms: (ES⁺): m/z 369.1 [M+Na] ⁺

### Example 5: Synthesis route of compound 7, compound 8, and compound 9

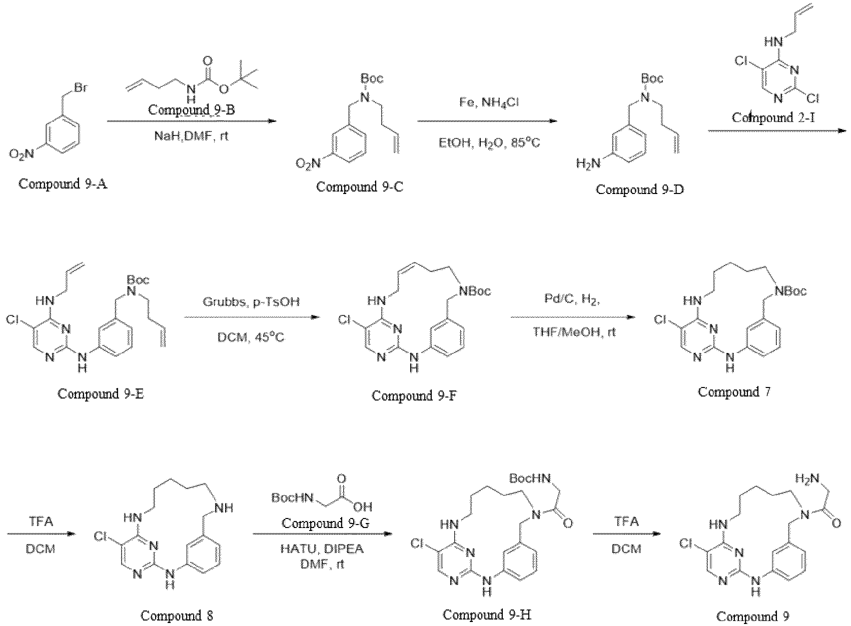

### Step 1: Compound 9-C tert-butyl but-3-en-1-yl (3-nitrobenzyl) carbamate

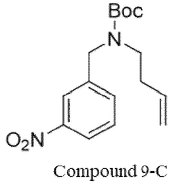

Sodium hydride (60% in kerosene, 233.6 mg, 5.8 mmol) was added to a solution of **compound 9-B** tert-butyl but-3-en-1-ylcarbamate (666 mg, 3.9 mmol) in N,N-dimethylformamide (10 mL) at 0°C. The reaction solution was warmed to room temperature, stirred for 30 minutes, then cooled to 0°C, and added with **compound 9**-A 1-(bromomethyl)-3-nitrobenzene (925 mg, 4.3 mmol) dropwise. The reaction solution was stirred for 12 hours under nitrogen atmosphere at room temperature. The reaction was monitored by TLC to be completed. The reaction solution was distributed between ethyl acetate (30 mL) and water (50 mL). The organic layer was collected, washed with saturated brine (30 mL), dried over anhydrous sodium sulfate, and concentrated under reduced pressure. The residue was separated and purified by silica gel column chromatography (eluted with a hexane solution containing 20% ethyl acetate) to obtain a brown oily **compound 9-C** tert-butyl but-3-en-1-yl (3-nitrobenzyl)carbamate (900 mg).

¹H NMR (400 MHz, CDCl₃): δ 1.44-1.51 (m, 9H), 2.23-2.28 (m, 2H), 3.26-3.34 (m, 2H), 5.29 (m, 2H), 5.02-5.07 (m, 2H), 5.71-5.79 (m, 1H), 7.48-7.59 (m, 2H), 8.10-8.13 (m, 2H).

### Step 2: Compound 9-D tert-butyl 3-aminobenzyl(but-3-en-1-yl)carbamate

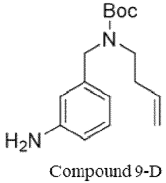

A solution of **compound 9-C** tert-butyl but-3-en-1-yl (3-nitrobenzyl) carbamate (900 mg, 2.94 mmol), iron powder (1.6 g, 29.4 mmol) and ammonium chloride (786.5 mg, 14.7 mmol) in ethanol (40 mL)/water (4 mL) was refluxed for 3 hours. The reaction was monitored by TLC to be completed. The reaction solution was filtered to remove iron powder, and the filter cake was washed with ethanol (2 mLx2). The filtrates were combined and concentrated under reduced pressure. The residue was separated and purified by silica gel column chromatography (eluted with n-hexane solution containing 20% ethyl acetate) to obtain a yellow oily **compound 9-D** tert-butyl 3-aminobenzyl (but-3-en-1-yl) carbamate (460 mg).

LC_MS: (ES⁺): m/z 221.2 [M+H] ⁺

¹H NMR (400 MHz, CDCl₃): δ 1.45-1.49 (m, 9H), 2.22-2.28 (m, 2H), 3.27-3.18 (m, 2H), 3.66 (s, 2H), 4.35 (s, 2H), 4.98-5.05 (m, 2H), 5.73-5.77 (m, 1H), 6.51-6.62 (m, 3H), 7.09 (t, *J=* 7.8 Hz, 1H).

### Step 3: Compound 9-E tert-butyl 3-((4-(allylamino)-5-chloropyrimidin-2-yl)amino)benzyl(but-3-en-1-yl)carbamate

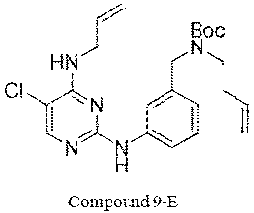

The synthesis of this step referred to the synthesis process of the sixth step of compound 2 in Example 1.

LC_MS: (ES⁺): m/z 444.6 [M+H] ⁺.

¹H NMR (400 MHz, CDCl₃): δ 1.45-1.50 (m, 9H), 2.20-2.26 (m, 2H), 3.20-3.29 (m, 2H), 4.13-4.17 (m, 2H), 4.43 (s, 2H), 4.98-5.05 (m, 2H), 5.19-5.36 (m, 3H), 5.74 (s, 1H), 5.94-6.04 (m, 1H), 6.88-6.94 (m, 2H), 7.23-7.27 (m, 1H), 7.40 (s, 1H), 7.53 (s, 1H), 7.91 (s, 1H).

### Step 4: Compound 9-F tert-butyl (Z)-1⁵-chloro-2,5,11-triaza-1(2,4)-pyrimidina-3(1,3)-benzenacycloundecaphan-8-en-5-carboxylat e

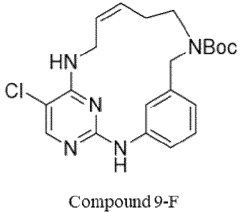

The synthesis of this step referred to the synthesis process of the seventh step of compound 2 in Example 1.

¹H NMR (400 MHz, DMSO-d6): δ 1.23-1.41 (m, 9H), 2.16-2.32 (m, 2H), 3.02-3.22 (m, 2H), 3.88-4.00 (m, 1H), 4.18-4.24 (m, 1H), 4.34-4.40 (m, 2H), 5.38-5.43 (m, 1H), 5.58-5.76 (m, 1H), 6.74-6.88 (m, 1H), 6.99-7.04 (m, 1H), 7.14-7.22 (m, 1H), 7.46-7.58 (m, 1H), 7.93 (s, 1H), 8.19-8.33 (m, 1H), 9.18-9.29 (m, 1H).

### Step 5: Compound 7 tert-butyl 1⁵-chloro-2,5,11-triaza-1(2,4)-pyrimidina-3(1,3)-benzenacycloundecaphan-5-carboxylate

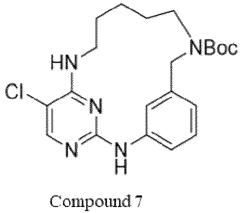

The synthesis of this step referred to the synthesis process of the first step of compound 4 in Example 2.

LC_MS: (ES⁺): m/z 418.2 [M+H] ⁺.

¹H NMR (400 MHz, CDCl₃): δ 0.77-1.10 (m, 9H), 1.61-1.72 (m, 6H), 3.12-3.26 (m, 2H), 3.52-3.60 (m, 2H), 4.39-4.45 (m, 2H), 5.03-5.54 (m, 1H), 6.78-6.81 (m, 1H), 7.05-7.13 (m, 2H), 7.24-7.28 (m, 1H), 7.87-7.89 (m, 1H), 7.28 (s, 1H).

### Step 6: Compound 8 1⁵ -chloro-2,5,11-triaza-1(2,4)-pyrimidina-3(1,3)-benzenacyclodecaphane

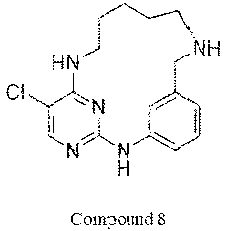

A solution of **compound 7** tert-butyl 1⁵-chloro-2,5,11-triaza-1(2,4)-pyrimidina-3(1,3)-benzenacycloundecaphan-5-carboxylate (50 mg, 0.12 mmol) and trifluoroacetic acid (1 mL) in dichloromethane (1 mL) was stirred for 3 hours under nitrogen atmosphere at 0 °C. The reaction was monitored by TLC to be completed. The reaction solution was adjusted to pH = 7-8 with saturated sodium carbonate and then extracted with dichloromethane (10 mLx 2). The organic layers were combined, washed with saturated brine (20 mL), dried over anhydrous sodium sulfate, and concentrated under reduced pressure. The residue was separated and purified by Pre-TLC (eluted with dichloromethane containing 10% methanol) to obtain white solid **compound 8** 1⁵-chloro-2,5,11-triaza-1(2,4)-pyrimidina-3(1,3)-benzenacyclodecaphane (35 mg).

LC_MS: (ES⁺): m/z 318.1 [M+H] ⁺

¹H NMR (400 MHz, DMSO-d6): δ 1.50-1.57 (m, 4H), 1.78 (s, 2H), 2.70-2.74 (m, 2H), 3.39-3.48 (m, 3H), 4.01 (s, 2H), 6.99 (d, *J* = 7.6 Hz, 1H), 7.12-7.14 (m, 1H), 7.27 (t, *J* = 7.8 Hz, 1H), 7.38 (t, *J* = 5.8 Hz, 1H), 7.92 (s, 1H), 8.37 (s, 1H), 9.33 (s, 1H).

### Step 7: Compound 9-H tert-butyl (2-(1⁵-chloro-2,5,11-triaza-1(2,4)-pyrimidina-3(1,3)-benzenacycloundecaphan-5-yl)-2-oxoethyl) carbamate

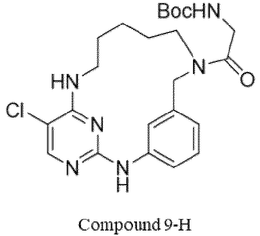

N,N,N,N-tetramethyl-O-(7-azabenzotriazol-1-yl)uronium hexafluorophosphate (91.3 mg, 0.24 mmol) was added to a solution of **compound 8** 1⁵-chloro-2,5,11-triaza-1(2,4)-pyrimidina-3(1,3)-benzenacyclodecaphane (50 mg, 0.1 mmol), **compound 9-G** 2-((tert-butoxycarbonyl)amino)acetic acid (21 mg, 0.12 mmol) and N,N-diisopropylethylamine (61.9 mg, 0.48 mmol) in N,N-dimethylformamide (2 mL) at 0 °C, and the reaction solution was stirred for 2 hours at room temperature. The reaction was monitored by TLC to be completed. The reaction solution was diluted with water (20 mL) and extracted with ethyl acetate (20 mL x 2). The organic layers were combined, washed with saturated brine (20 mL), dried over anhydrous sodium sulfate, and concentrated under reduced pressure. The residue was separated and purified by Pre-TLC (eluted with dichloromethane containing 3% methanol) to obtain an off-white solid **compound 9-H** tert-butyl (2-(1⁵-chloro-2,5,11-triaza-1(2,4)-pyrimidina-3(1,3)-benzenacycloundecaphan-5-yl)-2-oxoethyl) carbamate (30 mg).

### Step 8: Compound 9 2-amino-1-(1⁵-chloro-2,5,11-triaza-1(2,4)-pyrimidina-3(1,3)-benzenacycloundecaphan-5-yl)ethan -1-one

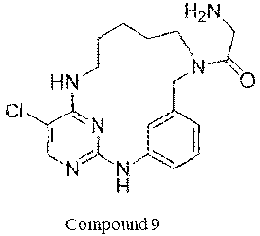

A solution of **compound 9-H** tert-butyl (2-(1⁵-chloro-2,5,11-triaza-1(2,4)-pyrimidina-3(1,3)-benzenacycloundecaphan-5-yl)-2-oxoethyl)c arbamate (30 mg, 0.06 mmol) and trifluoroacetic acid (1 mL) in dichloromethane (1 mL) was stirred at 0 °C for 2 hours. The reaction was monitored by TLC to be completed. The reaction solution was adjusted to pH = 7-8 with saturated sodium carbonate and then extracted with dichloromethane (10 mLx 2). The organic layers were combined, washed with saturated brine (20 mL), dried over anhydrous sodium sulfate, and concentrated under reduced pressure. The residue was separated and purified by Pre-TLC (eluted with dichloromethane containing 10% methanol) and HPLC to obtain an off-white solid **compound 9** 2-amino-1-(1⁵-chloro-2,5,11-triaza-1(2,4)-pyrimidina-3(1,3)-benzenacycloundecaphan-5-yl)ethan -1-one (22.1 mg).

LC_MS: (ES⁺): m/z 375.1 [M+H] ⁺

¹H NMR (400 MHz, DMSO-*d*6): δ 0.74-0.85 (m, 4H), 1.54-1.73 (m, 4H), 3.09-3.17 (m, 2H), 3.34-3.48 (m, 4H), 4.36-4.52 (m, 2H), 6.83-6.94 (m, 1H), 7.03-7.08 (m, 1H), 7.16-7.24 (m, 1H), 7.34-7.40 (m, 1H), 7.91 (s, 1H), 8.35-8.48 (m, 1H), 9.32-9.40 (m, 1H).

### Example 6: Synthesis route of compound 10

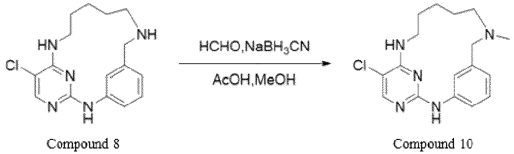

### Compound 10 1⁵-chloro-5-methyl-2,5,11-triaza-1(2,4)-pyrimidina-3(1,3)-benzene cycloundecane

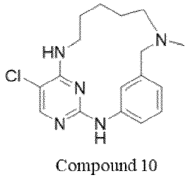

A drop of acetic acid solution was added to a solution of **compound 8** 1⁵-chloro-2,5,11-triaza-1(2,4)-pyrimidina-3(1,3)-benzenacyclodecaphane (8 mg, 0.0252 mmol) and formaldehyde (3.78 mg, 0.126 mmol) in methanol (1 ml). The reaction solution was stirred at room temperature for 30 minutes, and sodium cyanoborohydride (2.51 mg, 004 mmol) was added. The reaction mixture was further stirred at room temperature for 14 hours. The reaction was monitored by TLC to be completed. The reaction solution was concentrated. The residue was separated and purified by preparative-TLC (eluted with dichloromethane containing 9.09% methanol) to obtain a brown solid **compound** 10 1⁵-chloro-5-methyl-2,5,11-triaza-1(2,4)-pyrimidina-3(1,3)-benzenacyclodecaphane (5.1 mg).

LC_MS: (ES⁺): m/z 332.2 [M+Na] ⁺

### Example 7: Synthesis route of compound 11

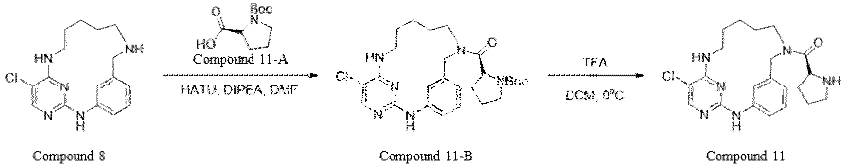

### Step 1: Compound 11-B tert-butyl (R)-2-(1⁵-chloro-2,5,11-triaza-1(2,4)-pyrimidina-3(1,3)-benzenacycloundecaphan-5-carbonyl) pyrrolidin-1-carboxylate

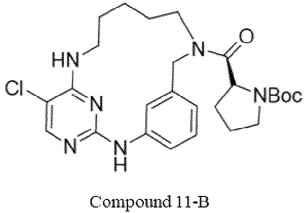

The synthesis of this step referred to the synthesis process of the seventh step of **compound 9 in Example 5.**

LC_MS: (ES⁺): m/z 515.3 [M+H] ⁺.

### Step 2: Compound 11 (R)-1⁵-chloro-5-prolyl-2,5,11-triaza-1(2,4)-pyrimidina-3(1,3)-benzenacyclodecaphane

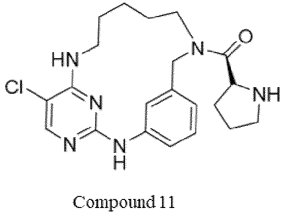

The synthesis of this step referred to the synthesis process of the eighth step of **compound 9 in Example 5.**

LC_MS: (ES⁺): m/z 415.4 [M+H] ⁺

¹H NMR (400 MHz, DMSO-d6): δ 1.41-1.54 (m, 4H), 1.66-1.82 (m, 4H), 1.95-2.15 (m, 2H), 2.83-3.02 (m, 2H), 3.10-3.19 (m, 2H), 3.38-3.56 (m, 4H), 4.34-4.43 (m, 1H), 4.61-4.72 (m, 1H), 6.83-6.96 (m, 1H), 7.04-7.11 (m, 1H), 7.18-7.23 (m, 1H), 7.36-7.39 (m, 1H), 7.90-7.96 (m, 1H), 8.39-8.44 (m, 1H), 9.34 (s, 1H).

### Example 8: Synthesis route of compound 12

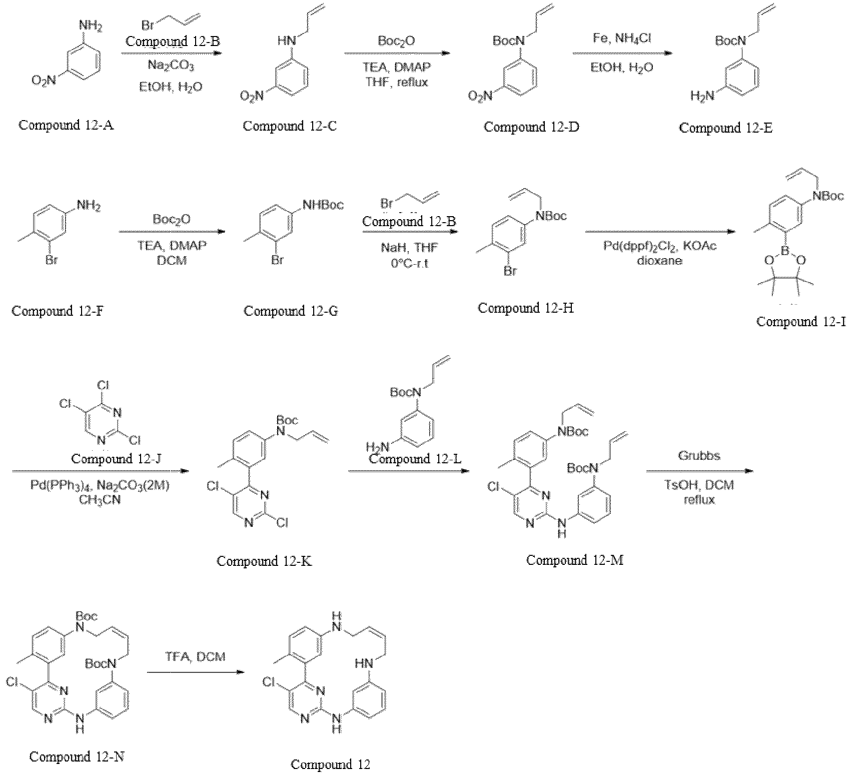

### Step 1: Compound 12-C N-allyl-3-nitroaniline

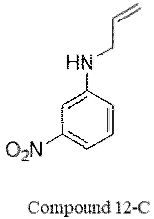

A solution of **compound 12-A** 3-nitroaniline (1 g, 7.2 mmol), **compound 12-B** 3-bromopropylene (1.3 g, 10.8 mmol) and sodium carbonate (768 mg, 7.2 mmol) in ethanol (4 mL)-water (1 mL) was refluxed for 4 hours. The reaction was monitored by TLC to be completed. The reaction solution was cooled to room temperature and distributed between water (20 mL) and ethyl acetate (20 mL). The organic layer was collected and the aqueous layer was extracted with ethyl acetate (20 mL x 2). The organic layers were combined, washed with saturated brine (30 mL), dried over anhydrous sodium sulfate, and concentrated under reduced pressure. The obtained crude product was separated and purified by silica gel column chromatography (eluted with a hexane solution containing 1% ethyl acetate) to obtain a yellow solid **compound 12-C** N-allyl-3-nitroaniline (660 mg).

LC_MS: (ES⁺): m/z 179.00 [M+H] ⁺

¹H NMR (400 MHz, CDCl₃): δ 3.82-3.86 (m, 2H), 4.16 (s, 1H), 5.20-5.33 (m, 2H), 5.89-5.97 (m, 1H), 6.86-6.89 (m, 1H), 7.27 (t, *J =* 8.0 Hz, 1H), 7.40 (t, *J =* 2.0 Hz, 1H), 7.51-7.54 (m, 1H).

### Step 2: Compound 12-D tert-butyl allyl(3-nitrophenyl)carbamate

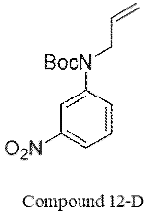

A solution of **compound 12-C** N-allyl-3-nitroaniline (600 mg, 3.4 mmol), triethylamine (477 mg, 4.7 mmol), 4-dimethylaminopyridine (40 mg, 0.3 mmol) and di-tert-butyl dicarbonate (1.1 g, 5.1 mmol) in anhydrous tetrahydrofuran (10 mL) was stirred at 60°C for 8 hours. The reaction was monitored by TLC to be completed. The reaction solution was quenched with water (20 mL) and extracted with ethyl acetate (10 mL x 2). The organic layers were combined, washed with saturated brine (20 mL), dried over anhydrous sodium sulfate, and concentrated under reduced pressure. The obtained crude product was separated and purified by silica gel column chromatography (eluted with a hexane solution containing 3.3% ethyl acetate) to obtain a yellow solid **compound 12**-D tert-butyl allyl(3-nitrophenyl)carbamate (800 mg).

¹H NMR (400 MHz, CDCl₃): δ 1.48 (s, 9H), 4.29-4.30 (m, 2H), 5.16-5.22 (m, 2H), 5.87-5.97 (m, 2H), 7.47 (t, *J* = 8.0 Hz, 1H), 7.61 (d, *J* = 7.6 Hz, 1H), 8.01-8.03 (m, 1H), 8.16 (t, *J* = 2.0 Hz, 1H).

### Step 3: Compound 12-E tert-butyl allyl(3-aminophenyl)carbamate

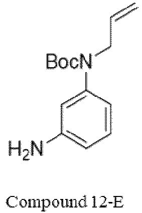

The synthesis of this step referred to the synthesis process of the fourth step of **compound 2 in Example 1.**

LC_MS: (ES⁺): m/z 193.30 [M+H-56] ⁺

¹H NMR (400 MHz, CDCl₃): δ 1.45 (s, 9H), 4.16-4.18 (m, 2H), 5.11-5.18 (m, 2H), 5.87-5.94 (m, 1H), 6.49-6.52 (m, 1H), 6.58-6.62 (m, 2H), 7.08 (t, *J* = 8.0 Hz, 1H).

### Step 4: Compound 12-G tert-butyl (3-bromo-4-methylphenyl)carbamate

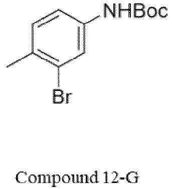

A solution of **compound 12-F** 3-bromo-4-methylaniline (5 g, 27 mmol), triethylamine (3.03 g, 30 mmol), 4-dimethylaminopyridine (329 mg, 2.7 mmol) and di-tert-butyl dicarbonate (5.9 g, 27 mmol) in dichloromethane (20 mL) was stirred at room temperature for overnight reaction. The reaction was monitored by TLC to be completed. The reaction solution was distributed between dichloromethane (40 mL) and water (40 mL). The organic layer was collected, washed with saturated brine (30 mL), dried over anhydrous sodium sulfate, and concentrated under reduced pressure. The crude product was separated and purified by silica gel column chromatography (eluted with n-hexane solution containing 1.4% ethyl acetate) to obtain a white solid **compound 12-G** tert-butyl (3-bromo-4-methylphenyl)carbamate (5.3 g).

LC_MS: (ES⁺): m/z 229.85 [M+H-56] ⁺

### Step 5: Compound 12-H tert-butyl allyl(3-bromo-4-methylphenyl)carbamate

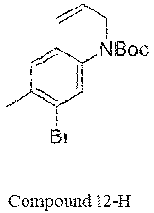

Sodium hydride (60% mass fraction in kerosene, 280 mg, 7.0 mmol) was added to a solution of compound 12-G tert-butyl (3-bromo-4-methylphenyl) carbamate (1 g, 3.5 mmol) in tetrahydrofuran (5 ml) at 0°C. The reaction solution was warmed to room temperature, reacted for 30 minutes under stirring, then cooled to 0 °C and added with **compound 12-B** 3-bromopropene (635 mg, 5.3 mmol) dropwise. The reaction solution was stirred for 14 hours under nitrogen atmosphere at room temperature. The reaction was monitored by TLC to be completed. The reaction solution was distributed between ethyl acetate (30 mL) and ice water (20 mL). The organic layer was collected, washed with saturated brine (20 mL), dried over anhydrous sodium sulfate, and concentrated under reduced pressure. The residue was separated and purified by silica gel column chromatography (eluted with a hexane solution containing 1% ethyl acetate) to obtain a light yellow oily **compound 12-H** tert-butyl allyl(3-bromo-4-methylphenyl)carbamate (874 mg).

LC_MS: (ES⁺): m/z 269.85 [M+H-56] ⁺

¹H NMR (400 MHz, CDCl₃): δ 1.44 (s, 9H), 2.36 (s, 3H), 4.17-1.49 (m, 2H), 5.12-5.16 (m, 2H), 5.83-5.92 (m, 1H), 7.06-7.08 (m, 1H), 7.14-7.16 (m, 1H), 7.41-7.42 (m, 1H).

### Step 6: Compound 12-I tert-butyl allyl(4-methyl-3-(4,4,5,5-tetramethyl-1,3,2-dioxaborolan-2-yl)phenyl)carbamate

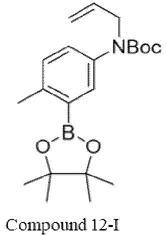

Under a nitrogen atmosphere at room temperature, PdCl₂(dppf) (174 mg, 0.24 mmol) was added to a solution of **compound 12-H** tert-butyl allyl (3-bromo-4-methylphenyl) carbamate (774 mg, 2.38 mmol), bis-pinacolatodiboron (907 mg, 3.57 mmol), and potassium acetate (467 mg, 4.76 mmol) in 1,4-dioxane (5 mL). The reaction solution was replaced with nitrogen three times and stirred at 90 °C for 5 hours. The reaction was monitored by TLC to be completed. The reaction solution was filtered and the filtrate was concentrated under reduced pressure. The crude product was separated and purified by silica gel column chromatography (eluted with a hexane solution containing 2.5% ethyl acetate) to obtain a light yellow oily **compound 12-I** tert-butyl allyl(4-methyl-3-(4,4,5,5-tetramethyl-1,3,2-dioxaborolan-2-yl)phenyl)carbamate (745 mg).

LC_MS: (ES⁺): m/z 318.00 [M+H-56] ⁺

¹H NMR (400 MHz, CDCl₃): δ 1.33 (s, 12H), 1.43 (s, 9H), 2.50 (s, 3H), 4.20 (d, *J* = 3.2 Hz, 2H), 5.09-5.16 (m, 2H), 5.85-5.94 (m, 1H), 7.08-7.16 (m, 2H), 7.57 (d, *J* = 2.4 Hz, 1H).

### Step 7: Compound 12-K tert-butyl allyl(3-(2,5-dichloropyrimidin-4-yl)-4-methylphenyl)carbamate

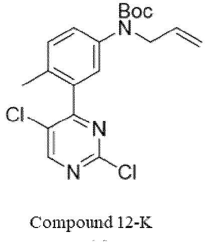

In a sealed tube at room temperature under nitrogen atmosphere, tetrakis(triphenylphosphine)palladium (231 mg, 0.2 mmol) was added to a suspension of **compound 12-1** tert-butyl allyl(4-methyl-3-(4,4,5,5-tetramethyl-1,3,2-dioxaborolan-2-yl)phenyl)carbamate (745 mg, 2.0 mmol), **compound 12-J** 2,4,5-trichloropyrimidine (727 mg, 4.0 mmol), and sodium carbonate (2N, 2 mL, 4.0 mmol) in acetonitrile. The reaction solution was replaced with nitrogen three times and stirred at 70 °C for two days of reaction. The reaction was monitored by TLC to be completed. The reaction solution was distributed between ethyl acetate (20 mL) and water (20 mL). The organic layer was collected, washed with saturated brine (20 mL), dried over anhydrous sodium sulfate, and concentrated under reduced pressure. The obtained crude product was separated and purified by silica gel column chromatography (eluted with a hexane solution containing 2% ethyl acetate) to obtain a light yellow solid **compound 12-K** tert-butyl allyl(3-(2,5-dichloropyrimidin-4-yl)-4-methylphenyl)carbamate (347 mg).

LC_MS: (ES⁺): m/z 337.9 [M+H-56] ⁺

¹H NMR (400 MHz, CDCl₃): δ 1.44 (s, 9H), 2.19 (s, 3H), 4.22 (d, *J* = 6.8 Hz, 2H), 5.12-5.17 (m, 2H), 5.87-5.94 (m, 1H), 7.15 (s, 1H), 7.25 (s, 2H), 8.68 (s, 1H).

### Step 8: Compound 12-M tert-butyl allyl(3-(2-((3-(allyl(tert-butoxycarbonyl)amino)phenyl)amino)-5-chloropyrimidin-4-yl)-4-methyl phenyl)carbamate

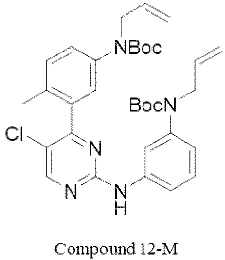

The synthesis of this step referred to the synthesis process of the sixth step of compound 2 in Example 1.

LC_MS: (ES⁺): m/z 606.3 [M+H] ⁺

¹H NMR (400 MHz, CDCl₃): δ 1.43 (m, 18H), 2.22 (s, 3H), 4.20-4.24 (m, 4H), 5.06-5.18 (m, 4H), 5.83-5.96 (m, 2H), 6.93 (d, *J =* 8.0 Hz, 1H), 7.14 (s, 1H), 7.22-7.24 (m, 3H), 7.37-7.40 (m, 1H), 7.59 (t, *J* = 2.0 Hz, 1H) , 8.44 (s, 1H).

### Step 9: Compound 12-N di-tert-butyl (Z)-2⁵-chloro-1⁶-methyl-3,5,10-triaza-2(4,2)-pyrimidina-1,4(1,3)-dibenzenacyclodecaphan-7-en-5,10-dicarboxylate

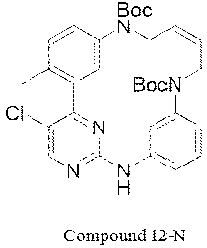

The synthesis of this step referred to the synthesis process of the seventh step of **compound 2 in Example 1.**

LC_MS: (ES⁺): m/z 578.3 [M+H] ⁺

¹H NMR (400 MHz, DMSO-d6): δ 1.30 (s, 9H), 1.39 (s, 9H), 2.22 (s, 3H), 3.79-3.84 (m, 1H) , 4.04-4.25 (m, 2H) , 4.41-4.45 (m, 1H) , 5.59-5.60 (m, 2H) , 6.81 (d, *J* = 7.6 Hz, 1H), 7.03-7.06 (m, 1H) , 7.21 (t, *J* = 8.4 Hz, 2H), 7.28 (d, *J* = 8.4 Hz, 1H), 7.44-7.46 (m, 1H) , 8.21-8.22 (m, 1H) , 8.63 (s, 1H) , 10.08 (s, 1H).

### Step 10: Compound 12 (Z)-2⁵-chloro-1⁶-methyl-3,5,10-triaza-2(4,2)-pyrimidina-1,4(1,3)-dibenzocyclodecane-7-ene

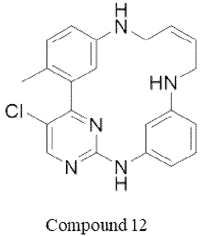

The synthesis of this step referred to the synthesis process of the sixth step of **compound 8 in Example 5.**

LC_MS: (ES⁺): m/z 378.3 [M+H] ⁺

¹H NMR (400 MHz, CDCl₃): δ 2.25 (s, 3H) , 3.68-3.91 (m, 4H), 4.10 (s, 2H) , 5.58 (d, *J* = 2.4 Hz, 2H), 6.23-6.32 (m, 2H), 6.65-6.72 (m, 2), 7.00-7.05 (m, 2H), 7.17 (s, 1H), 7.86 (t, *J* = 2.0 Hz, 1H), 8.34 (s, 1H).

### Example 9: Synthesis route of compound 13, compound 14, compound 15, and compound 16

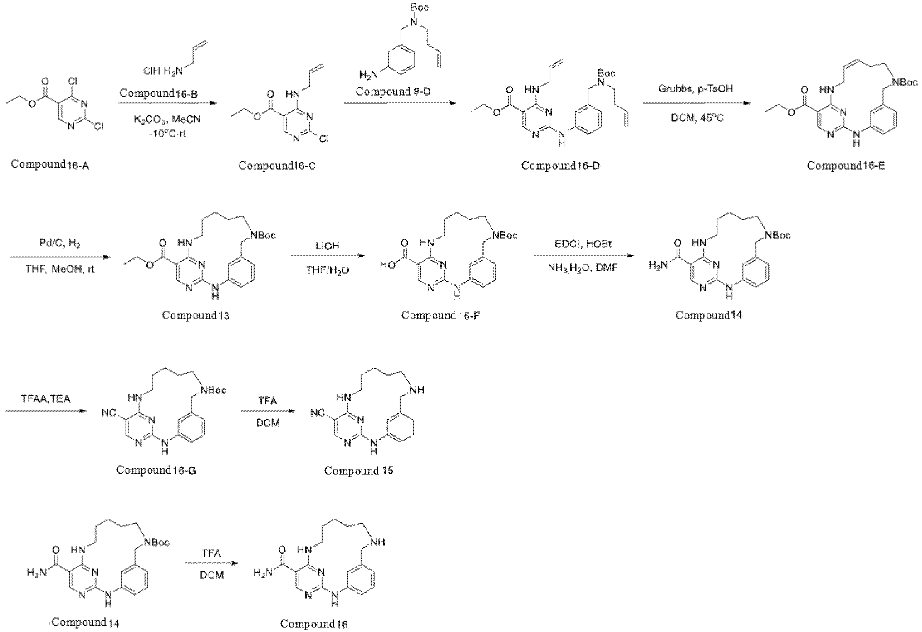

### Step 1: Compound 16-C ethyl 4-(allylamino)-2-chloropyrimidin-5-carboxylate

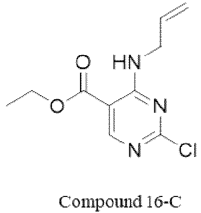

**Compound 16-B** allylamine hydrochloride (790 mg, 4.3 mmol) was added to a solution of **compound 16-A** ethyl 2,4-dichloropyrimidin-5-carboxylate (1 g, 4.53 mmol) and potassium carbonate (1.3 g, 9.42 mmol) in acetonitrile (8 mL) at -10 °C. The reaction solution was stirred at room temperature for 5 hours. The reaction was monitored by TLC to be completed. The reaction solution was distributed between ethyl acetate (20 mL) and water (20 mL). The organic layer was collected, washed with saturated brine (20 mL), dried over anhydrous sodium sulfate, and concentrated under reduced pressure. The crude product was separated and purified by silica gel column chromatography (eluted with a hexane solution containing 5% ethyl acetate) to obtain a white solid **compound 16-C** ethyl 4-(allylamino)-2-chloropyrimidin-5-carboxylate (860 mg).

LC_MS: (ES⁺): m/z 241.9 [M+H] ⁺

¹H NMR (400 MHz, CDCl₃): δ 1.38-1.41 (t, *J* = 7.2 Hz, 3H), 4.18-4.20 (t, *J* = 5.6 Hz, 2H), 4.34-4.39 (q, *J* = 6.8 Hz, 2H), 5.20-5.29 (m, 2H), 5.90-5.99 (m, 1H), 8.48 (brs, 1H), 8.68 (s, 1H).

### Step 2: Compound 16-D 4-(allylamino)-2-((3-((but-3-en-1-yl(tert-butyloxycarbonyl)amino)methyl)phenyl)amino)pyrimidi ne-5-carboxylate

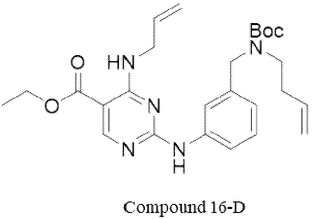

The synthesis of this step referred to the synthesis process of the sixth step of **compound 2 in Example 1.**

LC_MS: (ES⁺): m/z 482.7 [M+H] ⁺

### Step 3: Compound 16-E 5-(tert-butyl) 1⁵-ethyl(Z)-2,5,11-triaza-1(2,4)-pyrimidina-3(1,3)-benzenacycloundecaphan-8-en-15,5-dicarboxy late

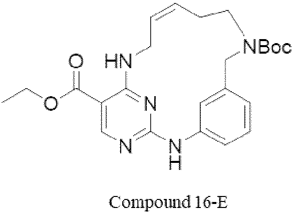

The synthesis of this step referred to the synthesis process of the seventh step of **compound 2 in Example 1.**

LC_MS: (ES⁺): m/z 454.4 [M+H] ⁺

### Step 4: Compound 13 5-(tert-butyl) 1⁵-ethyl-2,5,11-triaza-1(2,4)-pyrimidina-3(1,3)-benzenacycloundecaphan-1⁵,5-dicarboxylate

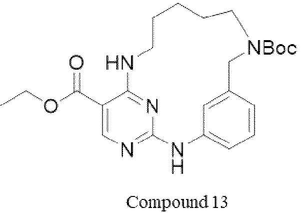

The synthesis of this step referred to the synthesis process of the first step of compound **4 in Example 2.**

LC_MS: (ES⁺): m/z 456.4 [M+H] ⁺

¹H NMR (400 MHz, CDCl₃): δ 1.35-1.39 (t, *J* = 7.2 Hz, 3H), 1.47-1.54 (m, 9H), 1.64-1.70 (m, 4H), 1.80-2.09 (m, 2H), 3.12-3.26 (m, 2H), 3.55-3.58 (m, 2H), 4.28-4.33 (q, *J =* 6.8 Hz, 2H), 4.40-4.46 (d, *J* = 25.2 Hz, 2H) , 6.84-6.86 (d, *J* = 8.4 Hz, 1H) , 7.10-7.31 (m, 1H), 7.29-7.31 (m, 1H), 7.52-7.55 (m, 1H) ,8.37-8.39 (d, *J* = 7.6 Hz, 1H), 8.55-8.56 (m, 1H), 8.62 (brs, 1H).

### Step 5: Compound 16-F 5-(tert-butyloxycarbonyl)-2,5,11-triaza-1(2,4)-pyrimidina-3(1,3)-benzenacycloundecaphan-1⁵-car boxylic acid

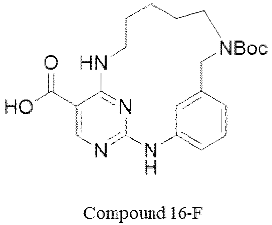

A solution of **compound 13** 5-(tert-butyl) 1⁵ -ethyl-2,5,11-triaza-1(2,4)-pyrimidina-3(1,3)-benzenacycloundecaphan-1⁵,5-dicarboxylate (220 mg, 0.48 mmol) and lithium hydroxide monohydrate (41 mg, 0.98 mmol) in tetrahydrofuran (6 mL)-water (1.5 mL)-methanol (1.5 mL) was stirred at 70 °C for 13 hours. The reaction was monitored by TLC to be completed. The reaction solution was cooled to room temperature, adjusted to pH=6 with citric acid, and extracted with ethyl acetate (20 mLx3). The organic layers were combined, washed with saturated brine (40 mL), dried over anhydrous sodium sulfate, and concentrated under reduced pressure to obtain a crude **compound 16-F** 5-(tert-butyloxycarbonyl)-2,5,11-triaza-1(2,4)-pyrimidina-3(1,3)-benzenacycloundecaphan-1⁵-car boxylic acid (200 mg), which was used directly in the next step without further purification.

LC_MS: (ES⁺): m/z 428.4 [M+H] ⁺.

### Step 6: Compound 14 tert-butyl 1⁵-carbamoyl-2,5,11-triaza-1(2,4)-pyrimidina-3(1,3)-benzenacycloundecaphan-5-carboxylate

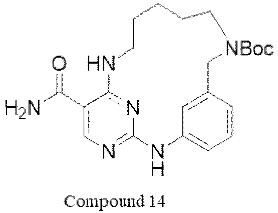

1-ethyl-3-(3-dimethylaminopropyl)carbodiimide hydrochloride (102 mg, 0.53 mmol) and N-hydroxybenzotriazole (72 mg, 0.53 mmol) were added to a solution of **compound 16-F** 5-(tert-butyloxycarbonyl)-2,5,11-triaza-1(2,4)-pyrimidina-3(1,3)-benzenacycloundecaphan-1⁵-car boxylic acid (200 mg, 0.47 mmol) in N,N-dimethylformamide (10 mL) at 0 °C, and stirred for 45 minutes. Then, the reaction solution was added with ammonia water (1 mL) at 0 °C. The reaction solution was stirred at 40 °C for two days. The reaction was monitored by TLC to be completed. The reaction solution was cooled to room temperature and distributed between saturated sodium carbonate aqueous solution (20 mL) and ethyl acetate (10 ml). The organic layer was collected and the aqueous layer was extracted with ethyl acetate (10 mL x 2). The organic layers were combined, washed with saturated brine (20 mL), dried over anhydrous sodium sulfate, and concentrated under reduced pressure. The crude product was separated and purified by Pre-TLC (eluted with dichloromethane containing 5% methanol) to obtain a white solid **compound 14** tert-butyl 1⁵-carbamoyl-2,5,11-triaza-1(2,4)-pyrimidina-3(1,3)-benzenacycloundecaphan-5-carboxylate (65 mg).

LC_MS: (ES⁺): m/z 427.4 [M+H] ⁺

¹H NMR (400 MHz, CDCl₃): δ 1.47-1.54 (d, *J =* 28.8 Hz, 9H), 1.63-1.72 (m, 4H), 1.76 (brs, 2H), 3.11-3.25 (m, 2H), 3.49-3.57 (m, 2H), 4.40-4.46 (d, *J =* 24.8 Hz, 2H), 5.62 (brs, 2H) , 6.82-6.84 (d, *J* = 8.0 Hz, 1H) ,7.10-7.18 (m, 1H), 7.28-7.30 (m, 1H), 7.45 (brs, 1H), 8.26 (s, 1H) ,8.36-8.39 (d, *J =* 11.6 Hz, 1H), 9.11-9.13 (m, 1H).

### Step 7: Compound 16-G tert-butyl 1⁵-cyano-2,5,11-triaza-1(2,4)-pyrimidina-3(1,3)-benzenacycloundecaphan-5-carboxylate

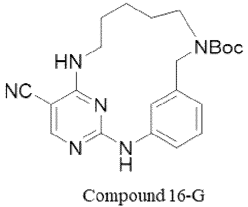

Trifluoroacetic anhydride (201 mg, 0.96 mmol) was added to a solution of **compound 14** tert-butyl 1⁵-carbamoyl-2,5,11-triaza-1(2,4)-pyrimidina-3(1,3)-benzenacycloundecaphan-5-carboxylate (37 mg, 0.087 mmol) and triethylamine (111 mg, 1.09 mmol) in anhydrous tetrahydrofuran (15 mL) at -50 °C under a nitrogen atmosphere. The reaction solution was stirred at -50 °C for 20 minutes, then heated to 0 °C and stirred for 2 hours. The reaction was monitored by TLC to be completed. The reaction solution was distributed between ethyl acetate (20 mL) and saturated sodium carbonate aqueous solution (20 mL). The organic layer was collected, washed with saturated brine (20 mL), dried over anhydrous sodium sulfate, and concentrated under reduced pressure. The residue was subjected to Pre-TLC (eluted with dichloromethane containing 7.5% methanol) to obtain a white solid **compound 16-G** tert-butyl 1⁵-cyano-2,5,11-triaza-1(2,4)-pyrimidina-3(1,3)-benzenacycloundecaphan-5-carboxylate (30 mg).

LC_MS: (ES⁺): m/z 409.4 [M+H] ⁺

### Step 8: Compound 15 2,5,11-triaza-1(2,4)-pyrimidina-3(1,3)-benzenacycloundecaphan-1⁵-carbonitrile

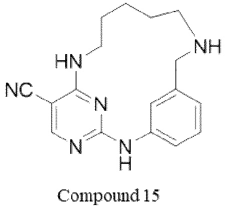

The synthesis of this step referred to the synthesis process of the sixth step of **compound 8 in Example 5.**

LC_MS: (ES⁺): m/z 309.0 [M+H] ⁺

¹H NMR (400 MHz, DMSO-d6): δ 1.46 (brs, 2H), 1.61 (brs, 4H), 2.55-2.59 (t, *J* = 7.6 Hz, 2H), 3.40-3.43 (m, 2H), 3.83 (s, 2H), 6.69 (brs, 1H), 6.96-6.98 (d, *J=* 7.6 Hz, 1H), 7.10-7.12 (d, *J = 8.0* Hz, 1H), 7.23-7.27 (d, *J* = 7.6 Hz, 1H), 7.86-7.89 (m, 1H), 8.32-8.34 (m, 2H), 9.86 (s, 1H).

### Step 9: Compound 16 2,5,11-triaza-1(2,4)-pyrimidina-3(1,3)-benzenacycloundecaphan-1⁵-carboxamide

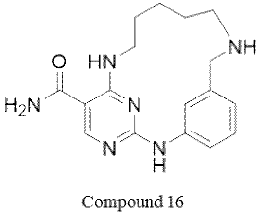

The synthesis of this step referred to the synthesis process of the sixth step of **compound 8 in Example 5.**

LC_MS: (ES⁺): m/z 327.2 [M+H] ⁺

### Example 10: Synthesis route of compound 17 and compound 18

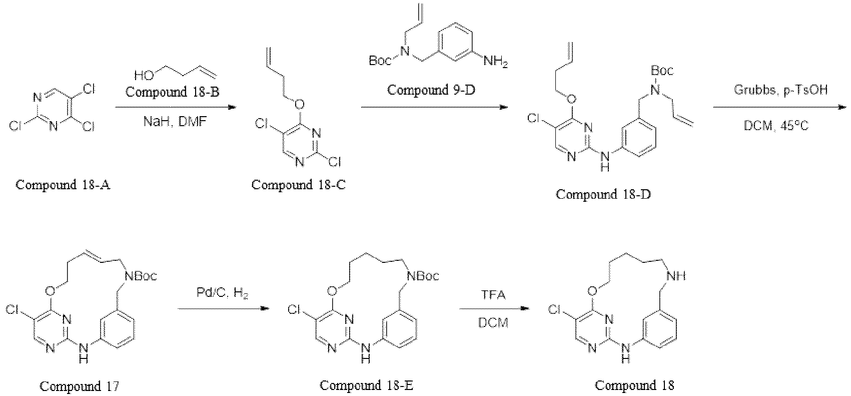

### Step 1: Compound 18-C 4-(but-3-en-1-acyloxy)-2,5-dichloropyrimidine

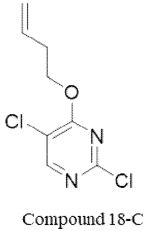

Sodium hydride (60% mass fraction in kerosene, 481 mg, 12.0 mmol) was added to a solution of **compound 18-B** but-3-en-1-ol (787 mg, 10.9 mmol) in N,N-dimethylformamide (10 mL) at 0 °C. The reaction solution was warmed to room temperature, reacted for 1 hour under stirring, then cooled to 0 °C, and added with a solution of **compound 18-A** 2,4,5-trichloropyrimidine (2 g, 10.9 mmol) in N,N-dimethylformamide (5 mL). The reaction solution was stirred for 15 hours under nitrogen atmosphere at room temperature. The reaction was monitored by TLC to be completed. The reaction solution was distributed between ethyl acetate (50 mL) and ice water (50 mL). The organic layer was collected, washed with saturated brine (30 mL), dried over anhydrous sodium sulfate, and concentrated under reduced pressure. The crude product was subjected to silica gel column chromatography (eluted with 0.3% ethyl acetate in n-hexane) to obtain a colorless oily **compound 18-C** 4-(but-3-en-1-acyloxy)-2,5-dichloropyrimidine (1.35 g).

LC_MS: (ES⁺): m/z 218.8 [M+H] ⁺

### Step 2: Compound 18-D tert-butyl ally (3-((4-(but-3-en-1-yloxy)-5-chloropyrimidin-2-yl)amino)phenyl)carbamate

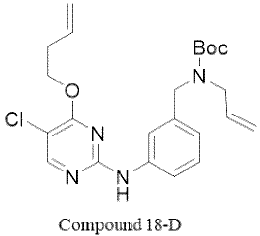

The synthesis of this step referred to the synthesis process of the sixth step of **compound 2 in Example 1.**

LC_MS: (ES⁺): m/z 445.0 [M+H] ⁺

### Step 3: Compound 17 tert-butyl (Z)-1⁵-chloro-11-oxa-2,5-diaza-1(2,4)-pyrimidina-3(1,3)-benzenacycloundecaphan-7-en-5-carbo xylate

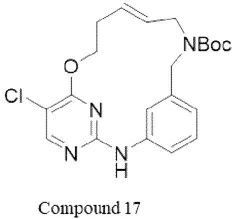

The synthesis of this step referred to the synthesis process of the seventh step of **compound 2 in Example 1.**

LC_MS: (ES⁺): m/z 417.2 [M+H] ⁺

¹H NMR (400 MHz, DMSO-d6): δ 1.41-1.48 (m, 9H), 2.66-2.67 (m, 2H), 3.70-3.81 (m, 2H), 4.17-4.30 (m, 2H) , 4.50-4.67 (m, 2H) , 5.38-5.85 (m, 2H), 6.96-7.32 (m, 3H), 8.15-8.39 (m, 2H), 9.81-10.00 (m, 1H)).

### Step 4: Compound 18-E tert-butyl 1⁵-chloro-11-oxa-2,5-diaza-1(2,4)-pyrimidina-3(1,3)-benzenacycloundecaphan-5-carboxylate

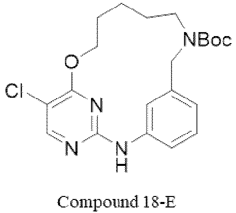

The synthesis of this step referred to the synthesis process of the first step of **compound 4 in Example 2.**

### Step 5: Compound 18 1⁵-chloro-11-oxa-2,5-diaza-1(2,4)-pyrimidina-3(1,3)-benzenacyclodecaphane

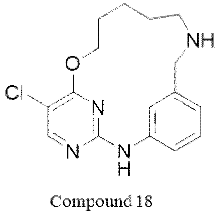

The synthesis of this step referred to the synthesis process of the sixth step of **compound 8 in Example 5.**

LC_MS: (ES⁺): m/z 319.1 [M+H] ⁺

¹H NMR (400 MHz, DMSO-d6): δ 1.49-1.60 (m, 4H), 1.80-1.85 (m, 2H), 2.56 (t, *J =* 7.2 Hz, 2H), 3.82 (s, 2H), 4.54 (t, *J =* 8.4 Hz, 2H), 6.91 (d, *J =* 7.6 Hz, 1H), 7.06 (d, *J =* 8.0 Hz, 1H), 7.23 (t, *J =* 7.6 Hz, 1H),8.24 (s, 1H), 8.30 (s, 1H), 9.78 (s, 1H).

### Example 11: Synthesis route of compound 19

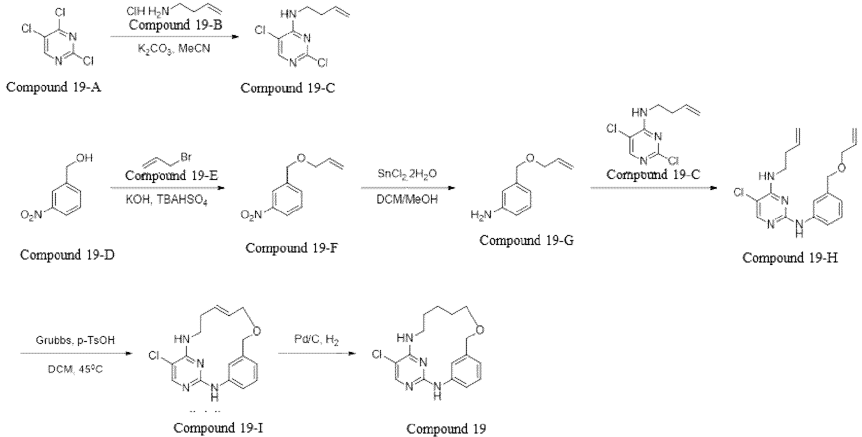

### Step 1: Compound 19-C N-(but-3-en-1-yl)-2,5-dichloropyrimidin-4-amine

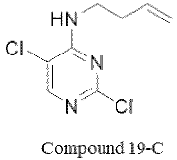

The synthesis of this step referred to the synthesis process of the fifth step of **compound 2 in Example 1.**

LC_MS: (ES⁺): m/z 217.8 [M+H] ⁺

¹H NMR (400 MHz, CDCl₃): δ 2.39-2.44 (q, *J =* 6.8 Hz, 2H), 3.58-3.62 (q, *J =* 6.2 Hz, 2H), 5.15 (s, 1H), 5.18-5.19 (d, *J =* 6.8 Hz, 1H),5.56 (brs, 1H), 5.77-5.87 (m, 1H), 8.01 (s, 1H).

### Step 2: Compound 19-F 1-((allyloxy)methyl)-3-nitrobenzene

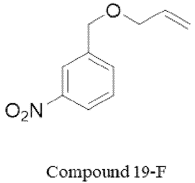

A solution of **compound 19-D** (3-nitrophenyl)methanol (1 g, 6.5 mmol), potassium hydroxide (733 mg, 13.1 mmol), and tetrabutylammonium hydrogen sulfate (111 mg, 0.32 mmol) in **compound 19-E** allyl bromide (4 mL) was stirred for 12 hours at room temperature. The reaction was monitored by TLC to be completed. The reaction solution was quenched with water (20 mL) and extracted with dichloromethane (20 mLx3). The organic layers were combined, washed with saturated brine (30 mL), dried over anhydrous sodium sulfate, and concentrated under reduced pressure. The residue was separated and purified by silica gel column chromatography (eluted with a hexane solution containing 15% ethyl acetate) to obtain a light yellow oily **compound 19-F** 1-((allyloxy)methyl)-3-nitrobenzene (1.1 g).

¹H NMR (400 MHz, CDCl₃): δ 4.09 (d, *J =* 5.6 Hz, 2H), 4.61 (s, 2H), 5.24-5.36 (m, 2H), 5.92-6.02 (m, 1H), 7.53 (t, *J =* 7.8 Hz, 1H), 7.69 (d, *J* = 7.6 Hz, 1H), 8.15 (d, *J =* 8.4 Hz, 1H), 8.23 (s, 1H).

### Step 3: Compound 19-G 3-((allyloxy)methyl)aniline

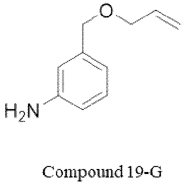

Dihydrated stannous chloride (2.34 g, 10.35 mmol) was added to a solution of **compound 19-F** 1-((allyloxy)methyl)-3-nitrobenzene (500 mg, 2.59 mmol) in methanol (5 mL)-dichloromethane (5 mL) at 0°C. The reaction mixture was slowly warmed to room temperature and stirred for 12 hours. The reaction was monitored by TLC to be completed. The reaction mixture was concentrated and the residue was distributed between dichloromethane (10 mL) and aqueous sodium carbonate solution (2N, 20 mL). The mixture was filtered through filter paper, the organic layer was separated from the mother liquor, and the aqueous layer was extracted with dichloromethane (10 mLx3). The organic layers were combined, washed with saturated brine (30 mL), dried over anhydrous sodium sulfate, and concentrated under reduced pressure to obtain a crude product, which was separated and purified by silica gel column chromatography (eluted with a hexane solution containing 33-50% ethyl acetate) to obtain a colorless oil **compound 19-G** 3-((allyloxy)methyl)aniline (355 mg).

LC_MS: (ES⁺): m/z 164.0 [M+H] ⁺

### Step 4: Compound 19-H N²-(3-((allyloxy)methyl)phenyl)-N⁴-(but-3-en-1-yl)-5-chloropyrimidin-2,4-diamine

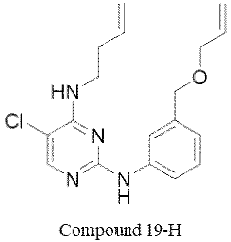

The synthesis of this step referred to the synthesis process of the sixth step of **compound 2 in Example 1.**

LC_MS: (ES⁺): m/z 345.2 [M+H] ⁺

¹H NMR (400 MHz, CDCl₃): δ 2.40-2.45 (m, 2H), 3.57-3.61 (m, 2H), 4.04 (d, *J =* 5.6 Hz, 2H), 4.52 (s, 2H), 5.13-5.22 (m, 3H), 5.29-5.34 (m, 2H), 5.79-6.01 (m, 2H), 6.97 (t, *J =* 7.6 Hz, 2H), 7.28 (t, *J* = 7.2 Hz, 1H), 7.53 (d, *J* = 8.0 Hz, 1H), 7.62 (s, 1H), 7.89 (s, 1H).

### Step 5: Compound 19-I (E)-1⁵-chloro-5-oxa-2,11-diaza-1(2,4)-pyrimidina-3(1,3)-benzenacycloundecaphan-7-ene

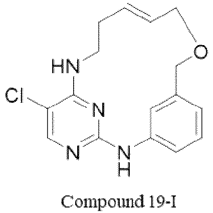

The synthesis of this step referred to the synthesis process of the seventh step of **compound 2 in Example 1.**

### Step 6: Compound 19 1⁵-chloro-5-oxa-2,11-diaza-1(2,4)-pyrimidina-3(1,3)-benzenacyclodecaphane

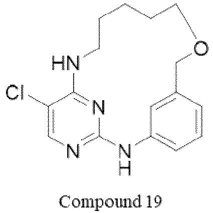

The synthesis of this step referred to the synthesis process of the first step of **compound 4 in Example 2.**

LC_MS: (ES⁺): m/z 319.3 [M+H] ⁺

¹H NMR (400 MHz, DMSO-d6): δ 1.45-1.51 (m, 2H), 1.58-1.65 (m, 2H), 1.74-1.82 (m, 2H), 3.30-3.33 (m, 2H), 3.54 (t, *J* = 5.8 Hz, 2H), 4.47 (s, 2H), 6.72 (d, *J* = 7.2 Hz, 1H), 6.98 (d, *J* = 8.4 Hz, 1H), 7.14 (t, *J =* 7.6 Hz, 1H), 7.36 (t, *J* = 6.0 Hz, 1H), 7.90 (s, 1H), 8.49 (s, 1H), 9.23 (s, 1H).

### Example 12: Synthesis route of compound 20 and compound 21

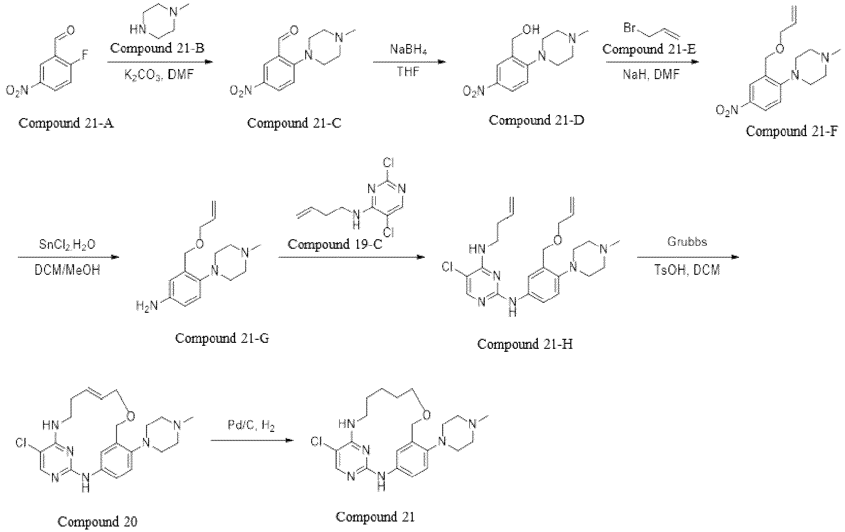

### Step 1: Compound 21-C 2-(4-methylpiperazin-1-yl)-5-nitrobenzaldehyde

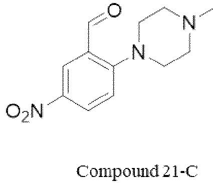

A solution of **compound 21-A** 2-fluoro-5-nitrobenzaldehyde (5 g, 29.6 mmol), potassium carbonate (6.12 g, 44.3 mmol) and **compound 21-B** N-methylpiperazine (2.96 g, 29.6 mmol) in N,N-dimethylformamide (100 mL) was stirred at 50°C for 15 hours. The reaction was monitored by TLC to be completed. The reaction solution was quenched with water (600 mL), filtered through filter paper, and the filter cake was washed with water (300 mL x 2). The filter cake was collected and dried to obtain yellow solid compound 21-C 2-(4-methylpiperazine-1-yl)-5-nitrobenzaldehyde (7 g).

LC_MS: (ES⁺): m/z 250.2 [M+H] ⁺

¹H NMR (400 MHz, CDCl₃): δ 2.40 (s, 3H), 2.64-2.66 (t, *J* = 4.8 Hz, 4H), 3.33-3.36 (t, *J =* 4.8 Hz, 4H), 7.09-7.11 (d, *J =* 9.2 Hz, 1H), 8.29-8.32 (m, 1H), 8.62-8.63 (d, *J =* 2.8 Hz, 1H), 10.10 (s, 1H).

### Step 2: Compound 21-D (2-(4-methylpiperazin-1-yl)-5-nitrophenyl)methanol

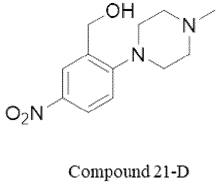

Sodium borohydride (763 mg, 20.0 mmol) was added to a solution of **compound 21-C** 2-(4-methylpiperazine-1-yl)-5-nitrobenzaldehyde (5 g, 20.0 mmol) in anhydrous tetrahydrofuran (30 mL) at 0 °C. The reaction solution was stirred at 0°C for 2 hours. The reaction was monitored by TLC to be completed. The reaction solution was diluted with water (30 mL) and extracted with dichloromethane (3 × 20 mL). The organic layers were combined, washed with saturated brine (30 mL), dried over anhydrous sodium sulfate, and concentrated under reduced pressure. The crude product was separated and purified by silica gel column chromatography (eluted with a dichloromethane solution containing 5% methanol) to obtain a yellow solid **compound 21-D** (2-(4-methylpiperazin-1-yl)-5-nitrophenyl)methanol (1.3 g).

LC_MS: (ES⁺): m/z 252.2 [M+H] ⁺

¹H NMR (400 MHz, CD₃OD): δ 2.40 (s, 3H), 2.67 (brs, 4H),3.10-3.12 (t, *J =* 4.8 Hz, 4H), 4.70 (s, 2H), 7.20-7.22 (d, *J =* 8.8 Hz, 1H), 8.12-8.15 (dd, *J* = 2.8 Hz, 1H), 8.41-8.42 (d, *J* = 3.2 Hz, 1H).

### Step 3: Compound 21-F 1-(2-((allyloxy)methyl)-4-nitrophenyl)-4-methylpiperazine

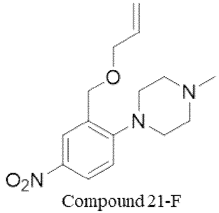

Sodium hydride (60% mass fraction in kerosene, 40 mg, 1.0 mmol) was added to a solution of **compound 21-D** (2-(4-methylpiperazine-1-yl)-5-nitrophenyl)methanol (228 mg, 0.91 mmol) in N,N-dimethylformamide (10 mL) at 0°C. The reaction solution was warmed to room temperature, stirred for 30 minutes of reaction, then cooled to 0°C and added with **compound 21-E** 3-bromopropylene (130 mg, 1.07 mmol) dropwise. The reaction solution was stirred for 15 hours under nitrogen atmosphere at room temperature. The reaction was monitored by TLC to be completed. The reaction solution was distributed between ethyl acetate (30 mL) and ice water (50 mL). The organic layer was collected, washed with saturated brine (50 mL), dried over anhydrous sodium sulfate, and concentrated under reduced pressure. The residue was separated and purified by silica gel column chromatography (eluted with a dichloromethane solution containing 5% methanol) to obtain a yellow solid **compound 21-F** 1-(2-((allyloxy)methyl)-4-nitrophenyl)-4-methylpiperazine (242 mg).

LC_MS: (ES⁺): m/z 292.2 [M+H] ⁺

### Step 4: Compound 21-G 3-((allyloxy)methyl)-4-(4-methylpiperazin-1-yl)aniline

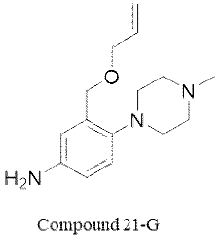

The synthesis of this step referred to the synthesis process of the third step of **compound 19 in Example 11.**

¹H NMR (400 MHz, CDCl₃): δ 2.38 (s, 3H), 2.59 (brs, 4H), 2.88-2.90 (t, *J =* 4.4 Hz, 4H), 3.49 (brs, 2H), 4.05-4.06 (d, *J* = 5.6 Hz, 2H), 5.55 (s, 2H), 5.19-5.22 (d, *J* = 10.4 Hz, 1H), 5.30-5.34 (m, 1H) , 5.92-6.02 (m, 1H) , 6.59-6.61 (dd, *J =* 2.4 Hz, 1H), 6.81-6.82 (d, *J =* 2.4 Hz, 1H), 6.96-6.98 (d, *J=* 8.4 Hz, 1H).

### Step 5: Compound 21-H N²-(3-((allyloxy)methyl)-4-(4-methylpiperazine-1-yl)phenyl)-N⁴-(but-3-en-1-yl)-5-chloropyrimid in-2,4-diamine

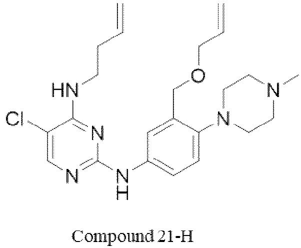

The synthesis of this step referred to the synthesis process of the sixth step of **compound 2 in Example 1.**

LC_MS: (ES⁺): m/z 443.1 [M+H] ⁺.

### Step 6: Compound 20 (E)-1⁵-chloro-3 ⁴-(4-methylpiperazine-1-yl)-5-oxa-2,11-diaza-1(2,4)-pyrimidina-3(1,3)-benzocycl oundecaphan-7-ene

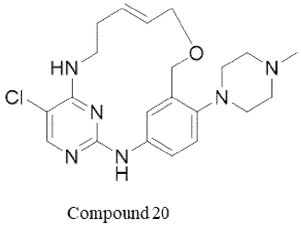

The synthesis of this step referred to the synthesis process of the seventh step of **compound 2 in Example 1.**

LC_MS: (ES⁺): m/z 415.3 [M+H] ⁺

### Step 7: Compound 21 1⁵-chloro-3⁴-(4-methylpiperazin-1-yl)-5-oxa-2,11-diaza-1(2,4)-pyrimidina-3(1,3)-benzenacyclou ndecaphane

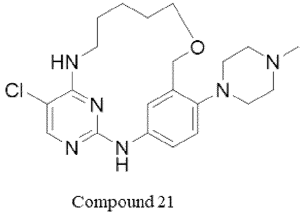

The synthesis of this step referred to the synthesis process of the first step of **compound 21 in Example 2.**

LC_MS: (ES⁺): m/z 417.1 [M+H] ⁺.

¹H NMR (400 MHz, DMSO-d6): δ 1.47-1.53 (m, 2H), 1.62-1.63 (m, 2H), 1.77-1.83 (m, 2H), 2.22 (s, 3H), 2.44-2.47 (m, 4H), 2.75-2.77 (t, *J =* 8.4 Hz, 4H), 3.31 (s, 2H), 3.58-3.60 (t, *J =* 5.6 Hz, 2H), 4.51 (s, 2H), 6.95-7.00 (m, 2H), 7.28-7.31 (t, *J =* 6.0 Hz, 1H), 7.86 (s, 1H), 8.53 (s, 1H), 9.10 (s, 1H).

### Example 13: Synthesis route of compound 22

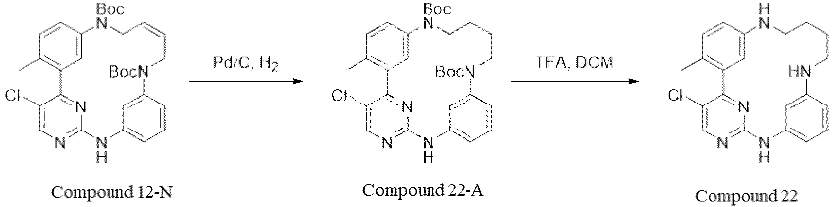

### Step 1: Compound 22-A Di-tert-butyl 2⁵-chloro-1⁶-methyl-3,5,10-triaza-2(4,2)-pyrimidina-1,4(1,3)-dibenzenacyclodecaphan-5,10-dicar boxylate

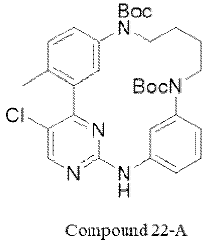

The synthesis of this step referred to the synthesis process of the first step of **compound 4 in Example 2.**

LC_MS: (ES⁺): m/z 580.3 [M+H] ⁺

¹H NMR (400 MHz, DMSO-d6): δ1.16 (s, 9H), 1.38 (s, 12H), 1.53-1.58 (m, 1H), 2.23 (s, 3H), 3.51-3.81 (m, 4H), 6.75-6.78 (m, 1H), 7.05-7.08 (m, 1H), 7.19-7.26 (m, 3H), 7.33-7.35 (m, 1H), 8.34 (s, 1H), 8.64 (s, 1H), 10.09 (s, 1H).

### Step 2: Compound 22 2⁵-chloro-1⁶-methyl-3,5,10-triaza-2(4,2)-pyrimidina-1,4(1,3)-dibenzocyclodecane

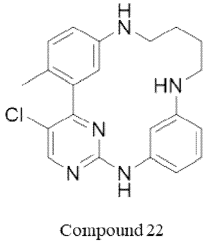

The synthesis of this step referred to the synthesis process of the sixth step of **compound 8 in Example 5.**

LC_MS: (ES⁺): m/z 380.3 [M+H] ⁺

¹H NMR (400 MHz, CDCl₃) δ 1.48-1.78 (m, 6H), 2.23 (s, 3H), 3.17 (t, *J =* 7.2 Hz, 2H), 3.28-3.34 (m, 2H), 6.27-6.35 (m, 2H), 6.69-6.72 (m, 1H), 6.85 (d, *J* = 2.8 Hz, 1H), 7.02-7.06 (m, 2H), 7.15 (s, 1H), 8.05 (t, *J =* 2.0 Hz, 1H), 8.36 (s, 1H).

### Example 14: Synthesis route of compound 23 and compound 24

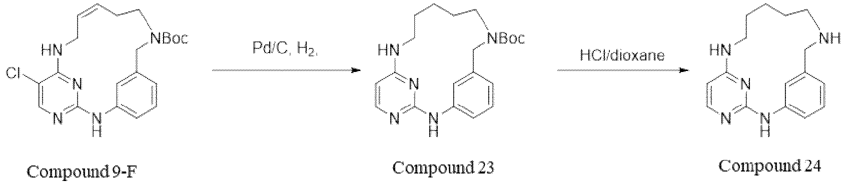

### Step 1: Compound 23 tert-butyl 2,5,11-triaza-1(2,4)-pyrimidina-3(1,3)-benzenacycloundecaphan-5-carboxylate

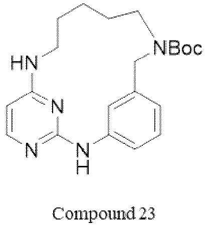

Palladium/carbon (50 mg) was added to a solution of **compound 9-F** tert-butyl (Z)-1⁵-chloro-2,5,11-triaza-1(2,4)-pyrimidina-3(1,3)-benzenacycloundecaphan-8-en-5-carboxylat e (50 mg, 130.38 µmol) in methanol. The reaction solution was stirred at 20°C under a hydrogen balloon atmosphere for 12 hours. The reaction was monitored by LCMS to be completed. The reaction solution was filtered through diatomaceous earth, and the residue was separated and purified by Pre-TLC (eluted with a petroleum ether solution containing 50% ethyl acetate) to obtain a brown solid **compound 23** tert-butyl 2,5,11-triaza-1(2,4)-pyrimidina-3(1,3)-benzenacycloundecaphan-5-carboxylate (25.0 mg).

LC_MS: (ES⁺): m/z 384.2 [M+H] ⁺.

### Step 2: Compound 24 2,5,11-triaza-1(2,4)-pyrimidina-3(1,3)-benzocycloundecaphane

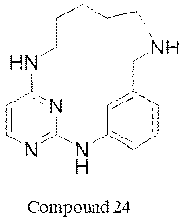

A solution of **compound 23** tert-butyl 2,5,11-triaza-1(2,4)-pyrimidina-3(1,3)-benzenacycloundecaphan-5-carboxylate (15 mg, 52.9 µmol) in hydrogen chloride/1,4-dioxane (1 mL) was stirred at 25°C for 2 hours. The reaction was monitored by LCMS to be completed. The reaction solution was concentrated under reduced pressure to obtain brown solid **compound 24** 2,5,11-triaza-1(2,4)-pyrimidina-3(1,3)-benzenacycloundecaphane (11.0 mg).

LC_MS: (ES⁺): m/z 284.2 [M+H] ⁺.

### Example 15: Synthesis route of compound 25

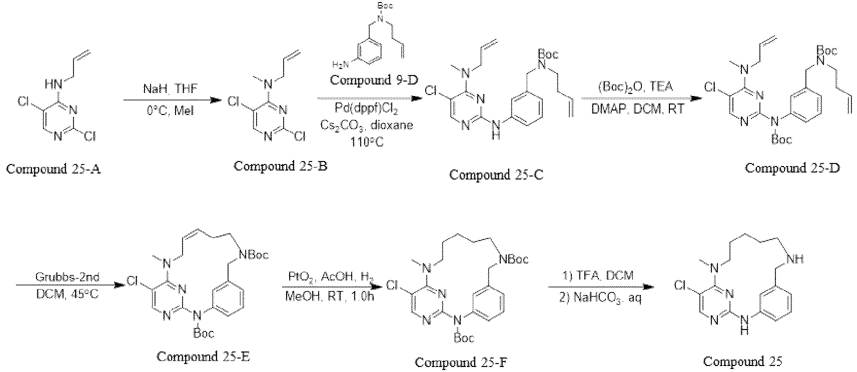

### Step 1: Compound 25-B N-allyl-2,5-dichloro-N-methylpyrimidin-4-amine

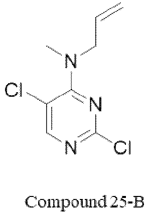

**Compound 25-A** (150 mg, 0.74 mmol) was dissolved in tetrahydrofuran (25 mL), and sodium hydride (31 mg, 0.77 mmol, 60% mass fraction in kerosene) was added in batches under an ice bath condition. The solution was stirred under an ice bath condition for 20 minutes, then added with iodomethane (228 mg, 0.1 mL, 1.60 mmol) dropwise, and stirred for 3 hours in an ice water bath. When the disappearance of the raw material was detected by TLC, the reaction solution was slowly quenched with a saturated ammonium chloride solution and extracted with ethyl acetate (25 mLx2). The organic layers were combined, washed with saturated brine (20 mL), dried over anhydrous sodium sulfate, filtered, and concentrated. The crude product was separated and purified by silica gel column chromatography (eluted with petroleum ether containing 0%-15% ethyl acetate) to obtain a yellow oily **compound 25-B** N-allyl-2,5-dichloro-N-methylpyrimidin-4-amine (130 mg).

LC_MS: (ES⁺): m/z 217.9 [M+H] ⁺

### Step 2: Compound 25-C tert-butyl (3-((4-(allyl(methyl)amino)-5-chloropyrimidin-2-yl)amino)benzyl)(but-3-en-1-yl)carbamate

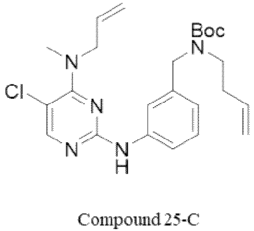

[1,1'-bis(dibenzenephosphino)ferrocene]dichloropalladium(II) (44 mg, 0.06 mmol) and cesium carbonate (587 mg, 1.8 mmol) were added to a solution of **compound 25-C** N-allyl-2,5-dichloro-N-methylpyrimidin-4-amine (130 mg, 0.60 mmol) and **compound 9-D** (166 mg, 0.6 mmol) in dioxane (2.5 mL). The reaction solution was stirred at 110°C under nitrogen atmosphere for 4.5 hours. The reaction was monitored by LCMS to be completed. The reaction solvent was removed by rotary evaporation under reduced pressure, and the residue was added with water (20 mL) and extracted with ethyl acetate (20 mLx2). The organic layers were combined, washed with saturated brine (30 mL), dried over anhydrous sodium sulfate, filtered, and concentrated. The crude product was separated and purified by silica gel column chromatography (eluted with petroleum ether containing 10%-20% ethyl acetate) to obtain a colorless oily **compound 25-C** tert-butyl (3-((4-(allyl(methyl)amino)-5-chloropyrimidin-2-yl)amino)benzyl)(but-3-en-1-yl)carbamate (65 mg).

LC_MS: (ES⁺): m/z 458.1 [M+H] ⁺

### Step 3: Compound 25-D tert-butyl (3-((4-(allyl(methyl)amino)-5-chloropyrimidin-2-yl)(tert-butoxycarbonyl)amino)benzyl)(but-3-en -1-yl)carbamate

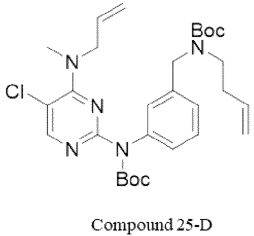

Di-tert-butyl dicarbonate (91 mg, 0.42 mmol) was added to a solution of **compound 25-C** tert-butyl (3-((4-(allyl(methyl)amino)-5-chloropyrimidin-2-yl)amino)benzyl)(but-3-en-1-yl)carbamate (152 mg, 0.33 mmol), triethylamine (42 mg, 0.42 mmol) and 4-dimethylaminopyridine (8 mg, 0.07 mmol) in dichloromethane (3.0 mL). The reaction solution was stirred at 25°C for 4 hours . The reaction was monitored by TLC to be completed. The reaction solvent was concentrated by rotary evaporation. The residue was separated and purified by silica gel column chromatography (eluted with petroleum ether containing 0%-15% ethyl acetate) to obtain a light yellow oily **compound 25-D** tert-butyl (3-((4-(allyl(methyl)amino)-5-chloropyrimidin-2-yl)(tert-butoxycarbonyl)amino)benzyl)(but-3-en -1-yl)carbamate (146 mg).

LC_MS: (ES⁺): m/z 558.2 [M+H] ⁺

### Step 4: Compound 25-E di-tert-butyl (Z)-1⁵-chloro-11-methyl-2,5,11-triaza-1(2,4)-pyrimidina-3(1,3)-benzenacycloundecaphan-8-en-2, 5-dicarboxylate

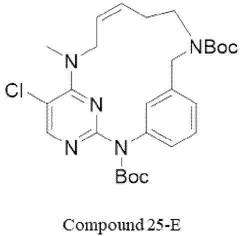

A second-generation Grubbs catalyst (33 mg, 0.04 mmol) was added to a solution of **compound 25-D** tert-butyl (3-((4-(allyl(methyl)amino)-5-chloropyrimidine-2-yl)(tert-butoxycarbonyl)amino)benzyl)(but-3-e n-1-yl)carbamate (146 mg, 0.26 mmol) in dichloromethane (75 mL). The reaction solution was stirred at 45°C under nitrogen atmosphere for 1.0 hour. The reaction was monitored by TLC to be completed. The reaction solution was concentrated. The residue was separated and purified by silica gel column chromatography (eluted with petroleum ether containing 0%-25% ethyl acetate) to obtain brown solid **compound 25-E** di-tert-butyl (Z)-1⁵-chloro-11-methyl-2,5,11-triaza-1(2,4)-pyrimidina-3(1,3)-benzenacycloundecaphan-8-en-2, 5-dicarboxylate (124 mg).

LC_MS: (ES⁺): m/z 530.2 [M+H]⁺

### Step 5: Compound 25-F di-tert-butyl 1⁵-chloro-11-methyl-2,5,11-triaza-1(2,4)-pyrimidina-3(1,3)-benzenacycloundecaphan-2,5-dicarbo xylate

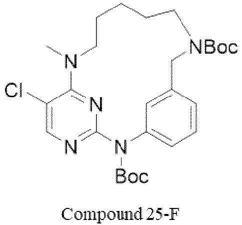

Platinum dioxide (20 mg, 0.09 mmol) and acetic acid (50 mg) were added to a solution of **compound 25-E** di-tert-butyl (Z)-1⁵-chloro-11-methyl-2,5,11-triaza-1(2,4)-pyrimidina-3(1,3)-benzenacycloundecaphan-8-en-2, 5-dicarboxylate (124 mg, 0.23 mmol) in methanol (25 mL) under nitrogen atmosphere. The reaction system was replaced with hydrogen three times and stirred at 25°C under hydrogen balloon atmosphere for 1.0 hour. The reaction was monitored by TLC to be completed. Most of the reaction solvent was evaporated, and the concentrated reaction solution was adjusted to pH = 7-8 with saturated sodium bicarbonate, diluted with water (30 mL), and extracted with ethyl acetate (25 mL x 2). The organic layers were combined, washed with saturated brine (50 mL), dried over anhydrous sodium sulfate, filtered, and concentrated. The residue was separated and purified by silica gel column chromatography (eluted with petroleum ether containing 0%-25% ethyl acetate) to obtain a white solid **compound 25-F** di-tert-butyl 1⁵-chloro-11-methyl-2,5,11-triaza-1(2,4)-pyrimidina-3(1,3)-benzenacycloundecaphan-2,5-dicarbo xylate (43 mg).

LC_MS: (ES⁺): 532.3 [M+H] ⁺

### Step 6: Compound 25 1⁵-chloro-11-methyl-2,5,11-triaza-1(2,4)-pyrimidina-3(1,3)-benzocycloundecaphane

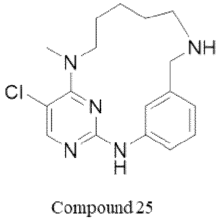

Trifluoroacetic acid (1.0 mL) was added to a solution of **compound 25-F** di-tert-butyl 1⁵-chloro-11-methyl-2,5,11-triaza-1(2,4)-pyrimidina-3(1,3)-benzenacycloundecaphan-2,5-dicarbo xylate (43 mg, 0.08 mmol) in dichloromethane (1 mL). The reaction solution was stirred at 25°C for 1.5 hours. The reaction was monitored by LCMS to be completed. The reaction solution was concentrated under reduced pressure and then dried under high vacuum. The residue was added with methanol (1 mL), and adjusted to pH 7-8 with saturated sodium bicarbonate under stirring. The suspension was filtered, and the filter cake was washed with water (5 mL). The filter cake was dried under high vacuum with an oil pump to obtain a white solid **compound 25** 1⁵-chloro-11-methyl-2,5,11-triaza-1(2,4)-pyrimidina-3(1,3)-benzocycloundecaphane (25.2 mg).

LC_MS: (ES⁺): 332.1 [M+H] ⁺

¹H NMR (400 MHz, DMSO-d₆) δ: 9.31 (s, 1H), 8.11 (s, 1H), 7.97 (s, 1H), 7.24 (t, *J* = 0.8 Hz, 1H), 7.06 (d, *J =* 0.8 Hz, 1H), 6.94 (d, *J =* 0.8 Hz, 1H), 3.88 (s, 2H), 3.57 - 3.52 (m, 2H), 3.27 (s, 3H), 2.58 (t, *J =* 0.8 Hz, 2H), 1.70 - 1.59 (m, 4H), 1.53 - 1.42 (m, 2H).

### Example 16: Synthesis route of compound 26:

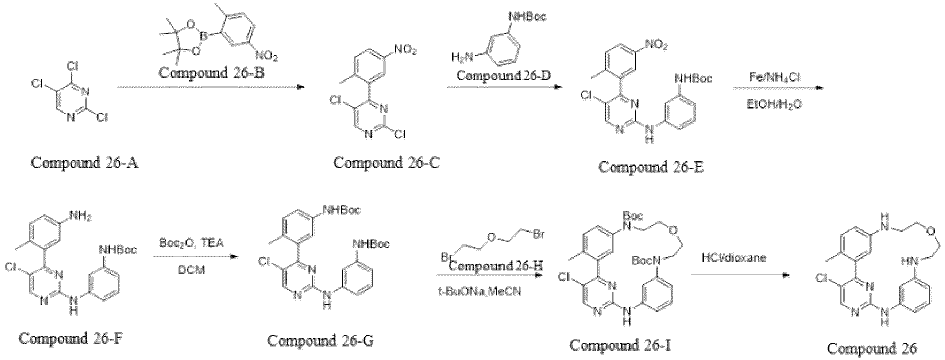

### Step 1: Compound 26-C 2,5-dichloro-4-(2-methyl-5-nitrophenyl)pyrimidine

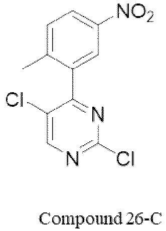

The synthesis of this step referred to the synthesis process of the seventh step of **compound 12 in Example 8.**

LC_MS: (ES⁺): m/z 285.0 [M+H] ⁺.

### Step 2: Compound 26-E tert-butyl (3-((5-chloro-4-(2-methyl-5-nitrophenyl)pyrimidin-2-yl)amino)phenyl)carbamate

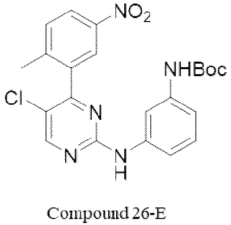

The synthesis of this step referred to the synthesis process of the sixth step of **compound 2 in Example 1.**

LC_MS: (ES⁺): m/z 456.1 [M+H] ⁺.

### Step 3: Compound 26-F tert-butyl (3-((4-(5-amino-2-methylphenyl)-5-chloropyrimidin-2-yl)amino)phenyl)carbamate

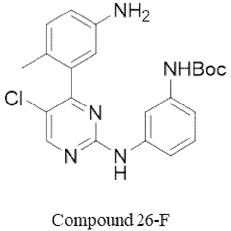

The synthesis of this step referred to the synthesis process of the fourth step of **compound 2 in Example 1.**

LC_MS: (ES⁺): m/z 426.2 [M+H] ⁺.

### Step 4: Compound 26-G tert-butyl (3-(2-((3-((tert-butoxycarbonyl)amino))phenyl)amino)-5-chloropyrimidin-4-yl)-4-methylphenyl)c arbamate

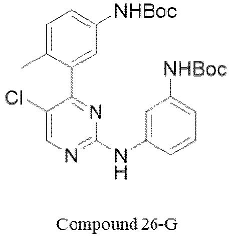

Di-tert-butyl dicarbonate (430.43 mg, 1.972 mmol) was added to a solution of **compound 26-F** tert-butyl (3-((4-(5-amino-2-methylphenyl)-5-chloropyrimidin-2-yl)amino)phenyl)carbamate (700 mg, 1.643 mmol) in ethanol (5 mL), and the reaction solution was stirred at room temperature for 14 hours. The reaction was monitored by TLC to be completed. The reaction solution was concentrated, and the residue was separated and purified by silica gel column chromatography (eluted with petroleum ether containing 0%-25% ethyl acetate) to obtain yellow solid **compound 26-G** tert-butyl (3-(2-((3-((tert-butoxycarbonyl)amino)phenyl)amino)-5-chloropyrimidin-4-yl)-4-methylphenyl)c arbamate (710 mg).

LC_MS: (ES⁺): m/z 527.3 [M+H] ⁺.

### Step 5: Compound 26-I di-tert-butyl-2⁵-chloro-1⁶-methyl-8-oxa-3,5,11-triaza-2(4,2)-pyrimidina-1,4(1,3)-dibenzenacyclou ndecaphan-5,11-dicarboxylate

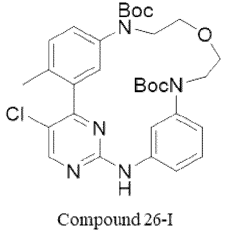

**Compound 26-H** 1-bromo-2-(2-bromoethoxy)ethane (44.06 mg, 0.19 mmol) was added to a solution of **compound 26-G** tert-butyl (3-(2-((3-((tert-butoxycarbonyl)amino)phenyl)amino)-5-chloropyrimidin-4-yl)-4-methylphenyl)c arbamate (100 mg) and sodium tert-butoxide (36.52 mg, 0.38 mmol) in acetonitrile (10 mL), and the reaction solution was stirred at 60°C for 8 hours . The reaction was monitored by TLC to be completed. The reaction solution was quenched with water (10 mL) and extracted with dichloromethane (10 mLx2). The organic layers were combined, dried over anhydrous sodium sulfate, and concentrated. The residue was separated and purified by Pre-TLC (eluted with petroleum ether containing 25% ethyl acetate) to obtain a white solid **compound 26-I** di-tert-butyl-2⁵-chloro-1⁶-methyl-8-oxa-3,5,11-triaza-2(4,2)-pyrimidina-1,4(1,3)-dibenzenacyclou ndecaphan-5,11-dicarboxylate (5 mg).

LC_MS: (ES⁺): m/z 618.3 [M+Na] ⁺.

### Step 6: Compound 26 2⁵-chloro-1⁶-methyl-8-oxa-3,5,11-triaza-2(4,2)-pyrimidina-1,4(1,3)-dibenzocycloundecaphane

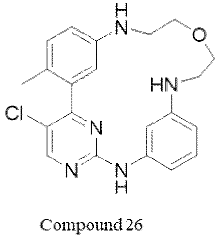

A hydrochloric acid/dioxane solution (0.5 mL) was added to a solution of **compound 26-I** di-tert-butyl-2⁵-chloro-1⁶-methyl-8-oxa-3,5,11-triaza-2(4,2)-pyrimidina-1,4(1,3)-dibenzenacycloundecaphan-5,11-dicarboxylate (5 mg, 0.0084 mmol) in methanol (0.5 mL). The reaction solution was stirred at room temperature for 5 hours. The reaction was monitored by TLC to be completed. The reaction solution was concentrated to obtain a yellow solid **compound 26** 2⁵-chloro-1⁶-methyl-8-oxa-3,5,11-triaza-2(4,2)-pyrimidina-1,4(1,3)-dibenzocycloundecaphane (5 mg, hydrochloride).

LC_MS: (ES⁺): m/z 396.1 [M+H] ⁺.

### Example 17: Synthesis route of compound 27:

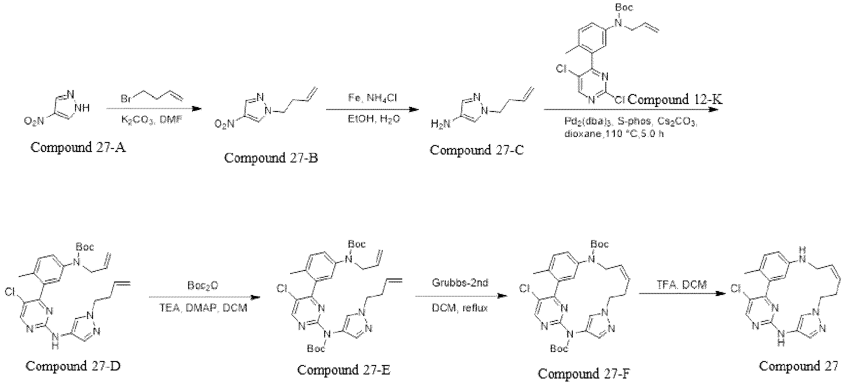

### Step 1: Compound 27-B 1-(but-3-en-1-yl)-4-nitro-1H-pyrazole

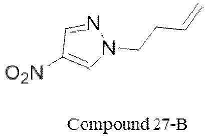

4-Bromobut-1-ene (1.31 g, 9.73 mmol) was slowly added dropwise to a mixed solution of **compound 27-A** (1.0 g, 8.84 mmol) and potassium carbonate (3.05 g, 22.1 mmol) in N,N-dimethylformamide (10 mL) at room temperature, and then the reaction solution was stirred at room temperature for 12 hours. When TLC detected the disappearance of the raw material, the reaction solution was added with water (50 mL) under stirring, and extracted with ethyl acetate (30 mL x 2). The organic layers were combined, washed with saturated brine (30 mL x 2), dried over anhydrous sodium sulfate, filtered, and concentrated. The crude product was dried under high vacuum to obtain a white solid **compound 27-B** 1-(but-3-en-1-yl)-4-nitro-1H-pyrazole (1.45 g).

### Step 2: Compound 27-C 1-(but-3-en-1-yl)-¹hydrogen-pyrazole-4-amine

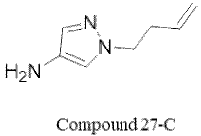

Iron powder (2.42 g, 43.37 mmol) was added to a solution of water (5 mL), ammonium chloride (3.71 g, 69.36 mmol) and **compound 27**-B (1.45 g, 8.67 mmol) in ethanol (20 mL) under stirring. The reaction solution was stirred at 110°C under a nitrogen atmosphere for 4.5 hours. The reaction was monitored by LCMS to be completed. The reaction solvent was evaporated under reduced pressure, and then water (30 mL) and ethyl acetate (30 mL) were added. The organic phase was separated, and the aqueous phase was extracted with ethyl acetate (30 mL x 2). The organic layers were combined, dried over anhydrous sodium sulfate, filtered, and concentrated. The crude product was eluted by silica gel column chromatography (using dichloromethane containing 2.5%-3.3% methanol) to obtain a brown oily **compound 27-C** 1-(but-3-en-1-yl)-1*H*-pyrazole-4-amine (370 mg).

¹H NMR (400 MHz, CDCl₃) *δ*: 7.17 (s, 1H), 7.02 (s, 1H), 5.82 - 5.72 (m, 1H), 5.13 - 5.04 (m, 2H), 4.07 (t, *J =* 8.0 Hz, 2H), 2.89 (brs, 2H), 2.63 - 2.55 (m, 2H).

### Step 3: Compound 27-D tert-butyl allyl (3-(2-((1-(but-3-en-1-yl)-1H-pyrazol-4-yl)amino)-5-chloropyrimidin-4-yl)-4-methylphenyl) carbamate

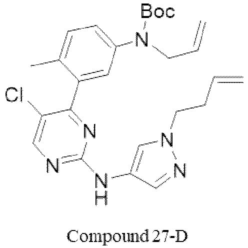

Pd₂(dba)₃(37 mg, 0.04 mmol) and cesium carbonate (280 mg, 0.81 mmol) were added to a solution of **compound 12-K** (170 mg, 0.43 mmol), S-phos (37 mg, 0.09 mmol) and **compound 27-C** (1.45 g, 0.09 mmol) in 1,4-dioxane (3.5 mL). The reaction solution was replaced with nitrogen three times under stirring, and then stirred at 110°C under nitrogen atmosphere for 5 hours of reaction. The reaction was monitored by LCMS to be completed. The reaction solvent was evaporated under reduced pressure, and then water (30 mL) and ethyl acetate (30 mL) were added. The organic phase was separated, dried over anhydrous sodium sulfate, filtered, and concentrated. The crude product was separated and purified by silica gel plate (using petroleum ether containing 30% ethyl acetate) to obtain yellow oily **compound 27-D** tert-butyl allyl (3-(2-((1-(but-3-en-1-yl)-1H-pyrazol-4-yl)amino)-5-chloropyrimidin-4-yl)-4-methylphenyl) carbamate (150 mg).

LC-MS: (ES⁺): m/z 495.3 [M+H] ⁺.

¹H NMR (400 MHz, CDCl₃) *δ*: 8.43 (s, 1H), 7.83 (s, 1H), 7.50 (s, 1H), 7.26 - 7.12 (m, 4H), 6.00 - 5.88 (m, 1H), 5.83 - 5.73 (m, 1H), 5.20 - 5.04 (m, 4H), 4.25 (dt, *J* = 3.0, 4.0 Hz, 2H), 4.17 - 4.13 (m, 2H), 2.62 (q, *J =* 8.0 Hz, 2H), 2.23 (s, 3H), 1.45 (s, 9H).

### Step 4: Compound 27-E tert-butyl allyl (3-(2-((1-(but-3-en-1-yl)-1H-pyrazol-4-yl)(tert-butoxycarbonyl)amino)-5-chloropyrimidin-4-yl)-4-methylphenyl) carbamate

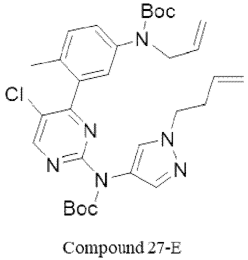

The synthesis of this step referred to the synthesis process of the third step of **compound 25 in Example 15.**

LC-MS: (ES⁺): m/z 595.3 [M+H] ⁺.

### Step 5: Compound 27-F di-tert-butyl (4⁴E,7Z)-2⁵-chloro- 1⁶-methyl-4¹hydro-3,10-diaza-2(4,2)-pyrimidina-4(4,1)-pyrazola-1(1,3)-benz enacyclodecaphan-7-en-3,10-dicarboxylate

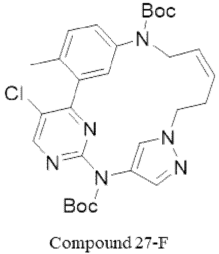

The synthesis of this step referred to the synthesis process of the fourth step of **compound 25 in Example 15.**

LC-MS: (ES⁺): m/z 567.2 [M+H] ⁺.

### Step 6: Compound 27 (4⁴E,7Z)-2⁵-chloro-1⁶-methyl-4¹hydro-3,10-diaza-2(4,2)-pyrimidina-4(4,1)-pyrazole-1(1,3)-benz enecyclodecane-7-ene

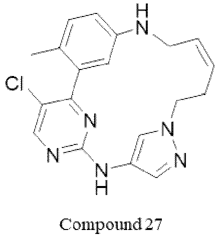

The synthesis of this step referred to the synthesis process of the sixth step of **compound 25 in Example 15.**

LC-MS: (ES⁺): 367.1 [M+H] ⁺.

### ¹H NMR (400 MHz, DMSO-d₆) δ: 9.80 (s, 1H), 8.47 (s, 1H), 8.18 (s, 1H), 7.26 (s, 1H), 6.99 (d, J = 8.0 Hz, 1H), 6.74 (dd, J = 4.0, 8.0 Hz, 1H), 6.52 (s, 1H), 6.08 (brs, 1H), 4.23 - 4.18 (m, 1H), 4.11 - 4.00 (m, 1H), 3.70 - 3.50 (m, 2H), 2.50 - 2.25 (m, 2H), 2.18 (s, 3H).

### Example 18: Synthesis route of compound 28:

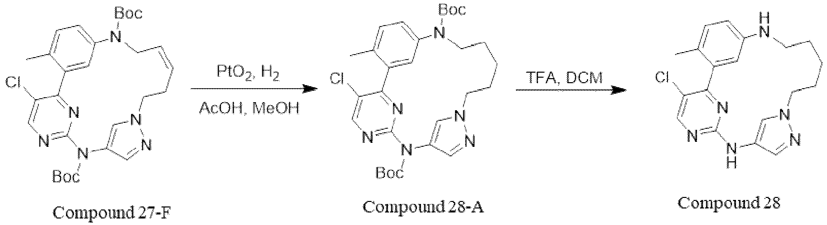

### Step 1: Compound 28-A di-tert-butyl (E)-2⁵-chloro-1⁶-methyl-4¹hydro-3,10-diaza-2(4,2)-pyrimidina-4(4,1)-pyrazola-1(1,3)-benzenacy clodecaphan-3, 10-dicarboxylate

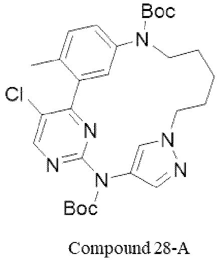

The synthesis of this step referred to the synthesis process of the fifth step of **compound 25 in Example 15.**

LC-MS: (ES⁺): m/z 569.3 [M+H] ⁺.

### Step 2: Compound 28 (E)-2⁵-chloro-1⁶-methyl-4¹hydro-3,10-diaza-2(4,2)-diaza-2(4,2)-pyrimidina-4(4,1)-pyrazola-1(1, 3)-benzenacyclodecaphane

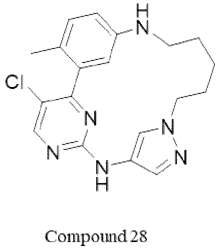

The synthesis of this step referred to the synthesis process of the sixth step of **compound 25 in Example 15.**

LC-MS: (ES⁺): m/z 369.1 [M+H] ⁺.

¹H NMR (400 MHz, DMSO-d₆) δ: 9.81 (s, 1H), 8.48 (s, 1H), 8.20 (s, 1H), 7.32 (s, 1H), 6.98 (d, *J =* 8.0 Hz, 1H), 6.67 (dd, *J =* 4.0, 8.0 Hz, 1H), 6.56 (d, *J =* 4.0 Hz, 1H), 5.95 - 5.85 (m, 1H), 4.11 - 4.06 (m, 2H), 3.11 - 2.93 (m, 2H), 2.17 (s, 3H), 2.00 - 1.75 (m, 2H), 1.55 - 1.49 (m, 1H), 1.25 - 1.10 (m, 2H), 1.05 - 0.95 (m, 1H).

### Example 19: Synthesis route of compound 29:

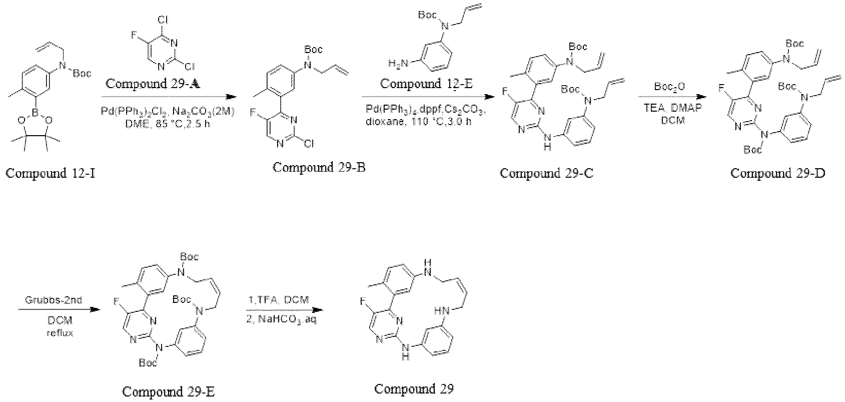

### Step 1: Compound 29-B tert-butyl allyl(3-(2-chloro-5-fluoropyrimidin-4-yl)-4-methylphenyl)carbamate

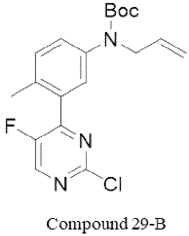

**Compound 12-1** (0.50 g, 1.34 mmol) was added to a mixed solution of A (0.56 g, 3.35 mmol) and Pd(PPh₃)Cl₂ (91 mg, 0.13 mmol) in 1,2-dimethoxyethane (10 mL) at room temperature, and then sodium carbonate solution (2.0 M, 5 mL) was added and the reaction solution was replaced with nitrogen three times at room temperature. The reaction solution was stirred at 85°C in a nitrogen atmosphere for 2.5 hours. When TLC detected the disappearance of the raw material, the reaction solution was added with water (30 mL), extracted with ethyl acetate (30 mL), dried over anhydrous sodium sulfate, filtered, and concentrated. The crude product was obtained by elution of silica gel column chromatography (using petroleum ether containing 0%-7% ethyl acetate) as a colorless oily **compound 29-B** tert-butyl allyl(3-(2-chloro-5-fluoropyrimidin-4-yl)-4-methylphenyl)carbamate (475 mg).

LC-MS: (ES⁺): m/z 423.2 [M+EtOH] ⁺.

¹H NMR (400 MHz, CDCl₃) δ: 8.56 (s, 1H), 7.33 - 7.27 (m, 3H), 5.98 - 5.88 (m, 1H), 5.21 - 5.12 (m, 2H), 4.25 (dt, *J =* 2.0, 4.0 Hz, 2H), 2.33 (d, *J =* 4.0 Hz, 3H), 1.49 (s, 9H).

### Step 2: Compound 29-C tert-butyl allyl(3-(2-((3-(allyl(tert-butoxycarbonyl)amino) phenyl)amino)-5-fluoropyrimidin-4-yl)-4-methylphenyl)carbamate

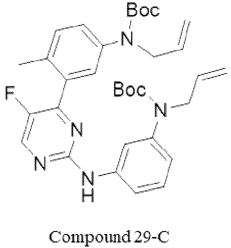

The synthesis of this step referred to the synthesis process of the second step of **compound 25 in Example 15.**

LC-MS: (ES⁺): m/z 612.3 [M+Na] ⁺.

### Step 3: Compound 29-D tert-butyl allyl(3-((4-(5-(allyl(tert-butoxycarbonyl)amino)-2-methylphenyl)-5-fluoropyrimidin-2-yl)(tert-butoxycarbonyl)amino)phenyl)carbamate

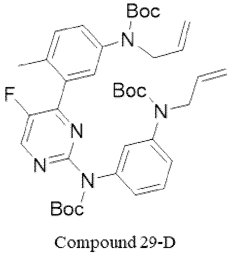

The synthesis of this step referred to the synthesis process of the third step of **compound 25 in Example 15.**

LC-MS: (ES⁺): m/z 690.0 [M+H] ⁺.

¹H NMR (400 MHz, CDCl₃) *δ*: 8.60 (s, 1H), 7.30 - 7.26 (m, 2H), 7.25 - 7.19 (m, 2H), 7.18 - 7.13 (m, 2H), 7.09 (dt, *J =* 4.0, 8.0 Hz, 1H), 5.94 - 5.84 (m, 2H), 5.18 - 5.08 (m, 4H), 4.21 (t, *J =* 4.0 Hz, 4H), 2.16 (s, 3H), 1.46 (s, 9H), 1.45 (s, 9H), 1.41 (s, 9H).

### Step 4: Compound 29-E tri-tert-butyl (Z)-2⁵-fluoro-1⁶-methyl-3,5,10-triaza-2(4,2)-pyrimidina-1,4(1,3)-dibenzenacyclodecaphan-7-en-3,5,10-tricarboxylate

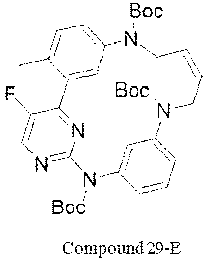

The synthesis of this step referred to the synthesis process of the fourth step of **compound 25 in Example 15.**

LC-MS: (ES⁺): m/z 662.3 [M+H] ⁺.

¹H NMR (400 MHz, CDCl₃) δ: 8.65 (d, *J =* 2.0 Hz, 1H), 7.37 - 7.30 (m, 2H), 7.21 - 7.12 (m, 3H), 7.04 (s, 1H), 6.83 (d, *J =* 4.0 Hz, 1H), 5.75 - 5.50 (m, 2H), 4.42 (d, *J =* 4.0 Hz, 2H), 4.08 (d, *J =* 4.0 Hz, 2H), 2.29 (d, *J =* 4.0 Hz, 3H), 2.16 (s, 3H), 1.48 (s, 9H), 1.46 (s, 9H), 1.43 (s, 9H).

### Step 5: Compound 29 (Z)-2⁵-fluoro-1⁶-methyl-3,5,10-triaza-2(4,2)-pyrimidina-1,4(1,3)-dibenzenacyclodecaphan-7-ene

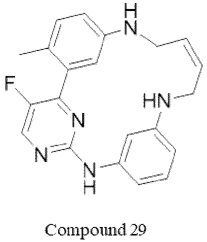

The synthesis of this step referred to the synthesis process of the sixth step of **compound 25 in Example 15.**

LC-MS: (ES⁺): m/z 362.1 [M+H] ⁺.

¹H NMR (400 MHz, DMSO-d₆) *δ*: 9.41 (s, 1H), 8.53 (d, *J =* 2.0 Hz, 1H), 7.67 (s, 1H), 7.00 (d, *J =* 8.0 Hz, 1H), 6.88 (t, *J =* 8.0 Hz, 1H), 6.75 (dd, *J =* 4.0, 8.0 Hz, 1H), 6.61 (d, *J =* 4.0 Hz, 1H), 6.43 (d, *J =* 8.0 Hz, 1H), 6.25 (d, *J =* 8.0 Hz, 1H), 6.09 (t, *J =* 4.0 Hz, 1H), 5.86 (t, *J =* 4.0 Hz, 1H), 5.40 (s, 2H), 3.70 (s, 2H), 3.54 (s, 2H), 2.14 (d, *J* = 4.0 Hz, 3H).

### Example 20: Synthesis route of compound 30:

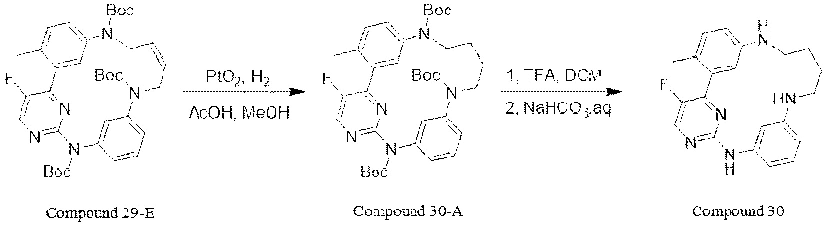

### Step 1: Compound 30-A tri-tert-butyl 2⁵-fluoro-1⁶-methyl-3,5,10-triaza-2(4,2)-pyrimidina-1,4(1,3)-dibenzenacyclodecaphan-3,5,10-tric arboxylate

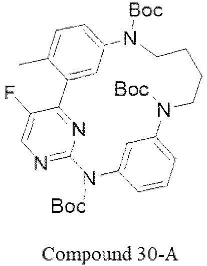

The synthesis of this step is based on the synthesis process of the fifth step of **compound 25 in Example** 15.

LC-MS: (ES⁺): m/z 664.4 [M+H] ⁺.

¹H NMR (400 MHz, CDCl₃) δ: 8.64 (d, *J =* 4.0 Hz, 1H), 7.32 - 7.29 (m, 1H), 7.28 - 7.26 (m, 2H), 7.20 - 7.15 (m, 2H), 7.10 - 7.07 (m, 1H), 6.80 (brs, 1H), 3.69 (t, *J* = 8.0 Hz, 2H), 3.62 (t, *J* = 8.0 Hz, 2H), 2.32 (d, *J =* 4.0 Hz, 3H), 1.58 - 1.35 (m, 31H).

### Step 2: Compound 30 2⁵-fluoro-1⁶-methyl-3,5,10-triaza-2(4,2)-pyrimidina-1,4(1,3)-dibenzenacyclodecaphane

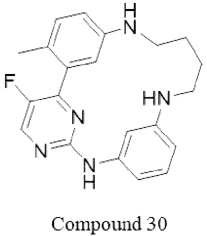

The synthesis of this step referred to the synthesis process of the sixth step of **compound 25 in Example 15.**

LC-MS: (ES⁺): m/z 364.2 [M+H] ⁺.

¹H NMR (400 MHz, DMSO-d₆) *δ*: 9.45 (s, 1H), 8.53 (d, *J =* 4.0 Hz, 1H), 7.83 (s, 1H), 6.99 (d, *J =* 8.0 Hz, 1H), 6.92 - 6.85 (m, 2H), 6.69 (dd, *J =* 4.0, 8.0 Hz, 1H), 6.35 (d, *J =* 8.0 Hz, 1H), 6.22 (dd, *J =* 2.0, 8.0 Hz, 1H), 5.54 (t, *J =* 6.0 Hz, 1H), 5.29 (t, *J =* 6.0 Hz, 1H), 3.19 - 3.17 (m, 3H), 3.10 - 2.95 (m, 2H), 2.10 (d, *J =* 4.0 Hz, 3H), 1.60 - 1.45 (m, 4H).

### Example 21: Synthesis route of compound 31:

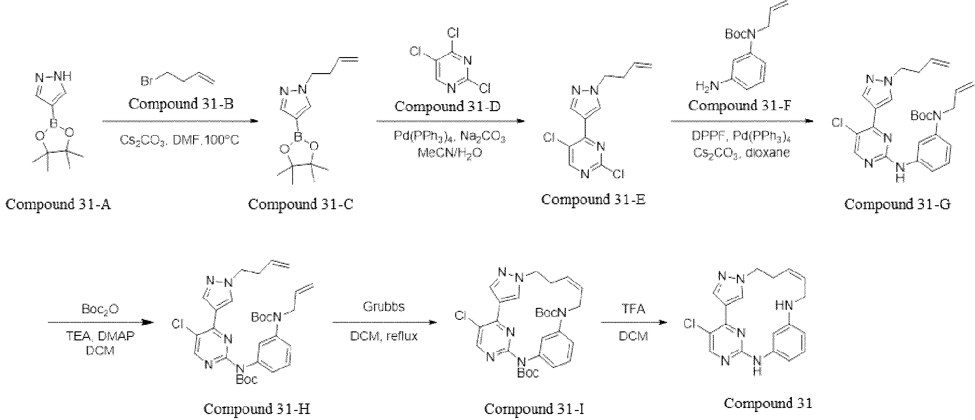

### Step 1: Compound 31-C 1-(but-3-enyl)-4-(4,4,5,5-tetramethyl-1,3,2-dioxaborolan-2-yl)-1H-pyrazole

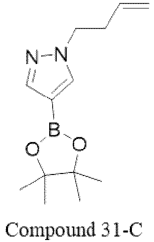

In a 250 ml single-neck flask, **compound 31-A** 4-(4,4,5,5-tetramethyl-1,3,2-dioxaborolan-2-yl)-1H-pyrazole (5 g, 25.77 mmol), **compound 31-B** 4-bromo-1-butene (5.22 g, 38.66 mmol) and cesium carbonate (16 g, 51.33 mmol) were refluxed in DMF (50 ml) for 2 hours. The reaction was monitored by TLC to be completed. The reaction solution was poured into water (500 ml) and extracted 8 times with ethyl acetate (100 ml). The organic phases were combined, washed twice with saturated brine, dried over anhydrous sodium sulfate, filtered and concentrated to obtain a brown oily crude **compound 31-C** 1-(but-3-enyl)-4-(4,4,5,5-tetramethyl-1,3,2-dioxaborolan-2-yl)-1H-pyrazole (4.3 g), which was used directly in the next step without further purification.

### Step 2: Compound 31-E 4-(1-(but-3-enyl))-1H-pyrazol-4-yl)-2,5-dichloropyrimidine.

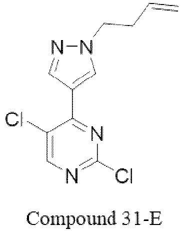

The synthesis of this step referred to the synthesis process of the seventh step of **compound 12 in Example 8.**

LC_MS: (ES⁺): m/z 269.15 [M+H] ⁺.

¹H NMR (400 MHz, CDCl₃): δ 8.50 (s, 1H), 8.41 (s, 1H), 8.35 (s, 1H), 5.80-5.72 (m, 1H), 5.12-5.08 (m, 2H), 4.26 (t, *J =* 7.2 Hz, 2H), 2.69-2.67 (m, 2H).

### Step 3: Compound 31-G tert-butyl allyl(3-((4-(1-(but-3-en-1-yl)-1H-pyrazol-4-yl)-5-chloropyrimidin-2-yl)amino)phenyl)carbamate

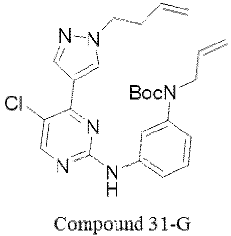

The synthesis of this step referred to the synthesis process of the sixth step of **compound 2 in Example 1.**

LC_MS: (ES⁺): m/z 481.55 [M+H] ⁺.

¹H NMR (400 MHz, CDCl₃): δ 8.35-8.30 (m, 3H), 7.56 (t, *J* = 2.0 Hz, 1H), 7.48-7.45 (m, 1H), 7.30-7.26 (m, 1H), 7.12 (s, 1H), 6.94-6.92 (m, 1H) , 5.99-5.90 (m, 1H) , 5.84-5.73 (m, 1H), 5.21-5.08 (m, 4H) , 4.27-4.24 (m, 4H) , 2.71-2.65 (m, 2H), 1.46 (s. 9H).

### Step 4: Compound 31-H tert-butyl (3-(allyl(tert-butoxycarbonyl)amino)phenyl) (4-(1-(but-3-en-1-yl)-1H-pyrazol-4-yl)-5-chloropyrimidin-2-yl)carbamate

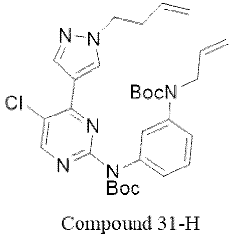

The synthesis of this step referred to the synthesis process of the third step of **Example 15 Compound 25.**

LC_MS: (ES⁺): m/z 581.45 [M+H] ⁺.

¹H NMR (400 MHz, CDCl₃): δ 8.53 (s, 1H), 8.20 (d, *J* = 1.6 Hz, 2H), 7.33-7.29 (m, 1H), 7.17-7.14 (m, 2H), 7.08-7.06 (m, 1H), 5.93-5.84 (m, 1H), 5.80-5.70 (m, 1H), 5.14-5.06 (m, 4H), 4.23-4.20 (m, 4H), 2.67-2.62 (m, 2H) , 1.46 (s. 9H) , 1.40 (s. 9H).

### Step 5: Compound 31-I di-tert-butyl (1⁴Z,7Z)-2⁵-chloro-1H-3,5-diazepina-2(4,2)-pyrimidina-1(4,1)-pyrazola-4(1,3)-benzenacyclodecaphan-7-en-3,5-dicarboxylate

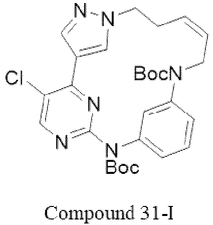

The synthesis of this step referred to the synthesis process of the fourth step of **compound 25 in Example 15.**

LC_MS: (ES⁺): m/z 553.35 [M+H] ⁺.

¹H NMR (400 MHz, DMSO-d6): δ 8.74 (s, 1H), 8.21 (s, 1H), 7.62 (s, 1H), 7.41-7.38 (m, 1H), 7.32-7.30 (m, 1H), 7.16-7.15 (m, 1H), 7.02-7.00 (m, 1H), 5.57-5.53 (m, 2H), 4.33-4.32 (m, 2H), 4.23-4.21 (m, 2H) , 2.45-2.44 (m, 2H), 1.42 (s, 9H), 1.37 (s, 9H).

### Step 6: Compound 31 (1⁴Z,7Z)-2⁵-chloro-1¹H-3,5-diaza-2(4,2)-pyrimidina-1(4,1)-pyrazola-4(1,3)-benzenacyclodecaph an-7-ene

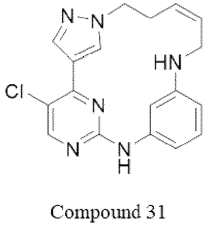

The synthesis of this step referred to the synthesis process of the sixth step of **compound 25 in Example 15**.

LC_MS: (ES⁺): m/z 353.15 [M+H] ⁺.

¹H NMR (400 MHz, DMSO-d6): δ 9.47 (s, 1H), 8.43 (s, 1H), 8.33 (s, 1H), 8.12 (s, 1H), 7.26 (s, 1H), 6.92 (t, *J =* 8.0 Hz, 1H), 6.34-6.32 (m, 2H), 5.86 (t, *J =* 6.0 Hz, 1H), 5.76-5.70 (m, 1H), 5.55-5.51 (m, 1H), 4.31-4.29 (m, 2H), 3.60 (s, 2H), 2.52-2.50 (m, 2H).

### Example 22: Synthesis route of compound 32:

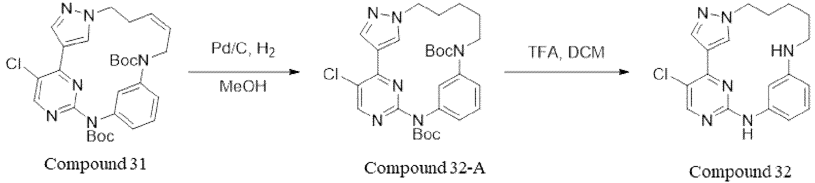

### Step 1: Compound 32-A di-tert-butyl (Z)-2⁵-chloro-1¹H-3,5-diaza-2(4,2)-pyrimidina-1(4,1)-pyrazola-4(1,3)-benzenacyclodecaphan-3,5 -dicarboxylate

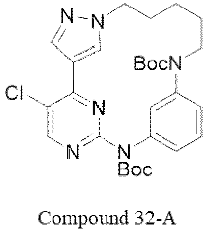

The synthesis of this step referred to the synthesis process of the first step of **compound 23 in Example 14.**

LC_MS: (ES⁺): m/z 555.35 [M+H] ⁺.

### Step 2: Compound 32 (Z)-2⁵-chloro-1¹H-3,5-diaza-2(4,2)-pyrimidina-1(4,1)-pyrazola-4(1,3)-benzenacyclodecaphane

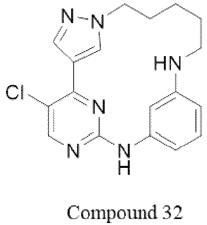

The synthesis of this step referred to the synthesis process of the sixth step of **compound 25 in Example 15.**

LC_MS: (ES⁺): m/z 355.10 [M+H] ⁺.

¹H NMR (400 MHz, DMSO-d6): δ 9.49 (s, 1H), 8.49 (s, 1H), 8.46 (s, 1H), 8.17 (s, 1H), 7.52 (s, 1H), 6.89 (t, *J* = 8.0 Hz, 1H), 6.30-6.28 (m, 1H), 6.21-6.18 (m, 1H), 5.76 (t, *J* = 6.4 Hz, 1H), 2.89-2.83 (m, 2H), 1.82-1.79 (m, 2H), 1.62-1.60 (m, 2H), 0.86-0.84 (m, 2H).

### Example 23: Synthesis route of compound 33:

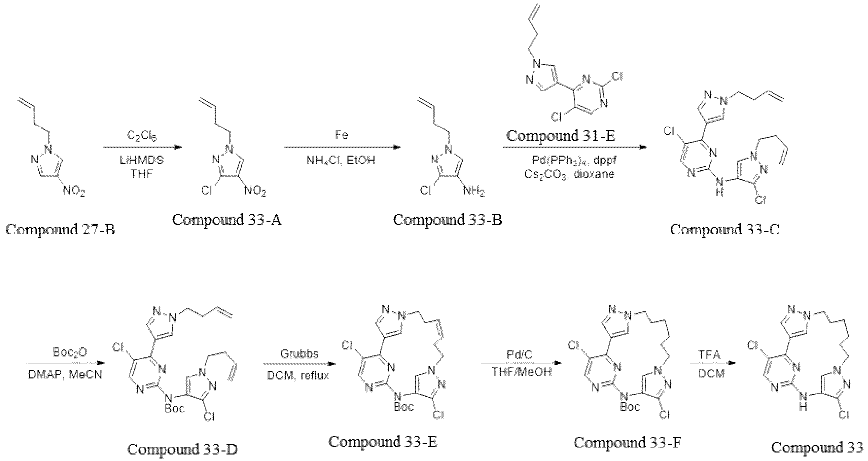

### Step 1: Compound 33-A 1-(but-3-enyl)-3-chloro-4-nitro-1H-pyrazole

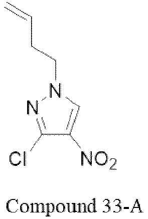

In a 50 ml three-necked flask with a thermometer, **compound 27-B** 1-(1-butenyl)-4-nitropyrazole (580 mg, 3.47 mmol) was added to dry tetrahydrofuran (20 ml). The reaction solution was replaced with nitrogen three times, maintained at a temperature of -78°C, and slowly added with Li-HMDS (8.7 ml, 8.68 mmol, 1M) dropwise. The reaction was performed at -78°C for 30 min, and then a solution of hexachloroethane (1.2 g, 5.2 mmol) in tetrahydrofuran (5 ml) was slowly dropwise added to the reaction solution at -78°C. The temperature was kept at -78°C for 2.5 hours of reaction. The reaction was monitored by TLC and LCMS to be completed. The reaction was quenched with water (20 ml), and the reaction solution was extracted with ethyl acetate (30 ml) three times. The organic phases were combined, dried over anhydrous sodium sulfate, filtered, and dried by rotation to obtain a crude yellow oily **compound 33-A** 1-(but-3-enyl)-3-chloro-4-nitro-1H-pyrazole (853 mg), which was directly used for the next step without further purification.

¹H NMR (400 MHz, CDCl₃): δ 8.17 (s, 1H), 5.79-5.70 (m, 1H), 5.11-5.06 (m, 2H), 4.27 (t, *J* = 6.8 Hz, 2H), 2.66-2.61 (m, 2H).

### Step 2: Compound 33-B 1-(but-3-enyl)-3-chloro-1H-pyrazol-4-amine

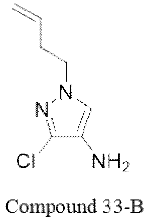

The synthesis of this step referred to the synthesis process of the fourth step of **compound 2 in Example 1.**

LC_MS: (ES⁺): m/z 171.75 [M+H] ⁺.

¹H NMR (400 MHz, CDCl₃): δ 7.21 (s, 1H), 5.82-5.71 (m, 1H), 5.10-5.04 (m, 2H), 4.10 (t, *J* = 7.6 Hz, 2H), 2.92 (brs, 2H), 2.57-2.52 (m, 2H).

### Step 3: Compound 33-C 4-(1-(but-3-enyl))-1H-pyrazol-4-yl)-nitrogen-(1-(but-3-enyl)-3-chloro-1H-pyrazol-4-yl)-5-chloropyrimidine-2-amine

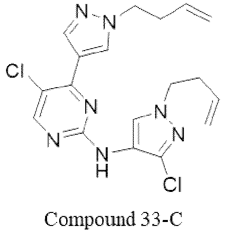

The synthesis of this step referred to the synthesis process of the third step of **compound 25 in Example 15.**

¹H NMR (400 MHz, CDCl₃): δ 8.34 (s, 1H), 8.30 (s, 1H), 8.26 (s, 1H), 8.06 (s, 1H), 6.54 (s, 1H), 5.83-5.75 (m, 2H), 5.14-5.07 (m, 4H), 4.27-4.18 (m, 4H), 2.68-2.61 (m, 4H).

### Step 4: Compound 33-D tert-butyl 4-(1-(but-3-enyl))-1H-pyrazol-4-yl)-5-chloropyrimidin-2-yl)(1-(but-3-enyl))-3-chloro-1H-pyrazo 1-4-yl)carbamate

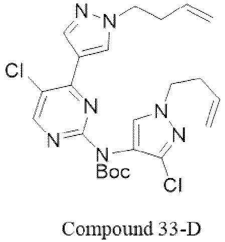

The synthesis of this step referred to the synthesis process of the third step of **compound 25 in Example 15.**

LC_MS: (ES⁺): m/z 504.00 [M+H] ⁺.

¹H NMR (400 MHz, CDCl₃): δ 8.54 (s, 1H), 8.23 (s, 1H), 8.21 (s, 1H), 7.56 (s, 1H), 5.82-5.71 (m, 2H), 5.12-5.05 (m, 4H), 4.24-4.20 (m, 4H), 2.66-2.61 (m, 4H), 1.48 (s, 9H).

### Step 5: Compound 33-E tert-butyl (1⁴Z, 4⁴E, 7Z)-2⁵,4³-chloro-1¹H, 4¹H-3-aza-2(4,2)-pyrimidina-1,4(4,1)-bipyrazolacyclodecaphan-7-en-3-carbamate

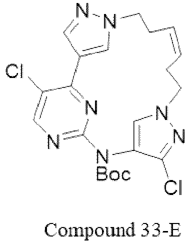

The synthesis of this step referred to the synthesis process of the fourth step of **compound 25 in Example 15.**

LC_MS: (ES⁺): m/z 477.65 [M+H] ⁺.

¹H NMR (400 MHz, CDCl₃): δ 8.59 (s, 1H), 8.28 (s, 1H), 7.83 (s, 1H), 7.46 (s, 1H), 5.67-5.61 (m, 2H), 4.44-4.35 (m, 2H), 4.27-4.14 (m, 2H), 2.74-2.57 (m, 4H), 1.49 (s, 9H).

### Step 6: Compound 33-F tert-butyl (1⁴Z, 4⁴E, 7Z)-2⁵,4³-chloro-1¹H, 4¹H-3-aza-2(4,2)-pyrimidina-1,4(4,1)-bipyrazolacyclodecaphan-3-carbamate

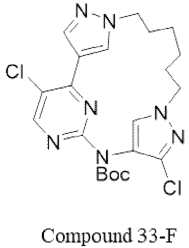

The synthesis of this step referred to the synthesis process of the first step of compound **23 in Example 14.**

LC_MS: (ES⁺): m/z 478.8 [M+H] ⁺.

¹H NMR (400 MHz, CDCl₃): δ 8.60 (s, 1H), 8.22 (s, 1H), 7.68 (s, 1H), 7.53 (s, 1H), 4.36-4.25 (m, 2H), 4.14-4.07 (m, 2H), 2.08-1.99 (m, 2H), 1.76-1.65 (m, 2H), 1.51 (s, 9H), 1.47-1.33 (m, 4H).

### Step 7: Compound 33 (1⁴Z,4⁴E,7Z)-2⁵,4³-chloro-1¹H,4¹H-3-aza-2(4,2)-pyrimidina-1,4(4,1)-bipyrazolacyclodecaphane

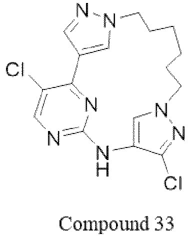

The synthesis of this step referred to the synthesis process of the sixth step of **compound 25 in Example 15.**

LC_MS: (ES⁺): m/z 377.95 [M+H] ⁺.

¹H NMR (400 MHz, DMSO-d6): δ 8.88 (s, 1H), 8.40 (s, 1H), 8.08 (s, 1H), 7.82 (s, 1H), 7.53 (s, 1H), 4.30-4.05 (m, 4H), 2.04-1.87 (m, 2H), 1.77-1.64 (m, 2H), 1.46-1.34 (m, 2H), 1.21-1.12 (m,2H).

### Example 24: Synthesis route of compound 34:

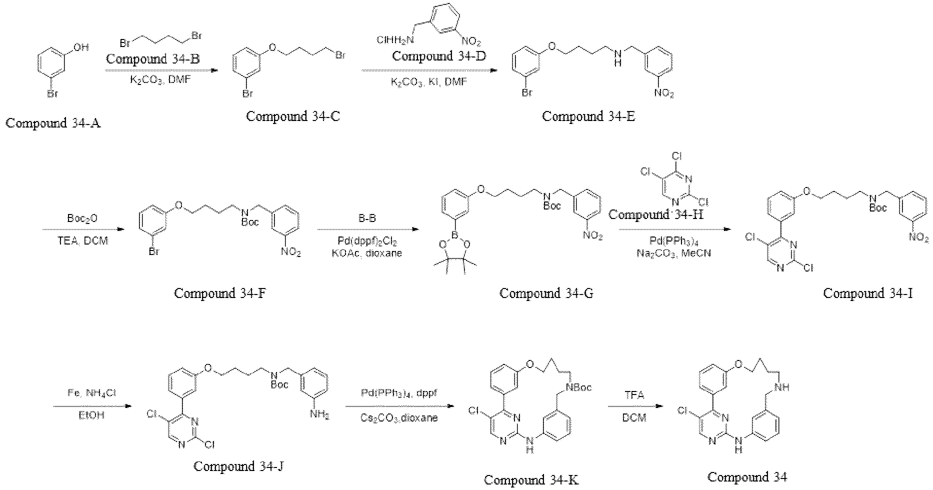

### Step 1: Compound 34-C 1-bromo-3-(4-bromobutoxy)benzene

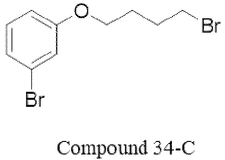

In a 100 ml single-necked flask, **compound 34-A** m-bromophenol (1 g, 1.0 eq) and **compound 34-B** 1,4-dibromobutane (6.24 g, 5.0 eq) were dissolved in DMF (12 ml), added with potassium carbonate (4 g, 5.0 eq) under stirring at room temperature, and heated to 70°C for 20 minutes. The reaction was monitored by TLC to be completed. The reaction solution was cooled to room temperature, poured into 100 ml of water, and extracted 3 times with ethyl acetate (50 ml). The organic phases were combined, backwashed twice with 100 ml of water, washed once with saturated sodium chloride, then dried and concentrated. The crude product was separated and purified by silica gel chromatography (petroleum ether: ethyl acetate = 40:1) to obtain a colorless oily **compound 34-C** 1-bromo-3-(4-bromobutoxy)benzene (2.36 g, with a purity of 60%).

¹H NMR (400 MHz, CDCl₃): δ 7.15-7.11 (m, 1H), 7.08-7.04 (m, 2H), 6.83-6.80 (m, 1H), 3.98 (t, *J =* 6.0 Hz, 2H), 3.50-3.46 (m, 2H), 2.10-2.07 (m, 2H), 1.97-1.92 (m, 2H).

### Step 2: Compound 34-E 4-(3-bromophenoxy)-N-(3-nitrobenzyl)butan-1-amine

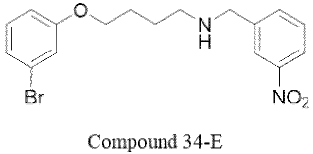

In a 100 ml single-neck flask, **compound 34-C** 1-bromo-3-(4-bromobutyloxy)benzene (2.36 g, 1.0 eq) and **compound 34-D** 3-nitrobenzylamine hydrochloride (4.36 g, 3.0 eq) were dissolved in 20 ml of DMF, added with potassium carbonate (8.5 g, 8.0 eq) and potassium iodide (1.92 g, 1.5 eq) under stirring at room temperature, and stirred at 40°C for 3 hours. The reaction was monitored by TLC to be completed. The reaction solution was cooled to room temperature, poured into 160 ml of water, and extracted twice with ethyl acetate (40 ml). The organic phases were combined, backwashed once with 150 ml of water, washed once with saturated sodium chloride, and then dried and concentrated. The crude product was separated and purified by silica gel chromatography column (petroleum ether: ethyl acetate: 7M ammonia in methanol = 10:1:0.1) to obtain a light yellow oily **compound 34-E** 4-(3-bromophenoxy)-N-(3-nitrobenzyl)but-1-amine (1.18 g).

LC_MS: (ES⁺): m/z 380.00 [M+H] ⁺.

¹H NMR (400 MHz, CDCl₃): δ 8.22 (m, 1H), 8.12-8.10 (m, 1H), 7.68 (d, *J =* 7.6 Hz, 1H), 7.49 (t, *J* = 8.0 Hz, 1H), 7.12 (t, *J* = 8.2 Hz, 1H), 7.07-7.05 (m, 1H), 7.04-7.03 (m, 1H) , 6.82-6.79 (m, 1H), 3.96 (t, *J =* 6.4 Hz, 2H), 3.91 (s, 2H), 2.71 (t, *J =* 7.0 Hz, 2H), 1.88-1.83 (m, 2H), 1.73-1.66 (m, 2H).

### Step 3: Compound 34-F tert-butyl (4-(3-bromophenoxy)butyl)(3-nitrobenzyl)carbamate

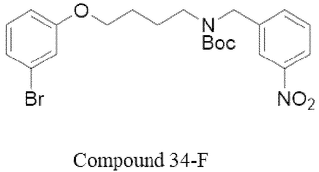

In a 100 ml single-necked flask, **compound 34-E** 4-(3-bromophenoxy)-N-(3-nitrobenzyl)but-1-amine (1.18 g, 1.0 eq) and triethylamine (380 mg, 1.2 eq) were dissolved in 20 ml of dichloromethane, added with Boc₂O (815 mg, 1.2 eq) under stirring at room temperature, and then stirred at room temperature for 4 hours. The reaction was monitored by TLC to be completed. The reaction solution was poured into 30 ml of water and extracted twice with 20 ml of dichloromethane. The organic phases were combined, backwashed with 30 ml of water, washed once with saturated sodium chloride aqueous solution (30 ml), and then dried and concentrated. The crude product was purified by silica gel chromatography (petroleum ether: ethyl acetate = 6:1) to obtain a colorless oily **compound 34-F** tert-butyl (4-(3-bromophenoxy)butyl)(3-nitrobenzyl)carbamate (1.3 g).

LC_MS: (ES⁺): m/z 380.55 [M-100]⁺.

¹H NMR (400 MHz, CDCl₃) δ 8.14-8.12 (m, 2H), 7.57 (brs, 1H), 7.52-7.48 (m, 2H), 7.14-7.10 (m, 1H), 7.08-7.06 (m, 1H), 7.02-7.01 (m, 1H), 6.80-6.78 (m, 1H), 4.53 (brs, 2H), 3.93 (t, *J=* 4.8 Hz, 2H), 3.28-3.27 (m, 2H), 1.75-1.74 (m, 4H), 1.48-1.45 (m, 9H).

### Step 4: Compound 34-G tert-butyl (3-nitrobenzyl)(4-(3-(4,4,5,5-tetramethyl-1,3,2-dioxaborolan-2-yl)phenoxy)butyl)carbamate

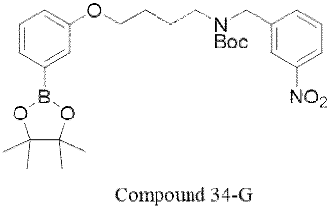

The synthesis of this step referred to the synthesis process of the sixth step of **compound 12 in Example 8.**

LC_MS: (ES⁺): m/z 526.90 [M+H] ⁺.

¹H NMR (400 MHz, CDCl₃): δ 8.12 (d, *J =* 7.6 Hz, 2H), 7.57 (brs, 1H), 7.51-7.47 (m, 1H), 7.39 (d, *J =* 7.2 Hz, 1H), 7.30-7.28 (m, 2H), 6.97-6.95 (m, 1H), 4.57-4.47 (m, 2H), 3.98 (t, *J* = 5.6 Hz, 2H), 3.34-3.27 (m, 2H), 1.74 (brs, 4H), 1.49-1.45 (m, 9H) , 1.34 (s, 12H).

### Step 5: Compound 34-I tert-butyl (4-(3-(2,5-dichloropyrimidin-4-yl)phenoxy)butyl)(3-nitrobenzyl)carbamate

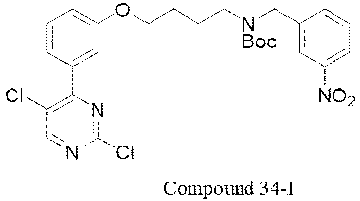

The synthesis of this step referred to the synthesis process of the seventh step of **compound 12 in Example 8.**

LC_MS: (ES⁺): m/z 547.10 [M+H] ⁺.

### Step 6: Compound 34-J tert-butyl (3-aminobenzyl)(4-(3-(2,5-dichloropyrimidin-4-yl)phenoxy)butyl)carbamate

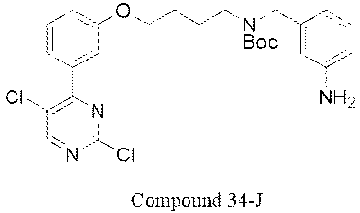

The synthesis of this step referred to the synthesis process of the fourth step of **compound 2 in Example 1.**

LC_MS: (ES⁺): m/z 516.90 [M+H] ⁺.

### Step 7: Compound 34-K tert-butyl 2⁵-chloro-11-oxa-3,6-diaza-2(4,2)-pyrimidina-1,4(1,3)-dibenzenacycloundecaphan-6-carboxylate

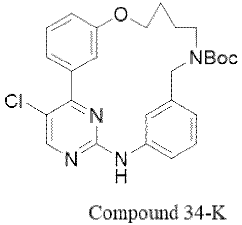

The synthesis of this step referred to the synthesis process of the eighth step of **compound 35 in Example 24.**

LC_MS: (ES⁺): m/z 481.15 [M+H] ⁺.

¹H NMR (400 MHz, d6-DMSO): δ 9.89 (s, 1H), 8.62 (s, 1H), 8.20 (brs, 1H), 7.64-7.62 (m, 2H), 7.43 (t, *J* = 8.0 Hz, 1H), 7.27 (t, *J =* 7.8 Hz, 1H), 7.18-7.16 (m, 1H), 7.10-7.08 (m, 1H) , 6.94 (d, *J =* 7.6 Hz, 1H), 4.28 (s, 2H), 4.19 (brs, 2H), 3.17 (brs, 2H), 1.68 (brs, 4H), 1.38 (s, 9H).

### Step 8: Compound 34 2⁵-chloro-11-oxa-3,6-diaza-2(4,2)-pyrimidina-1,4(1,3)-dibenzenacycloundecaphane

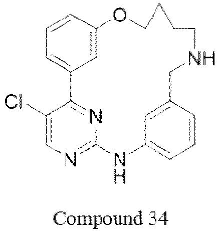

The synthesis of this step referred to the synthesis process of the sixth step of **compound 25 in Example 15.**

LC_MS: (ES⁺): m/z 381.90 [M+H] ⁺.

¹H NMR (400 MHz, d6-DMSO): δ 9.97 (s, 1H), 8.66 (s, 1H), 8.30 (s, 1H), 7.61 (m, 1H), 7.50-7.48 (m, 1H), 7.42 (t, *J =* 7.8 Hz, 1H), 7.36 (t, *J* = 7.8 Hz, 1H), 7.20-7.16 (m, 3H) , 4.28 (t, *J* = 5.8 Hz, 2H), 4.06 (s, 2H), 3.04-3.00 (m, 2H), 1.83-1.76 (m, 4H).

### Example 25: Synthesis route of compound 35:

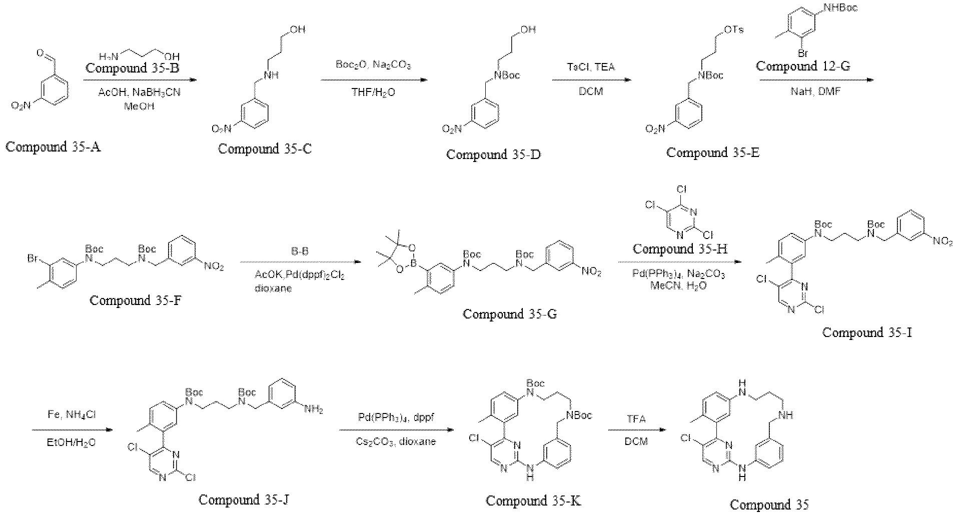

### Step 1: Compound 35-C 3-((3-nitrobenzyl)amino)propan-1-ol

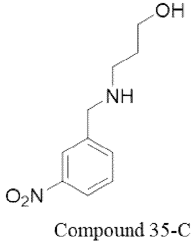

A mixture of **compound 35**-A 3-nitrobenzaldehyde (3 g, 19.8 mmol), **compound 35-B** 3-aminopropan-1-ol (1.5 g, 19.8 mmol) and acetic acid (2.4 g, 39.6 mmol) in methanol (30 ml) was stirred at room temperature for 30 minutes, and then added with sodium cyanoborohydride (2.5 g, 39.6 mmol). The resulting mixture was stirred at room temperature for 15 hours. The reaction was shown to be completed by TLC. The reaction mixture was concentrated and the residue was quenched with saturated sodium bicarbonate aqueous solution (30 ml). The aqueous phase was extracted with ethyl acetate (20 ml × 3). The organic layers were combined, washed with saturated brine (30 ml), dried over anhydrous sodium sulfate, and concentrated under reduced pressure. The resulting residue was separated and purified by silica gel column (eluted with a dichloromethane solution containing 5% methanol) to obtain a light yellow oily **compound 35-C** 3-((3-nitrobenzyl)amino)propan-1-ol (3.58 g, 86%).

LC_MS: (ES⁺): m/z 211.00 [M+H] ⁺.

¹H NMR (400 MHz, CDCl₃): δ 1.74-1.79 (m, 2H), 2.90 (t, *J* = 6.0 Hz, 2H), 3.81 (t, *J* = 5.2 Hz, 2H), 3.92 (s, 2H), 7.52 (t, *J =* 7.6 Hz, 1H), 7.68 (d, *J =* 7.6 Hz, 1H), 8.12-8.17 (m, 2H).

### Step 2: Compound 35-D tert-butyl (3-hydroxypropyl)(3-nitrobenzyl)carbamate

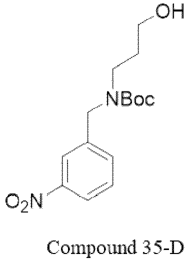

Di-tert-butyl dicarbonate (985 mg, 4.52 mmol) was added to a mixed solution of **compound 35-C** 3-((3-nitrobenzyl)amino)propan-1-ol (950 mg, 4.52 mmol) and sodium carbonate (958 mg, 9.04 mmol) in tetrahydrofuran (10 ml) and water (10 ml) at 0°C. The reaction mixture was warmed to room temperature and stirred for 2 hours. The reaction was monitored by TLC to be completed. The reaction mixture was distributed between ethyl acetate (20 ml) and water (20 ml). The organic layer was collected, dried over anhydrous sodium sulfate, and concentrated under reduced pressure to obtain a yellow oily compound 35-D tert-butyl (3-hydroxypropyl)(3-nitrobenzyl)carbamate (1.25 g, crude), which was used directly in the next step without further purification.

LC_MS: (ES⁺): m/z 254.80 [M+H] ⁺.

¹H NMR (400 MHz, CDCl₃): δ 1.46 (s, 9H), 1.70 (s, 2H), 3.39-3.61 (m, 5H), 4.47 (s, 2H), 7.50-7.59 (m, 2H), 8.13-8.15 (m, 2H).

### Step 3: Compound 35-E 3-((tert-butoxycarbonyl)(3-nitrobenzyl)amino)propyl 4-methylbenzenesulfonate

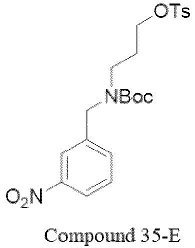

4-Toluenesulfonyl chloride (898 mg, 4.71 mmol) was added to a solution of **compound 35-D** tert-butyl (3-hydroxypropyl)(3-nitrobenzyl)carbamate (1.46 g, 4.71 mmol) and triethylamine (953 mg, 9.42 mmol) in dichloromethane (10 ml) at 0°C. The reaction mixture was warmed to room temperature and stirred for 14 hours. The reaction was monitored by TLC to be completed. The reaction mixture was distributed between dichloromethane (20 ml) and water (20 ml). The organic layer was collected, dried over anhydrous sodium sulfate, and concentrated under reduced pressure. The crude product was separated and purified by silica gel column chromatography to obtain a colorless oily **compound 35-E** 3-((tert-butoxycarbonyl)(3-nitrobenzyl)amino)propyl 4-methylbenzenesulfonate (1.48 g).

LC_MS: (ES⁺): m/z 364.9 [M+H] ⁺.

¹H NMR (400 MHz, CDCl₃): δ 1.44 (s, 9H), 1.90 (s, 2H), 2.45 (s, 3H), 3.27 (s, 2H), 4.04 (t, *J* = 6.0 Hz, 2H), 4.46 (s, 2H), 7.34 (d, *J* = 8.0 Hz, 2H), 7.49-7.54 (m, 2H), 7.77 (d, *J* = 8.4 Hz, 2H), 8.06-8.14 (m, 2H).

### Step 4: Compound 35-F tert-butyl (3-((3-bromo-4-methylphenyl)(tert-butoxycarbonyl)amino)propyl)(3-nitrobenzyl)carbamate

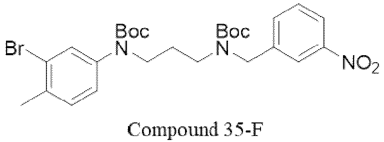

Sodium hydride (60% in kerosene) (203 mg, 5.08 mmol) was added to a solution of **compound 12-G** tert-butyl (3-bromo-4-methylphenyl)carbamate in anhydrous N,N-dimethylformamide (10 ml). The reaction mixture was stirred at room temperature for 30 minutes of reaction. Then, **compound 35-E** 3-((tert-butyloxycarbonyl)(3-nitrobenzyl)amino)propyl 4-methylbenzenesulfonate (1.08 g, 2.54 mmol) was added to the system at 0°C, and the reaction mixture was stirred at room temperature for 15 hours. The reaction was monitored by TLC to be completed. The reaction mixture was quenched by water (100 ml) and extracted with ethyl acetate (30 ml x 2). The organic layers were combined, washed with saturated brine (100 ml), dried over anhydrous sodium sulfate, and concentrated under reduced pressure. The obtained crude product was separated and purified by silica gel column chromatography (eluted with a petroleum ether solution containing 20% ethyl acetate) to obtain a yellow oily **compound 35-F** tert-butyl (3-((3-bromo-4-methylphenyl)(tert-butoxycarbonyl)amino)propyl)(3-nitrobenzyl)carbamate (930 mg, 63%).

¹H NMR (400 MHz, CDCl₃): δ 1.40 (s, 9H), 1.46 (s, 9H), 1.72-1.75 (m, 2H), 2.38 (s, 3H), 3.19-3.23 (m, 2H), 3.60 (t, *J =* 6.8 Hz, 2H), 4.47 (s, 2H), 6.96-7.00 (m, 1H), 7.16 (d, *J =* 8.0 Hz, 2H), 7.31 (s, 1H), 7.46-7.52 (m, 2H), 8.06-8.12 (m, 2H).

### Step 5: Compound 35-G tert-butyl (3-((tert-butyloxycarbonyl)(4-methyl-3-(4,4,5,5-tetramethyl-1,3,2-dioxaborolan-2-yl)phenyl)ami no)propyl)(3-nitrobenzyl)carbamate

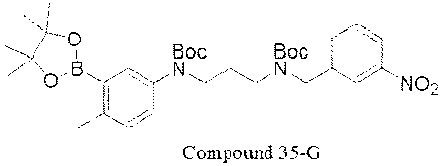

The synthesis of this step referred to the synthesis process of the sixth step of **compound 12 in Example 8.**

LC_MS: (ES⁺): m/z 725.15 [M+H] ⁺

¹H NMR (400 MHz, CDCl₃): δ 1.33 (s, 12H), 1.38 (s, 9H), 1.46 (s, 9H), 1.75 (s, 2H), 2.52 (s, 3H), 3.19-3.24 (m, 2H), 3.62 (t, *J* = 6.4 Hz, 2H), 4.46 (s, 2H), 7.05-7.11 (m, 2H), 7.44-7.52 (m, 3H), 8.07-8.11 (m, 2H).

### Step 6: Compound 35-I tert-butyl (3-((tert-butyloxycarbonyl)(3-(2,5-dichloropyrimidin-4-yl)-4-methylphenyl)amino)propyl)(3-nitr obenzyl)carbamate

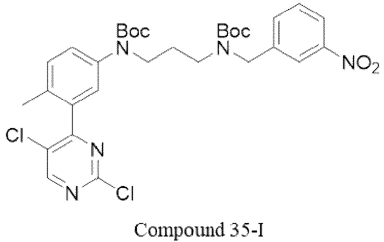

The synthesis of this step referred to the synthesis process of the seventh step of **compound 12 in Example 8.**

LC_MS: (ES⁺): m/z 645.95 [M+H] ⁺.

¹H NMR (400 MHz, CDCl₃): δ 1.39 (s, 9H), 1.44 (s, 9H), 1.75-1.79 (m, 2H), 2.21 (s, 3H), 3.19-3.25 (m, 2H), 3.63 (t, *J =* 4.8 Hz, 2H), 4.47 (s, 2H), 7.07-7.17 (m, 2H), 7.29-7.34 (m, 1H), 7.46-7.52 (m, 2H), 8.05-8.09 (m, 2H), 8.69 (s, 1H).

### Step 7: Compound 35-J tert-butyl 3-aminobenzyl (3-((tert-butoxycarbonyl)(3-(2,5-dichloropyrimidin-4-yl)-4-methylphenyl)amino)propyl)carbamat e

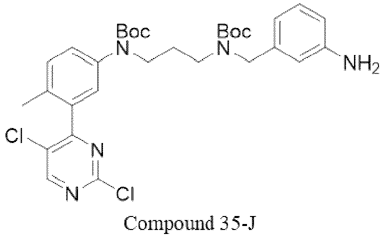

The synthesis of this step referred to the synthesis process of the fourth step of **compound 2 in Example 1.**

LC_MS: (ES⁺): m/z 517.60 [M+H] ⁺

¹H NMR (400 MHz, CDCl₃): δ 1.40 (s, 9H), 1.43 (s, 9H), 1.76 (s, 2H), 2.21 (s, 3H), 3.13-3.21 (m, 2H), 3.61-3.66 (m, 4H), 4.30 (s, 2H), 6.50-6.55 (m, 3H), 7.03-7.10 (m, 2H), 7.17 (s, 1H), 7.25 (s, 1H), 8.68 (s, 1H).

### Step 8: Compound 35-K tert-butyl 2⁵-chloro-1⁶-methyl-3,6,10-triaza-2(4,2)-pyrimidina-1,4(1,3)-dibenzenacyclodecaphan-6,10-dicar boxylate

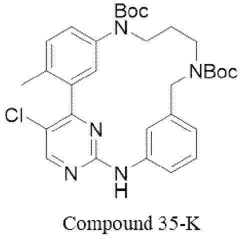

Tetrakistriphenylphosphine palladium (51 mg, 0.044 mmol) was added to a solution of **compound 35-J** tert-butyl 3-aminobenzyl (3-((tert-butoxycarbonyl)(3-(2,5-dichloropyrimidin-4-yl)-4-methylphenyl)amino)propyl)carbamat e (90 mg, 0.146 mmol), 1,1'-bis(diphenylphosphino)ferrocene (49 mg, 0.088 mmol) and cesium carbonate (143 mg, 0.44 mmol) in 1,4-dioxane (30 ml) in a nitrogen atmosphere at room temperature. The reaction system was replaced with nitrogen three times. The reaction mixture was stirred at 110°C for 16 hours. The reaction was monitored by TLC to be completed. The reaction mixture was filtered, the filtrate was concentrated under reduced pressure, and the obtained crude product was separated and purified by silica gel column chromatography (eluted with petroleum ether containing 25% ethyl acetate) to obtain a white solid **compound 35-K** tert-butyl 2⁵-chloro-1⁶-methyl-3,6,10-triaza-2(4,2)-pyrimidina-1,4(1,3)-dibenzocyclodecane-6,10-dicarbox ylate (35 mg, 41%).

LC_MS: (ES⁺): m/z 580.25 [M+H] ⁺.

¹H NMR (400 MHz, CDCl₃): δ 1.44 (s, 9H), 1.45 (s, 9H), 1.85 (s, 2H), 2.31 (s, 3H), 2.99-3.04 (m, 1H), 3.40-3.48 (m, 2H), 4.06-4.15 (m, 2H), 4.25-4.34 (m, 1H), 6.83-6.85 (s, 2H), 7.23 (s, 4H), 7.30 (s, 1H), 8.20-8.27 (m, 1H), 8.39 (s, 1H).

### Step 9: Compound 35 2⁵-chloro-1⁶-methyl-3,6,10-triaza-2(4,2)-pyrimidina-1,4(1,3)-dibenenacyclodecaphane

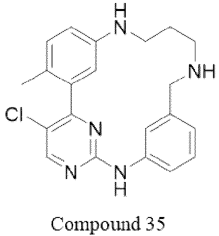

The synthesis of this step referred to the synthesis process of the sixth step of **compound 25 in Example 15.**

LC_MS: (ES⁺): m/z 380.10 [M+H] ⁺.

¹H NMR (400 MHz, DMSO-d6): δ 1.46-1.56 (m, 1H), 1.70-1.80 (m, 1H), 2.12 (s, 3H), 2.69-2.75 (m, 1H), 3.00-3.04 (m, 1H), 3.40-3.52 (m, 3H), 3.73 (d, *J =* 12.8 Hz, 1H), 5.61-5.65 (m, 1H), 6.66-6.68 (m, 1H), 6.72-6.73 (m, 1H), 6.86 (d, *J* = 7.6 Hz, 1H), 6.96 (d, *J* = 8.0 Hz, 1H), 7.10 (d, *J= 8.0* Hz, 1H), 7.20 (t, *J =* 7.6 Hz, 1H), 8.58 (s, 1H), 8.65 (s, 1H), 9.91 (s, 1H).

### Example 26: Synthesis route of compound 36:

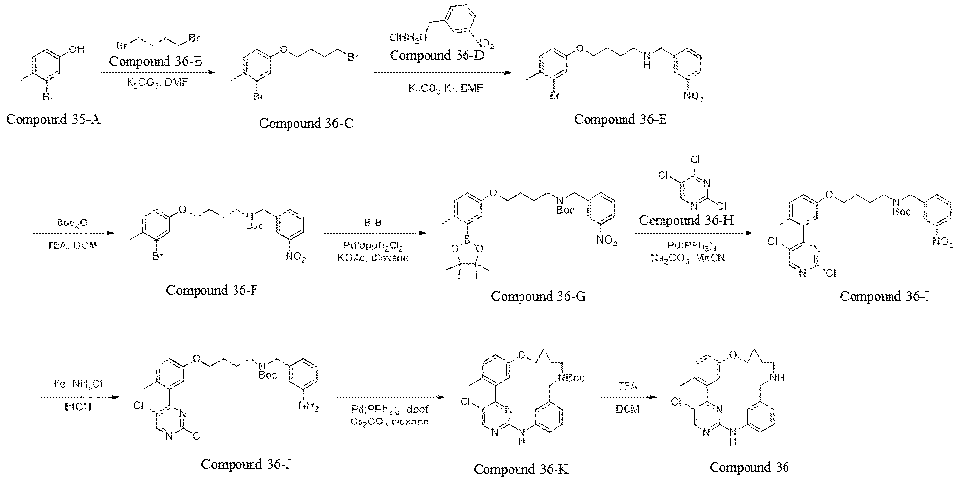

### Step 1: Compound 36-C 2-bromo-4-(4-bromobutoxy)-1-methylbenzene

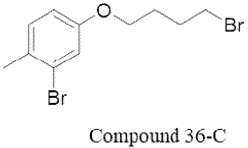

The synthesis of this step referred to the synthesis process of the first step of **compound 34 in Example 24.**

¹H NMR (400 MHz, CDCl₃): δ 7.11 (d, *J* = 8.4 Hz, 1H), 7.09 (d, *J* = 2.8 Hz, 1H), 6.76-6.73 (m, 1H), 3.95 (t, *J* = 6.0 Hz, 2H), 3.50-3.48 (m, 2H), 2.32 (s, 3H), 2.07-2.05 (m, 2H), 1.96-1.91 (m, 2H).

### Step 2: Compound 36-E 4-(3-bromo-4-methylphenoxy)-N-(3-nitrobenzyl)but-1-amine

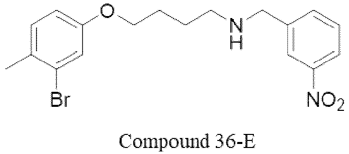

The synthesis of this step referred to the synthesis process of the second step of **compound 34 in Example 24.**

LC_MS: (ES⁺): m/z 392.7 [M+H] ⁺.

### Step 3: Compound 36-F tert-butyl (4-(3-bromo-4-methylphenoxy)butyl)(3-nitrobenzyl)carbamate

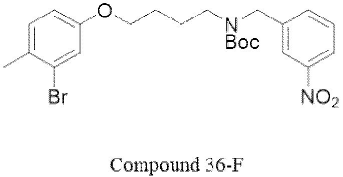

The synthesis of this step referred to the synthesis process of the third step of **compound 34 in Example 24.**

¹H NMR (400 MHz, CDCl₃): δ 8.13-8.07 (m, 2H), 7.57 (brs, 1H), 7.52-7.48 (m, 1H), 7.10 (d, *J =* 8.4 Hz, 1H), 7.05 (d, *J =* 2.4 Hz, 1H), 6.73-6.70 (m, 1H), 4.54-4.50 (m, 2H), 3.91 (t, *J* = 5.4 Hz, 2H), 3.35-.3.25 (m, 2H), 2.32 (s, 3H), 1.72 (brs, 4H), 1.49-1.44 (m, 9H).

### Step 4: Compound 36-G tert-butyl (4-(4-methyl-3-(4,4,5,5-tetramethyl-1,3,2-dioxaborolan-2-yl)phenoxybutyl)3-nitrobenzyl)carbam ate

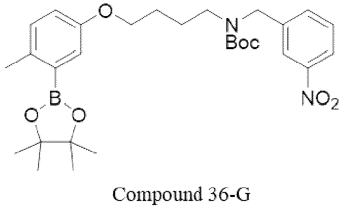

The synthesis of this step referred to the synthesis process of the sixth step of **compound 12 in Example 8.**

¹H NMR (400 MHz, CDCl₃): δ 8.13-8.11 (m, 2H), 7.56 (brs, 1H), 7.52-7.47 (m, 1H), 7.25 (s, 1H), 7.06 (d, *J =* 8.0 Hz, 1H), 6.84-6.81 (m, 1H), 4.54-4.50 (m, 2H), 3.94 (t, *J =* 5.4 Hz, 2H), 3.34-.3.26 (m, 2H), 2.46 (s, 3H), 1.72 (brs, 4H), 1.49-1.43 (m, 9H), 1.26 (s, 12H).

### Step 5: Compound 36-I tert-butyl (4-(3-(2,5-dichloropyrimidin-4-yl)-4-methylphenoxy)butyl)(3-nitrobenzyl)carbamate

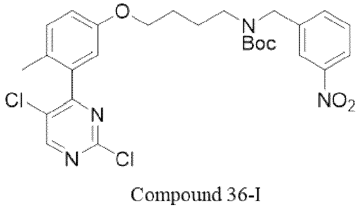

The synthesis of this step referred to the synthesis process of the seventh step of **compound 12 in Example 8.**

LC_MS: (ES⁺): m/z 561.10 [M+H] ⁺.

### Step 6: Compound 36-J tert-butyl (3-aminobenzyl)(4-(3-(2,5-dichloropyrimidin-4-yl)-4-methylphenoxy)butyl)carbamate

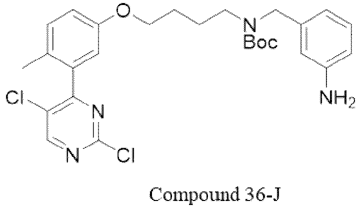

The synthesis of this step referred to the synthesis process of the fourth step of **compound 2 in Example 1.**

LC_MS: (ES⁺): m/z 530.95 [M+H] ⁺.

### Step 7: Compound 36-K tert-butyl 2⁵-chloro-1⁶-methyl-11-oxy-3,6-diaza-2(4,2)-pyrimidina-1,4(1,3)-dibenzenacycloundecaphan-6-c arboxylate

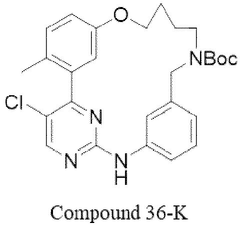

The synthesis of this step referred to the synthesis process of the eighth step of **compound 35 in Example 24.**

LC_MS: (ES⁺): m/z 495.20 [M+H] ⁺.

¹H NMR (400 MHz, d6-DMSO): δ 9.87 (s, 1H), 8.60 (s, 1H), 8.08 (brs, 1H), 7.24-7.20 (m, 2H), 7.14 (d, *J =* 2.8 Hz, 1H), 7.05-7.02 (m, 2H), 6.88 (d, *J =* 7.6 Hz, 1H), 4.31-4.17 (m, 4H) , 3.21 (brs, 1H) , 2.99 (brs, 1H), 2.14 (s, 3H), 1.70-1.59 (m, 4H), 1.35 (s, 9H).

### Step 8: Compound 36 2⁵-chloro-1⁶-methyl-11-oxy-3,6-diaza-2(4,2)-pyrimidina-1,4(1,3)-dibenzocycloundecaphane

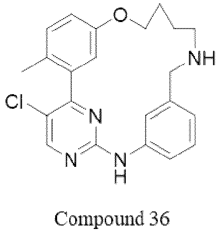

The synthesis of this step referred to the synthesis process of the sixth step of **compound 25 in Example 15.**

LC_MS: (ES⁺): m/z 395.25 [M+H] ⁺.

¹H NMR (400 MHz, d6-DMSO): δ 9.92 (s, 1H), 8.63 (s, 1H), 8.23 (brs, 1H), 7.28 (t, *J =* 7.6 Hz, 1H), 7.23 (d, *J =* 2.4 Hz, 1H), 7.19 (d, *J =* 8.4 Hz, 1H), 7.08-6.99 (m, 3H), 4.36-4.30 (m, 1H), 4.18-4.08 (m, 1H), 3.93-3.84 (m, 2H), 2.91-2.79 (m, 2H), 2.08 (s, 3H), 1.85-1.57 (m, 4H).

### Example 27: Synthesis route of compound 37:

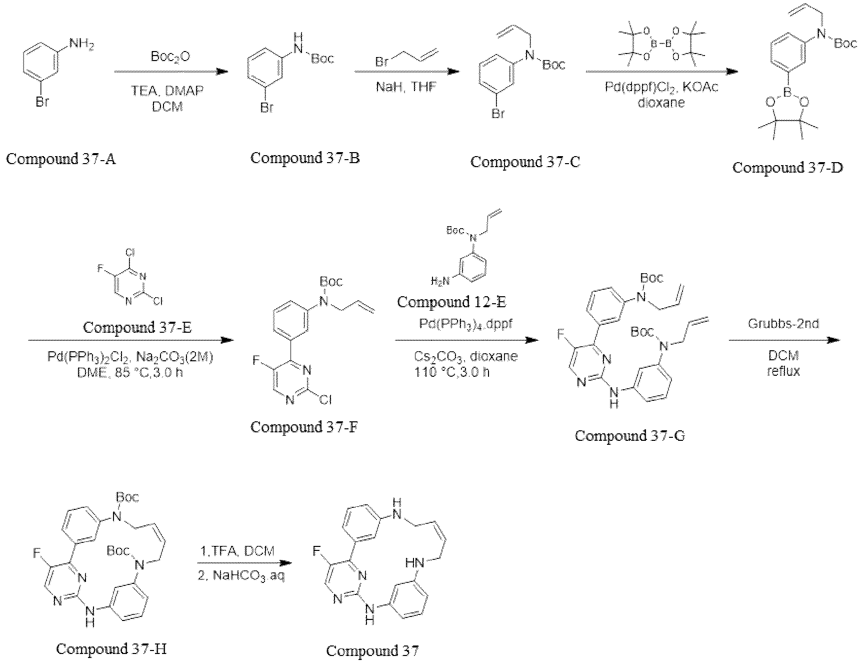

### Step 1: Compound 37-B tert-butyl (3-bromophenyl)carbamate

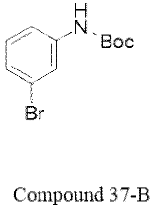

The synthesis of this step referred to the synthesis process of the fourth step of **compound 12 in Example 8.**

LC-MS: (ES⁺): m/z 217.0 [M+1-56] ⁺.

### Step 2: Compound 37-C tert-butyl allyl(3-bromophenyl)carbamate

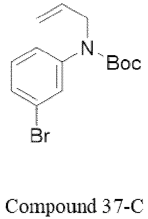

The synthesis of this step referred to the synthesis process of the fifth step of **compound 12 in Example 8.**

### Step 3: Compound 37-D tert-butyl allyl(3-(4,4,5,5-tetramethyl-1,3,2-dioxaborolan-2-yl) phenyl)carbamate

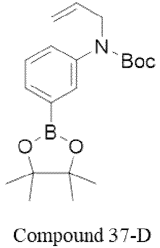

The synthesis of this step referred to the synthesis process of the sixth step of **compound 12 in Example 8.**

### Step 4: Compound 37-F tert-butyl allyl(3-(2-chloro-5-fluoropyrimidin-4-yl)phenyl) carbamate

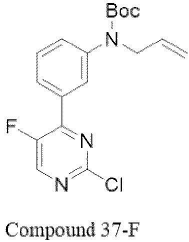

The synthesis of this step referred to the synthesis process of the seventh step of **compound 12 in Example 8.**

¹H NMR (400 MHz, CDCl₃) &: 8.55 (d, J= 4.0 Hz, 1H), 8.07 (s, 1H), 7.97 (dd, J= 4.0, 8.0 Hz, 1H), 7.53 - 7.45 (m, 2H), 6.01 - 5.02 (m, 1H), 5.23 - 5.17 (m, 2H), 4.31 (d, *J =* 8.0 Hz, 2H), 1.50 (s, 9H).

### Step 5: Compound 37-G tert-butyl allyl(3-(2-((3-(allyl(tert-butoxycarbonyl)amino) phenyl)amino)-5-fluoropyrimidin-4-yl) phenyl) carbamate

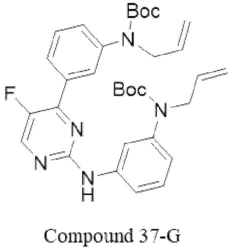

The synthesis of this step referred to the synthesis process of the eighth step of **compound 12 in Example 8.**

LC-MS: (ES⁺): m/z 574.0 [M-H] ⁺.

### Step 6: Compound 37-H di-tert-butyl (Z)-2⁵-fluoro-3,5,10-triaza-2(4,2)-pyrimidina-1,4(1,3)-dibenzenacyclooctyna-7-en-5,10-tricarbox ylate

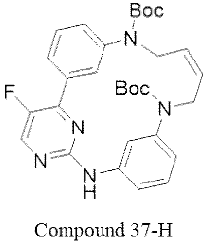

The synthesis of this step referred to the synthesis process of the ninth step of **compound 12 in Example 8.**

### Step 7: Compound 37 (Z)-2⁵-fluoro-3,5,10-triaza-2(4,2)-pyrimidina-1,4(1,3)-dibenzenacyclooctyna-7-ene

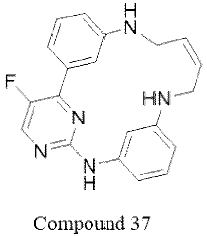

The synthesis of this step referred to the synthesis process of the tenth step of **compound 12 in Example 8.**

LC-MS: (ES⁺): m/z 348.12 [M+H] ⁺.

¹H NMR (400 MHz, DMSO-*d*₆) *δ*: 9.42 (s, 1H), 8.54 (d, *J =* 4.0 Hz, 1H), 7.52 (brs, 1H), 7.29 (s, 1H), 7.23 - 7.17 (m, 2H), 6.92 (brs, 1H), 6.85 (dd, *J =* 4.0, 8.0 Hz, 1H), 6.40 - 6.31 (m, 3H), 5.99 (brs, 1H), 5.52 (d, *J =* 16.0 Hz, 1H), 5.42 (d, *J =* 16.0 Hz, 1H), 3.71 (s, 2H), 3.57 (s, 2H).

### Example 28: Synthesis route of compound 38:

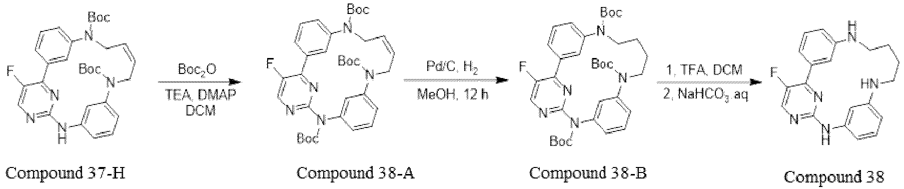

### Step 1: Compound 38-A tri-tert-butyl (Z) 2⁵-fluoro-3,5,10-triaza-2(4,2)-pyrimidina-1,4(1,3)-dibenzenacyclodecaphan-3,5,10-tricarboxylate

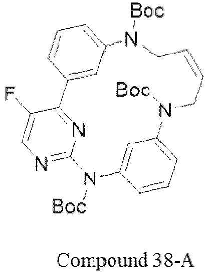

The synthesis of this step referred to the synthesis process of the third step of **compound 25 in Example 15.**

LC-MS: (ES⁺): m/z 648.3 [M+H] ⁺.

### Step 2: Compound 38-B tri-tert-butyl 2⁵-fluoro-3,5,10-triaza-2(4,2)-pyrimidina-1,4(1,3)-dibenzenacyclodecaphan-3,5,10-tricarboxylate

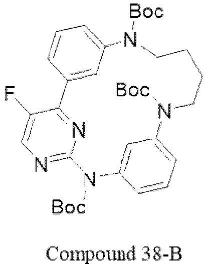

The synthesis of this step referred to the synthesis process of the first step of compound **4 in Example 2.**

LC-MS: (ES⁺): m/z 650.4 [M+H] ⁺.

### Step 2: Compound 38 2⁵-fluoro-3,5,10-triaza-2(4,2)-pyrimidina-1,4(1,3)-dibenzenacyclodecaphane

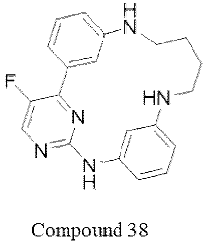

The synthesis of this step referred to the synthesis process of the sixth step of **compound 25 in Example 15.**

LC-MS: (ES⁺): m/z 350.1 [M+H] ⁺.

¹H NMR (400 MHz, DMSO-*d*₆) *δ*: 9.79 (s, 1H), 8.59 (d, *J =* 4.0 Hz, 1H), 8.04 (s, 1H), 7.35 (s, 1H), 7.31 - 7.15 (m, 3H), 6.82 - 6.75 (m, 2H), 6.62 (s, 1H),3.25 (s, 2H), 3.13 (t, *J =* 4.0 Hz, 2H), 1.73 - 1.62 (m, 2H), 1.58 - 1.51 (m, 2H).

### Example 29: Synthesis route of compound 39:

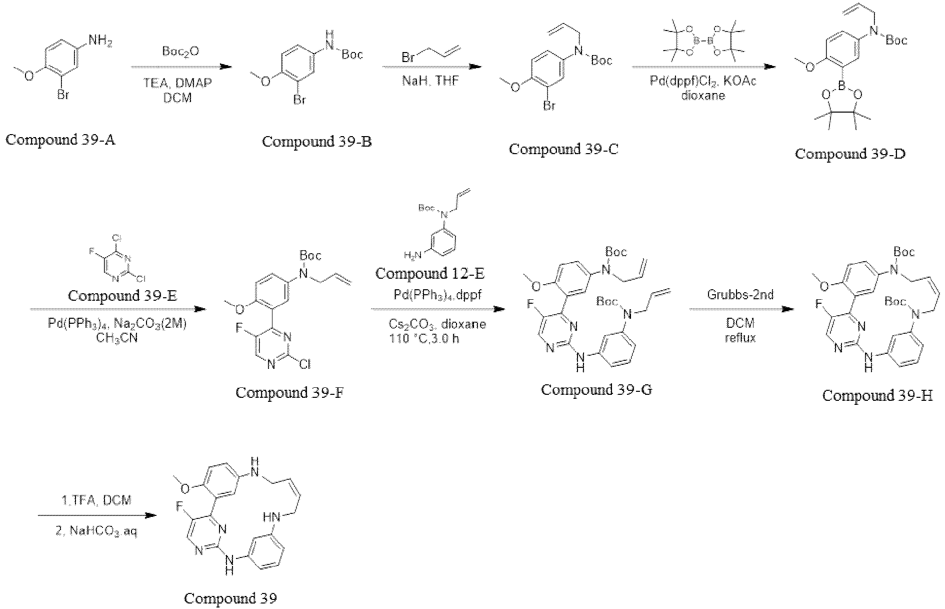

### Step 1: Compound 39-B tert-butyl (3-bromo-4-methoxyphenyl)carbamate

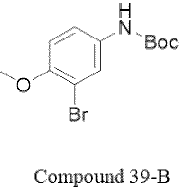

The synthesis of this step referred to the synthesis process of the fourth step of **compound 12 in Example 8.**

### Step 2: Compound 39-C tert-butyl allyl(3-bromo-4-methoxyphenyl)carbamate

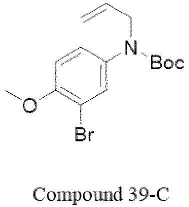

The synthesis of this step referred to the synthesis process of the fifth step of **compound 12 in Example 8.**

¹H NMR (400 MHz, CDCl₃) *δ*: 7.44 (d, *J =* 4.0 Hz, 1H), 7.15 (d, *J =* 8.0 Hz, 1H), 6.85 (dd, *J =* 4.0, 8.0 Hz, 1H), 5.95 - 5.85 (m, 1H), 5.18 - 5.12 (m, 2H), 4.18 (d, *J =* 8.0 Hz, 2H), 3.90 (s, 3H), 1.46 (s, 9H).

### Step 3: Compound 39-D tert-butyl allyl(4-methoxy-3-(4,4,5,5-tetramethyl-1,3,2-dioxaborolan-2-yl)phenyl) carbamate

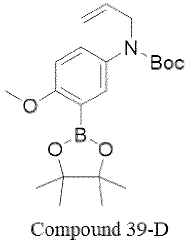

The synthesis of this step referred to the synthesis process of the sixth step of **compound 12 in Example 8.**

¹H NMR (400 MHz, CDCl₃) *δ*: 7.44 (s, 1H), 7.15 (d, *J =* 8.0 Hz, 1H), 6.85 (dd, *J =* 4.0, 8.0 Hz, 1H), 5.93 - 5.87 (m, 1H), 5.18 - 5.10 (m, 2H), 4.18 (d, *J=* 8.0 Hz, 2H), 3.90 (s, 3H), 1.46 (s, 12H), 1.31 (s, 9H).

### Step 4: Compound 39-F tert-butyl allyl(3-(2-chloro-5-fluoropyrimidin-4-yl)-4-methoxyphenyl) carbamate

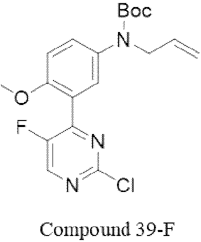

The synthesis of this step referred to the synthesis process of the seventh step of **compound 12 in Example 8.**

¹H NMR (400 MHz, CDCl₃) *δ*: 8.49 (d, *J =* 4.0 Hz, 1H), 7.42 - 7.37 (m, 2H), 6.97 (d, *J* = 8.0 Hz, 1H), 5.98 - 5.89 (m, 1H), 5.21 - 5.16 (m, 2H), 4.24 (d, *J =* 8.0 Hz, 2H), 3.86 (s, 3H), 1.47 (s, 9H).

### Step 5: Compound 39-G tert-butyl allyl(3-(2-((3-(allyl(tert-butoxycarbonyl)amino) phenyl)amino)-5-fluoropyrimidin-4-yl)-4-methoxyphenyl)carbamate

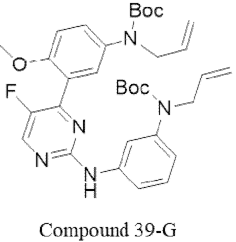

The synthesis of this step referred to the synthesis process of the eighth step of **compound 12 in Example 8.**

LC-MS: (ES⁺): m/z 606.0 [M+H] ⁺.

### Step 6: Compound 39-H di-tert-butyl (Z)-2⁵-fluoro-1⁶-methoxy-3,5,10-triaza-2(4,2)-pyrimidina-1,4(1,3)-dibenzenacyclooctyna-7-en-5, 10-dicarboxylate

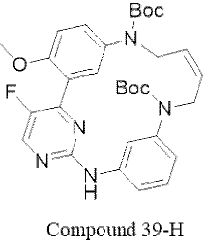

The synthesis of this step referred to the synthesis process of the ninth step of **compound 12 in Example 8.**

### Step 7: Compound 12 (Z)-2⁵-fluoro-1⁶-methoxy-3,5,10-triaza-2(4,2)-pyrimidina-1,4(1,3)-dibenzenacyclooctyna-7-ene

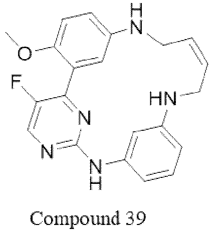

The synthesis of this step referred to the synthesis process of the tenth step of **compound 12 in Example 8.**

LC-MS: (ES⁺): m/z 378.17 [M+H] ⁺.

¹H NMR (400 MHz, DMSO-*d*₆) *δ*: 9.41 (s, 1H), 8.46 (d, *J =* 4.0 Hz, 1H), 7.76 (brs, 1H), 7.00 - 6.84 (m, 4H), 6.71 (s, 1H), 6.46 (s, 1H), 6.22 (s, 1H), 5.48 (s, 2H), 3.74 (s, 3H), 3.69 (s, 2H), 3.57 (s, 2H).

### Example 30: Synthesis route of compound 40:

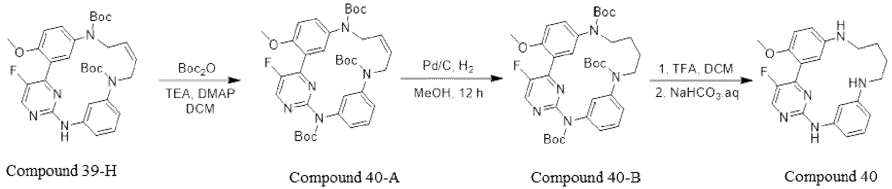

### Step 1: Compound 40-A tri-tert-butyl (Z) 2⁵-fluoro-1⁶-methoxy-3,5,10-triaza-2(4,2)-pyrimidina-1,4(1,3)-dibenzenacyclodecaphan-7-en-3,5 ,10-tricarboxylate

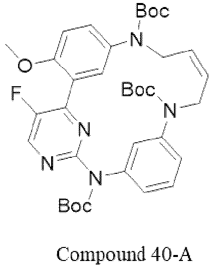

The synthesis of this step referred to the synthesis process of the third step of **compound 25 in Example 15.**

LC-MS: (ES⁺): m/z 678.3 [M+H] ⁺.

### Step 2: Compound 40-B tri-tert-butyl 2⁵-fluoro-1⁶-methoxy-3,5,10-triaza-2(4,2)-pyrimidina-1,4(1,3)-dibenzenacyclodecaphan-3,5,10-tr icarboxylate

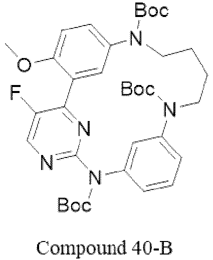

The synthesis of this step referred to the synthesis process of the first step of **compound 4 in Example 2.**

LC-MS: (ES⁺): m/z 680.3 [M+H] ⁺.

### Step 3: Compound 40 2⁵-fluoro-1⁶-methoxy-3,5,10-triaza-2(4,2)-pyrimidina-1,4(1,3)-dibenzenacyclodecaphane

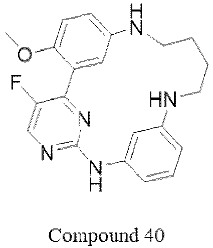

The synthesis of this step referred to the synthesis process of the sixth step of **compound 25 in Example 15.**

LC-MS: (ES⁺): m/z 380.2 [M+H] ⁺.

¹H NMR (400 MHz, DMSO-*d*₆) *δ:* 9.51 (s, 1H), 8.51 (s, 1H), 8.02 (s, 1H), 7.04 - 6.87 (m, 4H), 6.56 (s, 1H), 6.35 (s, 1H), 3.75 (s, 3H), 3.19 (d, *J =* 8.0 Hz, 2H), 3.06 (t, *J =* 8.0 Hz, 2H), 1.64 - 1.55 (m, 2H), 1.51 - 1.43 (m, 2H).

### Example 31: Synthesis route of compound 41:

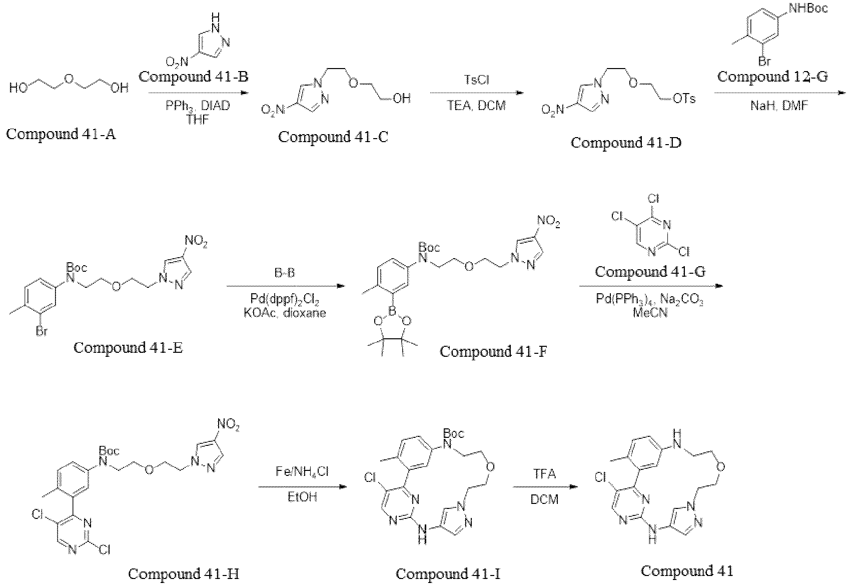

### Step 1: Compound 41-C 2-(2-(4-nitro-1H-pyrazol-1-yl)ethoxy)ethan-1-ol

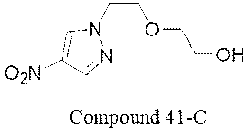

In a 100 ml three-necked flask with a thermometer, **compound 41-A** diethylene glycol (7 g, 66.3 mmol), **compound 41-B** 4-nitropyrazole (5 g, 44.2 mmol) and triphenylphosphine (23.2 g, 88.4 mmol) were added to dry tetrahydrofuran (50 ml). The reaction solution was replaced nitrogen three times, kept at a temperature between 0°C and 5°C, slowly added with diisopropyl azodicarboxylate (15.4 g, 88.4 mmol) dropwise, and then stirred at room temperature overnight. The reaction was monitored by TLC and LCMS to be completed. The reaction solution was spin-dried, the crude product was slurried with methyl tert-butyl ether for 30 minutes, the precipitated solid (triphenylphosphine oxide) was filtered, the filtrate was collected and spin-dried, and the crude filtrate was separated and purified by silica gel column chromatography (PE: EA= 5:1 to DCM: MeOH=15:1) to obtain a light yellow liquid **compound 41-C** 2-(2-(4-nitro-1H-pyrazol-1-yl)ethoxy)ethane-1-ol (6.5 g).

LC_MS: (ES⁺): m/z 201.75 [M+H] ⁺.

### Step 3: Compound 41-D 2-(2-(4-nitro-1H-pyrazol-1-yl)ethoxy)ethyl 4-methylbenzenesulfonate

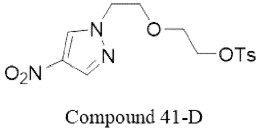

The synthesis of this step referred to the synthesis process of the third step of **compound 35 in Example 25.**

LC_MS: (ES⁺): m/z 356.40 [M+H] ⁺.

### Step 4: Compound 41-E tert-butyl (3-bromo-4-methylphenyl)(2-(2-(4-nitro-1H-pyrazol-1-yl)ethoxy)ethyl)carbamate

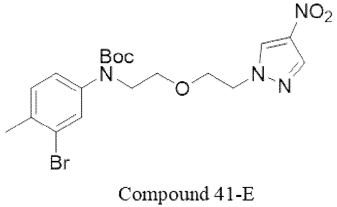

The synthesis of this step referred to the synthesis process of the fourth step of **compound 35 in Example 25.**

LC_MS: (ES⁺): m/z 412.20 [M-56] ⁺.

¹H NMR (400 MHz, CDCl₃): δ 8.06 (d, *J* = 5.2 Hz, 2H), 7.39 (brs, 1H), 7.17 (d, *J* = 8.0 Hz, 1H), 6.99 (d, *J =* 8.0 Hz, 1H), 4.26 (t, *J =* 4.8 Hz, 2H), 3.79-3.73 (m, 4H), 3.58 (t, *J =* 5.6 Hz, 1H), 2.38 (s, 3H), 1.42 (s, 9H).

### Step 5: Compound 41-F tert-butyl (4-methyl-3-(4,4,5,5-tetramethyl-1,3,2-dioxaborolan-2-yl)phenyl)(2-(2-(4-nitro-1H-pyrazol-)1-yl )ethoxy) ethyl) carbamate

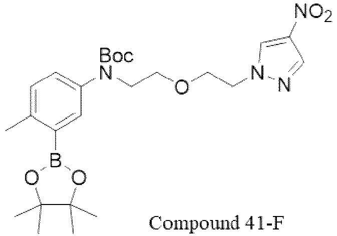

The synthesis of this step referred to the synthesis process of the sixth step of **compound 12 in Example 8.**

LC_MS: (ES⁺): m/z 417.05 [M-100] ⁺.

### Step 6: Compound 41-G tert-butyl (3-(2,5-dichloropyrimidin-4-yl)-4-methylphenyl)(2-(2-(4-nitro-1H-pyrazol-1-yl)ethoxy)ethyl)car bamate

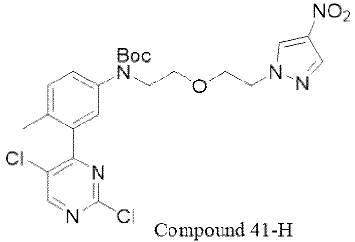

The synthesis of this step referred to the synthesis process of the seventh step of **compound 12 in Example 8.**

LC_MS: (ES⁺): m/z 480.75 [M-56] ⁺.

¹H NMR (400 MHz, CDCl₃): δ 8.69 (s, 1H), 8.02 (s, 2H), 7.29-7.26 (m, 2H), 7.21-7.08 (m, 2H), 4.28 (t, *J =* 5.0 Hz, 2H), 3.80-3.74 (m, 4H), 3.59 (t, *J=* 5.4 Hz, 2H), 2.21 (s, 3H), 1.40 (s, 9H).

### Step 7: Compound 41-I tert-butyl (E)-2⁵-chloro-1⁶-methyl-4¹H-7-oxa-3,10-diaza-2(4,2)-pyrimidina-4(4,1)-pyrazola-1(1,3)-benzenacyclodecaphan-10-carboxylate

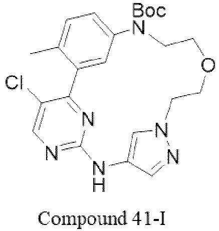

The synthesis of this step referred to the synthesis process of the eighth step of **compound 35 in Example 24.**

LC_MS: (ES⁺): m/z 471.25 [M+H] ⁺.

¹H NMR (400 MHz, CDCl₃): δ 8.41 (s, 1H), 8.36 (s, 1H), 7.49 (brs, 1H), 7.31 (s, 1H), 7.24 (s, 2H), 6.98 (s, 1H), 4.32-4.27 (m, 1H), 4.23-4.17 (m, 1H), 3.90-3.85 (m, 1H), 3.79-3.75 (m, 1H), 3.68-3.62 (m, 2H), 3.57-3.49 (m, 2H), 2.36 (s, 3H), 1.42 (s, 9H).

### Step 8: Compound 41 (E)-2⁵-chloro-1⁶-methyl-4¹H-7-oxa-3,10-diaza-2(4,2)-pyrimidina-4(4,1)-pyrazola-1(1,3)-benzena cyclodecaphane

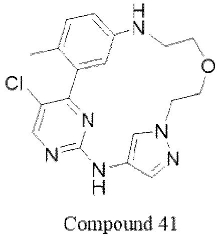

The synthesis of this step referred to the synthesis process of the sixth step of **compound 25 in Example 15.**

LC_MS: (ES⁺): m/z 371.30 [M+H] ⁺.

¹H NMR (400 MHz, CDCl₃): δ 8.51 (s, 1H), 8.34 (s, 1H), 7.34 (s, 1H), 7.07-7.02 (m, 3H), 6.72-6.69 (m, 1H), 4.38-4.23 (m, 2H), 3.94-3.89 (m, 1H), 3.75-3.70 (m, 1H), 3.67-3.56 (m, 2H), 3.46-3.40 (m, 2H), 2.28 (s, 3H).

### Example 32: Synthesis route of compound 42 and compound 43:

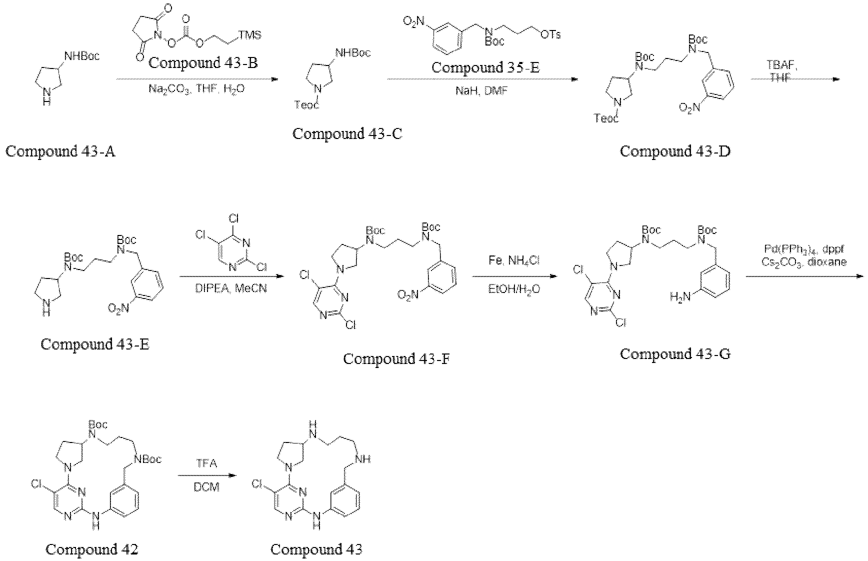

### Step 1: Compound 43-C 2-(trimethylsilyl)ethyl 3-((tert-butyloxycarbonyl)amino)pyrrolidin-1-carboxylate

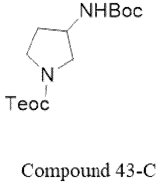

In a 100 ml single-necked flask, **compound 43-A** tert-butylpyrrolidin-3-ylcarbamate (1 g, 5.37 mmol) and **compound 43-B** 2,5-dioxopyrrolidin-1-yl (2-(trimethylsilyl)ethyl) carbonate (1.67 g, 6.44 mmol) were dissolved in a mixture of tetrahydrofuran (10 ml) and water (10 ml), added with potassium carbonate (1.14 g, 10.74 mmol) and stirred at room temperature for 2 hours. The reaction was monitored to be completed by TLC. The reaction solution was poured into water (50 ml) and extracted twice with ethyl acetate (30 ml). The organic phase was washed once with saturated brine, dried over anhydrous sodium sulfate, filtered and concentrated to obtain a light yellow oily liquid **compound 43-C** 2-(trimethylsilyl)ethyl 3-((tert-butoxycarbonyl)amino)pyrrolidine-1-carboxylate (1.85 g crude product), which was directly used in the next step without further purification.

¹H NMR (400 MHz, CDCl₃): δ 4.61 (s, 1H), 4.19-4.14 (m, 3H), 3.62-3.59 (m, 1H), 3.46-3.41 (m, 2H), 3.25-3.17 (m, 1H), 2.12-2.10 (m, 1H), 1.84-1.80 (m, 1H), 1.44 (s, 9H), 1.01-0.97 (m, 1H), 0.03 (s, 9H).

### Step 2: Compound 43-D 2-(trimethylsilyl)ethyl 3-((tert-butoxycarbonyl)(3-((tert-butoxycarbonyl)(3-nitrobenzyl)amino)propyl)amino)pyrrolidine -1-carboxylate

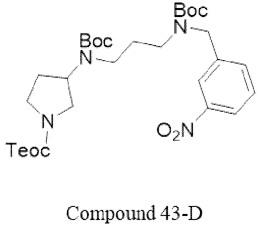

The synthesis of this step referred to the synthesis process of the fourth step of compound 35 in Example 25.

¹H NMR (400 MHz, CDCl₃): δ 8.12 (t, *J =* 8.4 Hz, 2H), 7.56-7.49 (m, 2H), 4.51-4.45 (m, 3H), 4.18 (t, *J =* 8.0 Hz, 2H), 3.63-3.49 (m, 2H), 3.33-3.10 (m, 6H), 2.00-1.93 (m, 2H), 1.74 (s, 2H), 1.48-1.44 (m, 18H), 0.04 (s, 9H).

### Step 3: Compound 43-E tert-butyl (3-((tert-butoxycarbonyl)(3-nitrobenzyl)amino)propyl)(pyrrolidin-3-yl)carbamate

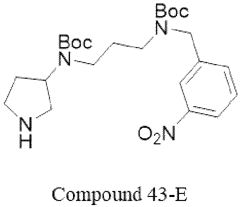

In a 10 ml single-necked flask, **compound 43-D** 2-(trimethylsilyl)ethyl 3-((tert-butoxycarbonyl)(3-((tert-butoxycarbonyl)(3-nitrobenzyl)amino)propyl)amino)pyrrolidin-1-carboxylate (50 mg, 0.08 mmol) was dissolved in 1 ml of tetrahydrofuran, added with a solution of TBAF in THF (0.1 ml, 0.08 mmol, 1 M) at room temperature, and stirred at 30°C overnight. The reaction was monitored by TLC to be completed. The reaction solution was poured into water (10 ml) and extracted twice with ethyl acetate (10 ml). The organic phases were combined, washed once with saturated brine, dried over anhydrous sodium sulfate, filtered and concentrated. The crude product was purified by thin layer chromatography (DCM: MeOH = 10: 1) to obtain a colorless oily **compound 43-E** tert-butyl (3-((tert-butoxycarbonyl)(3-nitrobenzyl)amino)propyl)(pyrrolidin-3-yl)carbamate (21 mg).

LC_MS: (ES⁺): m/z 479.60 [M+H] ⁺.

### Step 4: Compound 43-F tert-butyl (3-((tert-butoxycarbonyl)(3-nitrobenzyl)amino)propyl)(1-(2,5-dichloropyrimidin-4-yl)pyrrolidin-3-yl)carbamate

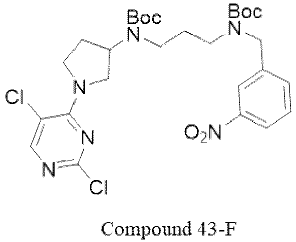

**Compound 43-E** tert-butyl (3-((tert-butoxycarbonyl)(3-nitrobenzyl)amino)propyl)(pyrrolidin-3-yl)carbamate (66 mg, 0.138 mmol), acetonitrile (2 ml) and N,N-diisopropylethylamine (54 mg, 0.414 mmol) were sequentially added to a 10 ml single-neck flask at room temperature. The reaction system was replaced with nitrogen three times, then sealed, and added with 2,4,5-trichloropyrimidine (38 mg, 0.207 mmol) using a syringe. The resulting reaction system was sealed at 60°C for 2 hours. The reaction was monitored by TLC to be completed. The reaction solution was poured into water (10 ml) and extracted with ethyl acetate (10 ml) three times. The organic phases were combined, washed once with saturated brine (10 ml), dried over anhydrous sodium sulfate, filtered and concentrated. The crude product was purified by thin layer chromatography (DCM: MeOH = 40: 1) to obtain a colorless viscous oily **compound 43-F** tert-butyl (3-((tert-butoxycarbonyl)(3-nitrobenzyl)amino)propyl)(1-(2,5-dichloropyrimidin-4-yl)pyrrolidin-3-yl)carbamate (70 mg).

LC_MS: (ES⁺): m/z 625.20 [M+H] ⁺.

### Step 5: Compound 43-G tert-butyl (3-((3-aminobenzyl)(tert-butoxycarbonyl)amino)propyl)(1-(2,5-dichloropyrimidin-4-yl)pyrrolidin -3 -yl)carbamate

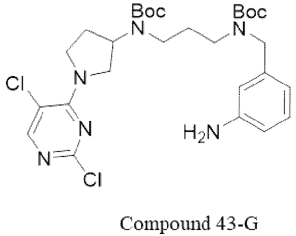

The synthesis of this step referred to the synthesis process of the fourth step of **compound 2 in Example 1.**

LC_MS: (ES⁺): m/z 595.20 [M+H] ⁺.

¹H NMR (400 MHz, CDCl₃): δ 8.00 (s, 1H), 7.08 (t, *J =* 3.6 Hz, 2H), 6.62-6.53 (m, 3H), 4.42-4.34 (m, 3H), 4.00 (s, 2H), 3.70-3.49 (m, 4H), 3.18-3.02 (m, 4H), 2.03-2.01 (m, 2H), 1.71 (s, 2H), 1.48-1.45 (m, 18H).

### Step 6: Compound 42 di-tert-butyl 2⁵-chloro-3,6,10-triaza-2(4,2)-pyrimidina-1(1,3)-pyrrolidina-4(1,3)-benzenacyclodecaphan-6,10-dicarboxylate

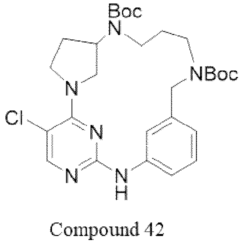

The synthesis of this step referred to the synthesis process of the eighth step of **compound 12 in Example 8.**

LC_MS: (ES⁺): m/z 560.55 [M+H] ⁺.

¹H NMR (400 MHz, CDCl₃): δ 9.32 (s, 1H), 8.04-7.99 (m, 2H), 7.22 (t, *J* = 8.0 Hz, 1H), 7.03-6.94 (m, 2H), 4.74-4.71 (m, 1H), 4.44 (s, 1H), 3.93-3.69 (m, 5H), 3.42 (m, 2H), 2.96 (s, 1H), 2.67 (s, 1H), 2.06-1.96 (m, 2H), 1.75 (s, 2H), 1.45-1.23 (m, 18H).

### Step 7: Compound 43 2⁵-chloro-3,6,10-triaza-2(4,2)-pyrimidina-1(1,3)-pyrrolidina-4(1,3)-benzenacyclodecaphane

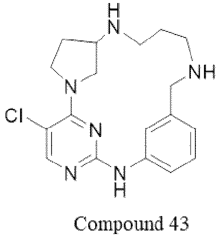

The synthesis of this step referred to the synthesis process of the sixth step of **compound 25 in Example 15.**

LC_MS: (ES⁺): m/z 360.15 [M+H] ⁺.

¹H NMR (400 MHz, CDCl₃): δ 9.25 (s, 1H), 8.45 (s, 1H), 7.95 (s, 1H), 7.13 (t, *J =* 7.6 Hz, 1H), 6.96-6.94 (m, 1H), 6.79 (d, *J* = 7.2 Hz, 1H), 3.99-3.88 (m, 1H), 3.87-3.80 (m, 2H), 3.76-3.59 (m, 3H), 3.35-3.28 (m, 3H), 2.89-2.80 (m, 2H), 2.73-2.64 (m, 2H), 2.11-2.06 (m, 1H), 1.85-1.75 (m, 1H), 1.70-1.64 (m, 1H), 1.57-1.48 (m, 1H).

### Example 33: Synthesis route of compound 44:

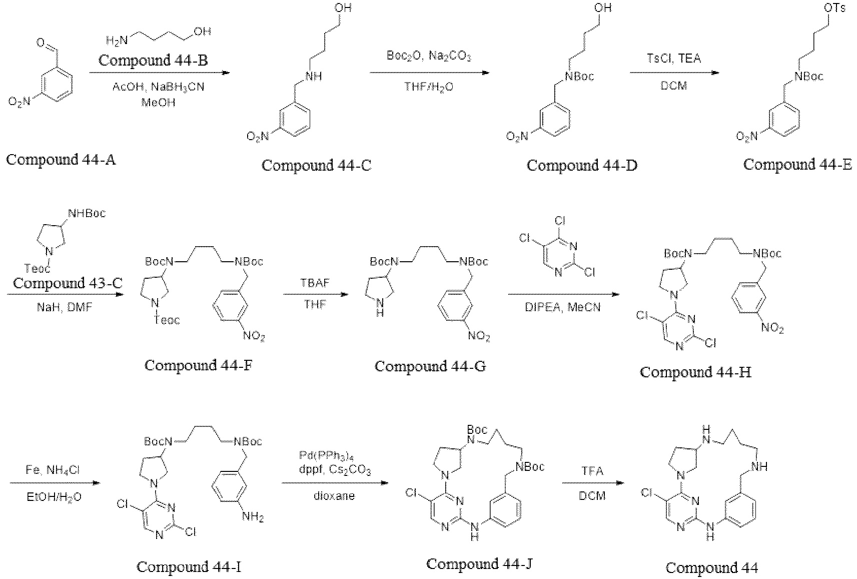

### Step 1: Compound 44-C 4-((3-nitrobenzyl)amino)butan-1-ol

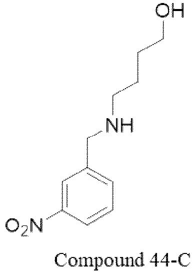

The synthesis of this step referred to the synthesis process of the first step of **compound 35 in Example 25.**

LC_MS: (ES⁺): m/z 225.05 [M+H] ⁺.

### Step 2: Compound 44-D tert-butyl (4-hydroxybutyl)(3-nitrobenzyl)carbamate

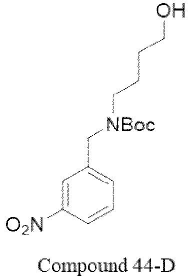

The synthesis of this step referred to the synthesis process of the second step of **compound 35 in Example 25.**

LC_MS: (ES⁺): m/z 324.75 [M+H] ⁺.

### Step 3: Compound 44-E 4-((tert-butoxycarbonyl)(3-nitrobenzyl)amino)butyl 4-methylbenzenesulfonate

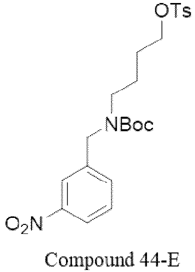

The synthesis of this step referred to the synthesis process of the third step of **compound 35 in Example 25.**

LC_MS: (ES⁺): m/z 378.90 [M+H-100] ⁺.

### Step 4: Compound 44-F 2-(trimethylsilyl)ethyl 3-((tert-butoxycarbonyl)(4-((tert-butoxycarbonyl)(3-nitrobenzyl)amino)butyl)amino)pyrrolidin-1-carboxylate

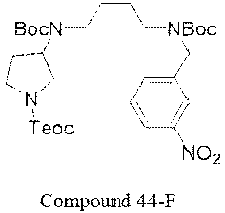

The synthesis of this step referred to the synthesis process of the fourth step of **compound 35 in Example 25.**

LC_MS: (ES⁺): m/z 324.75 [M+H] ⁺.

¹H NMR (400 MHz, CDCl₃): δ 8.13-8.09 (m, 2H), 7.57-7.48 (m, 2H), 4.55-4.44 (m, 2H), 4.24-4.09 (m, 4H), 3.64-3.54 (m, 2H), 3.48-3.41 (m, 1H), 3.27-3.08 (m, 4H), 2.06-1.90 (m, 2H), 1.49, 1.44 (two singles, 18H), 1.30-1.22 (m, 4H), 0.99 (t, *J =* 8.4 Hz, 2H), 0.03 (s, 9H).

### Step 5: Compound 44-G tert-butyl (4-((tert-butyloxycarbonyl)(3-nitrobenzyl)amino)butyl)(pyrrolidin-3-yl)carbamate

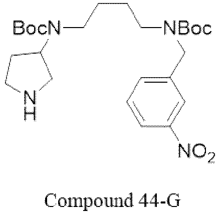

The synthesis of this step referred to the synthesis process of the third step of **compound 43 in Example 32.**

LC_MS: (ES⁺): m/z 493.30 [M+H] ⁺.

### Step 6: Compound 44-H tert-butyl (4-((tert-butyloxycarbonyl)(3-nitrobenzyl)amino)butyl)(1-(2,5-dichloropyrimidin-4-yl)pyrrolidin-3-yl)carbamate

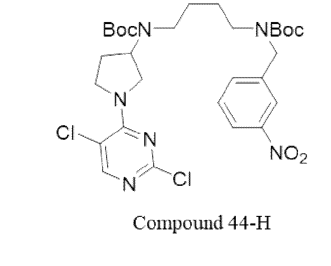

The synthesis of this step referred to the synthesis process of the fourth step of **compound 43 in Example 32.**

LC_MS: (ES⁺): m/z 639.20 [M+H] ⁺.

### Step 7: Compound 44-1 tert-butyl (4-((3-aminobenzyl)(tert-butyloxycarbonyl)amino)butyl)(1-(2,5-dichloropyrimidin-4-yl)pyrrolidi n-3-yl)carbamate

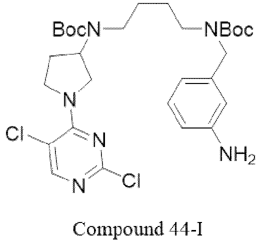

The synthesis of this step referred to the synthesis process of the fourth step of **compound 2 in Example 1.**

LC_MS: (ES⁺): m/z 609.45 [M+H] ⁺.

### Step 8: Compound 44-J di-tert-butyl 2⁵-chloro-3,6,11-triaza-2(4,2)-pyrimidina-1(1,3)-pyrrolidina-4(1,3)-benzocycloalkaphan-6,11-dic arboxylate

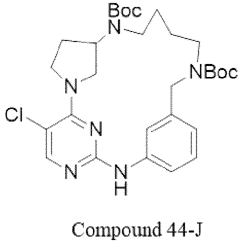

The synthesis of this step referred to the synthesis process of the eighth step of **compound 12 in Example 8.**

LC_MS: (ES⁺): m/z 573.55 [M+H] ⁺.

¹H NMR (400 MHz, DMSO-d6): δ 9.39 (s, 1H), 8.17 (s, 1H), 7.96 (s, 1H), 7.24-7.21 (m, 1H), 7.06-7.04 (m, 1H), 6.91-6.85 (m, 1H), 4.39 (d, *J* = 14.8 Hz, 1H), 4.17-4.05 (m, 3H), 3.90-3.80 (m, 2H), 3.64 (t, *J =* 11.2 Hz, 1H), 3.47-3.39 (m, 1H), 3.27-3.19 (m, 1H), 3.15-3.03 (m, 1H), 2.88-2.77 (m, 1H), 2.11-1.90 (m, 2H), 1.45-1.37 (m, 22H).

### Step 9: Compound 44 2⁵-chloro-3,6,11-triaza-2(4,2)-pyrimidina-1(1,3)-pyrrolidina-4(1,3)-benzenacycloalkaphane

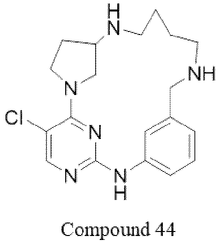

The synthesis of this step referred to the synthesis process of the sixth step of **compound 25 in Example 15.**

LC_MS: (ES⁺): m/z 373.30 [M+H] ⁺.

¹H NMR (400 MHz, CDCl₃): δ 9.25 (s, 1H), 8.34 (s, 1H), 7.93 (s, 1H), 7.15 (t, *J =* 7.8 Hz, 1H), 6.99 (d, *J* = 8.0 Hz, 1H), 6.85 (d, *J* = 7.6 Hz, 1H), 4.02-3.98 (m, 3H) , 3.69-3.62 (m, 2H), 3.51-3.40 (m, 2H), 3.27-3.19 (m, 2H), 2.78-2.60 (m, 4H), 2.13-2.08 (m, 1H), 1.57-1.51 (m, 1H), 1.45-1.34 (m, 2H).

### Example 34: Synthesis route of compound 45:

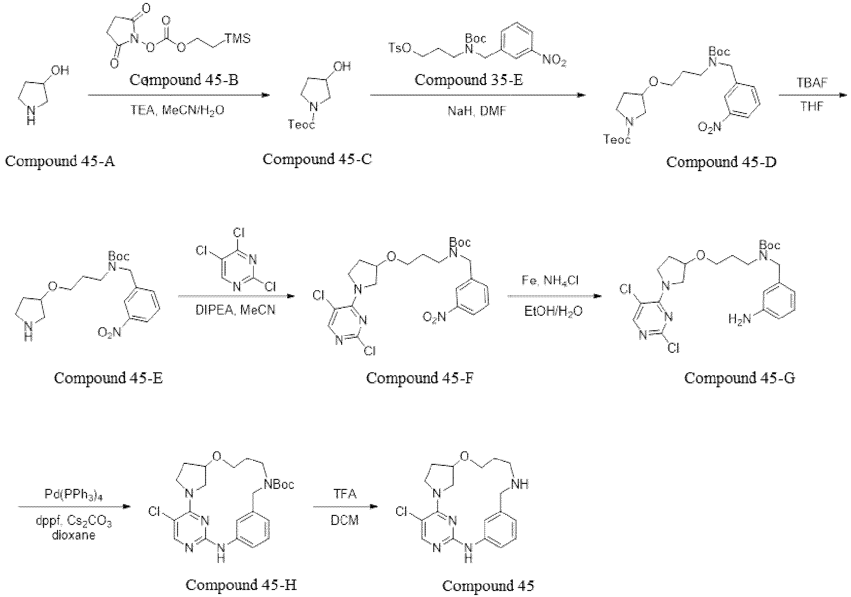

### Step 1: Compound 45-C 2-(trimethylsilyl)ethyl 3-hydroxypyrrolidin-1-carboxylate

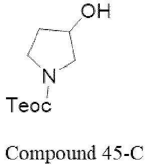

In a 100 ml three-necked flask with a thermometer, **compound 45-A** 3-hydroxypyrrolidine (500 mg, 5.74 mmol) and triethylamine (581 mg, 5.74 mmol) were dissolved in a mixture of acetonitrile (5 ml) and water (5 ml). The temperature was kept at 0°C, and substrate **compound 45-B** N-[2-(trimethylsilyl)ethoxycarbonyloxy]succinimide (1.5 g, 5.74 mmol) was added dropwise. After the dropwise addition was completed, the reaction solution was warmed to room temperature and reacted for 2 hours. The reaction was monitored to be completed by TLC. The reaction mixture was poured into 10 ml of water and extracted twice with ethyl acetate (10 ml). The organic phase was washed once with saturated brine, dried over anhydrous sodium sulfate, filtered and concentrated to obtain a colorless oily liquid crude **compound 45-C** 2-(trimethylsilyl)ethyl 3-hydroxypyrrolidin-1-carboxylate (1.2 g), which was directly used in the next reaction without further purification.

¹H NMR (400 MHz, CDCl₃): δ 4.46 (brs, 1H), 4.19-4.15 (m, 2H), 3.55-3.34 (m, 4H), 2.03-1.88 (m, 3H), 0.99 (t, *J =* 8.4 Hz, 2H), 0.03 (s, 9H).

### Step 2: Compound 45-D 2-(trimethylsilyl)ethyl 3-(3-((tert-butoxycarbonyl)(3-nitrobenzyl)amino)propoxy)pyrrolidin-1-carboxylate

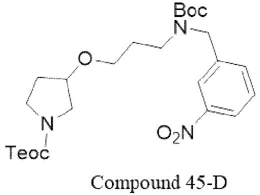

In a 50 ml three-necked flask with a thermometer, **compound 45-C** 2-(trimethylsilyl)ethyl 3-hydroxypyrrolidin-1-carboxylate (172 mg, 0.74 mmol) was dissolved in DMF (2 ml), added with sodium hydride (30 mg, 0.74 mmol) in batches at 0°C. After replaced with nitrogen, the mixture was heated to room temperature for 1 hour of reaction. Then the system was cooled to 0°C, and a solution of **compound 35-E** 3-((tert-butoxycarbonyl)(3-nitrobenzyl)amino)propyl 4-methylbenzenesulfonate (230 mg, 0.5 mmol) in DMF (1 ml) was slowly added dropwise, and the mixture was heated to room temperature for overnight reaction. The reaction was monitored by TLC to be completed. The reaction solution was poured into 30 ml of water and extracted 3 times with ethyl acetate (10 ml). The organic phases were combined, backwashed twice with water (10 ml), washed once with saturated brine, dried over anhydrous sodium sulfate, and concentrated to obtain a crude product. The crude product was separated and purified by silica gel column chromatography (PE:EA=10:1~3:1) to obtain a light yellow oily **compound 45-D** 2-(trimethylsilyl)ethyl 3-(3-((tert-butoxycarbonyl)(3-nitrobenzyl)amino)propoxy)pyrrolidin-1-carboxylate (113 mg).

LC_MS: (ES⁺): m/z 524.20 [M+H] ⁺.

¹H NMR (400 MHz, CDCl₃): δ 8.13-8.04 (m, 2H), 7.55-7.48 (m, 2H), 4.58-4.39 (m, 2H), 4.18-4.14 (m, 2H), 3.98 (brs, 1H), 3.49-3.26 (m, 8H), 2.01-1.88 (m, 2H), 1.84-1.73 (m, 2H), 1.49-1.44 (m, 9H), 0.99 (t, *J =* 8.2 Hz, 2H), 0.03 (s, 9H).

### Step 3: Compound 45-E tert-butyl (3-nitrobenzyl)(3-(pyrrolidin-3-yloxy)propyl)carbamate

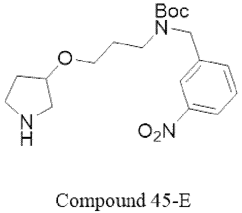

The synthesis of this step referred to the synthesis process of the third step of **compound 43 in Example 32.**

LC_MS: (ES⁺): m/z 380.75 [M+H] ⁺.

### Step 4: Compound 45-F tert-butyl (3-((1-(2,5-dichloropyrimidin-4-yl)pyrrolidin-3-yl)oxy)propyl)(3-nitrobenzyl)carbamate

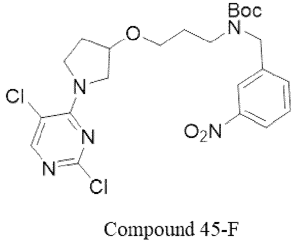

The synthesis of this step referred to the synthesis process of the fourth step of **compound 43 in Example 32.**

LC_MS: (ES⁺): m/z 526.30 [M+H] ⁺.

¹H NMR (400 MHz, CDCl₃): δ 8.13-8.08 (m,2H), 7.98 (s, 1H), 7.54-7.48 (m, 2H), 4.57-4.39 (m, 2H), 4.10-4.05 (m, 1H), 4.03-3.74 (m, 4H), 3.49-3.24 (m, 4H), 2.14-2.07 (m, 1H), 1.98-1.94 (m, 1H), 1.87-1.75 (m, 2H), 1.48-1.43 (m, 9H).

### Step 5: Compound 45-G tert-butyl (3-aminobenzyl)(3-((1-(2,5-dichloropyrimidin-4-yl)pyrrolidin-3-yl)oxy)propyl)carbamate

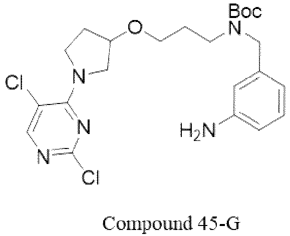

The synthesis of this step referred to the synthesis process of the fourth step of **compound 2 in Example 1.**

LC_MS: (ES⁺): m/z 496.40 [M+H] ⁺.

¹H NMR (400 MHz, DMSO-d6): δ 8.16 (s, 1H), 6.93 (t, J = 7.6 Hz, 1H), 6.44-6.39 (m, 2H), 6.31 (d, J = 7.6 Hz, 1H), 5.10 (brs, 2H), 4.20 (s, 2H), 4.08 (brs, 1H), 3.96-3.56 (m, 4H), 3.41-3.36 (m, 2H), 3.21-3.06 (m, 2H), 2.06-1.85 (m, 2H), 1.69-1.63 (m, 2H), 1.39 (brs, 9H).

### Step 6: Compound 45-H tert-butyl 2⁵-chloro-10-oxa-3,6-diaza-2(4,2)-pyrimidina-1(1,3)-pyrrolidina-4(1,3)-benzenacyclodecaphan-6 -carboxylate

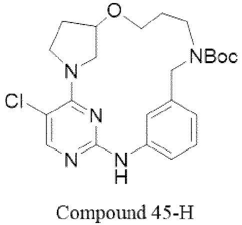

The synthesis of this step referred to the synthesis process of the eighth step of **compound 12 in Example 8.**

LC_MS: (ES⁺): m/z 460.40 [M+H] ⁺.

### Step 7: Compound 45 2⁵-chloro-10-oxa-3,6-diaza-2(4,2)-pyrimidina-1(1,3)-pyrrolidina-4(1,3)-benzenacyclodecaphane

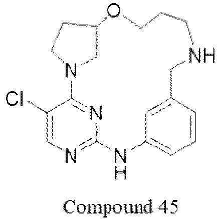

The synthesis of this step referred to the synthesis process of the sixth step of **compound 25 in Example 15.**

LC_MS: (ES⁺): m/z 360.30&360.50 [M+H] ⁺.

¹H NMR (400 MHz, DMSO-d6): δ 9.26 (s, 1H), 8.42 (s, 1H), 7.95 (s, 1H), 7.13 (t, *J =* 7.8 Hz, 1H), 6.95-6.92 (m, 1H), 6.79 (d, *J =* 7.2 Hz, 1H), 4.14-4.09 (m, 1H), 4.02-3.96 (m, 1H), 3.89-3.66 (m, 5H), 3.60-3.53 (m, 2H), 3.40-3.38 (m, 1H), 2.85-2.79 (m, 1H), 2.71-2.66 (m, 1H), 2.17-2.11 (m, 1H), 1.99-1.92 (m, 1H), 1.73-1.66 (m, 2H).

### Example 35: Synthesis route of compound 46:

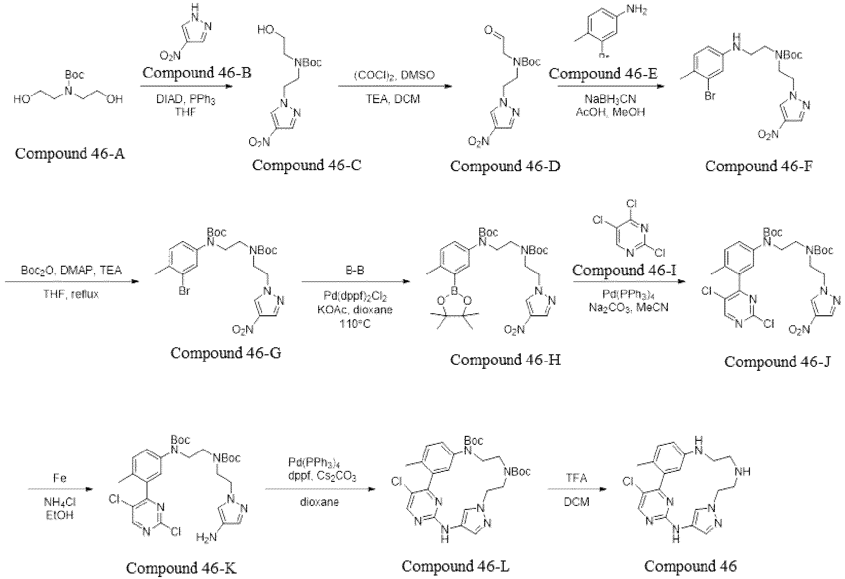

### Step 1: Compound 46-C tert-butyl (2-hydroxyethyl)(2-(4-nitro- 1H-pyrazol-1-yl)ethyl)carbamate

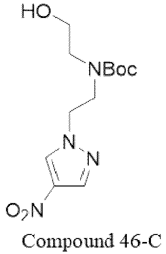

In a 500 ml three-necked flask with a thermometer, **compound 46-A** N-tert-butyloxycarbonyldiethanolamine (25 g, 0.12 mol), **compound 46-B** 4-nitropyrazole (6.9 g, 0.06 mol) and triphenylphosphine (32 g, 0.12 mol) were added to dry tetrahydrofuran (200 ml). The reaction solution was replaced with nitrogen three times, kept at a temperature between 0°C and 5°C, slowly dropwise added with diisopropyl azodicarboxylate (25 g, 0.12 mol), and then stirred at room temperature overnight. The reaction was monitored by TLC and LCMS to be completed. The reaction solution was spin-dried, and the crude product was slurried with methyl tert-butyl ether for 1 hour. The precipitated solid (triphenylphosphine oxide) was filtered, and the filtrate was collected and spin-dried. The crude filtrate was separated and purified by silica gel column chromatography (DCM: MeOH=100:1 to 50:1) to obtain a yellow oil product **compound 46-C** tert-butyl (2-hydroxyethyl)(2-(4-nitro-1H-pyrazol-1-yl)ethyl)carbamate (15.6 g).

LC_MS: (ES⁺): m/z 301.15 [M+H] ⁺.

¹H NMR (400 MHz, CDCl₃): δ 8.17 (s, 1H), 8.08-8.06 (m, 1H), 4.38 (s, 2H), 3.71 (t, *J* = 5.6 Hz, 4H), 3.31-3.23 (m, 2H), 1.37 (d, *J =* 13.6 Hz, 9H).

### Step 2: Compound 46-D tert-butyl (2-(4-nitro-1H-pyrazol-1-yl)ethyl)(2-oxoethyl)carbamate

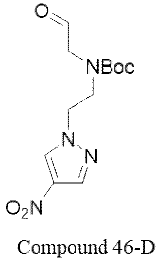

In a 250 ml three-necked flask with a low-temperature thermometer, oxalyl chloride (3.7 g, 0.03 mol) was added to dichloromethane (50 ml). The reaction solution was protected by nitrogen and kept at a temperature between -78°C and -60°C. DMSO (4.6 g, 0.06 mol) was dissolved in dichloromethane (10 ml), and slowly added dropwise to the above reaction solution at a temperature controlled between -78°C and -60°C, and the obtained reaction solution was kept at this temperature for 30 minutes. Then **compound 46-C** tert-butyl (2-hydroxyethyl)(2-(4-nitro-1H-pyrazol-1-yl)ethyl)carbamate (5.87 g, 0.02 mol) was dissolved in DCM (10 ml), and slowly added dropwise to the above reaction solution at a temperature controlled between -78°C and -60°C, and the obtained reaction solution was kept at this temperature for 30 minutes. Then triethylamine (11.8 g, 0.12 mol) was dissolved in DCM (10 ml), and slowly added dropwise to the above reaction solution at a temperature controlled between -78°C and -60°C, and reaction was performed for 30 minutes. The low temperature environment was removed, and the reaction solution was slowly heated to room temperature. The reaction was monitored by TLC to be completed. The reaction solution was filtered and the filter cake was washed with ethyl acetate (50 ml). The filtrate was collected, spin-dried, and extracted with water (50 ml) and ethyl acetate (100 ml). The organic phase was washed three times with saturated aqueous ammonium chloride solution (50 ml), then dried over anhydrous sodium sulfate, filtered and spin-dried to obtain a yellow oily crude product, **compound 46-D** tert-butyl (2-(4-nitro-1H-pyrazol-1-yl)ethyl)(2-oxoethyl)carbamate (5.8 g), which was directly used for the next step without purification.

LC_MS: (ES⁺): m/z 298.95 [M+H] ⁺.

### Step 3: Compound 46-F tert-butyl (2-((3-bromo-4-methylphenyl)amino)ethyl)(2-(4-nitro-1H-pyrazol-1-yl)ethyl)carbamate

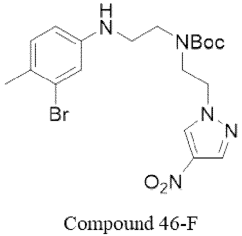

In a 250 ml single-neck flask, **compound 46**-D tert-butyl (2-(4-nitro-1H-pyrazol-1-yl)ethyl)(2-oxoethyl)carbamate (5.8 g, 0.02 mol) and **compound 46-E** 3-bromo-4-methylaniline (3.6 g, 0.02 mol) were added to anhydrous methanol (100 ml). The reaction solution was adjusted to pH 4-5 with acetic acid, then reacted at room temperature for 30 minutes, added with sodium cyanoborohydride (2.5 g, 0.04 mol) in batches, and reacted at room temperature overnight. The reaction was monitored by TLC to be completed. The reaction solution was directly spin-dried and separated and purified by silica gel column chromatography (PE:EA=4:1) to obtain yellow oily **compound 46-F** tert-butyl (2-((3-bromo-4-methylphenyl)amino)ethyl)(2-(4-nitro-1H-pyrazol-1-yl)ethyl)carbamate (3.46 g).

LC_MS: (ES⁺): m/z 468.10 [M+H] ⁺.

¹H NMR (400 MHz, CDCl₃): δ 8.07-8.06 (m, 2H), 6.99 (d, *J =* 8.4 Hz, 1H), 6.74 (d, *J* = 2.0 Hz, 1H), 6.43-6.41 (m, 1H), 4.35-4.25 (m, 2H), 3.66 (t, *J =* 6.0 Hz, 2H), 3.31-3.12 (m, 4H), 2.27 (s, 3H), 1.45 (s, 9H).

### Step 4: Compound 46-G tert-butyl (3-bromo-4-methylphenyl)(2-((tert-butoxycarbonyl)(2-(4-nitro-1H-pyrazol-1-yl)ethyl)amino)ethy l)carbamate

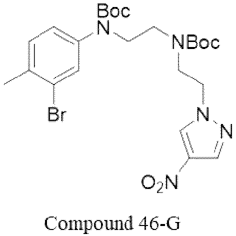

In a 250 ml single-necked flask with a reflux tube, **compound 46-F** tert-butyl (2-((3-bromo-4-methylphenyl)amino)ethyl)(2-(4-nitro-1H-pyrazol-1-yl)ethyl)carbamate (1.3 g, 2.78 mmol), triethylamine (2.8 g, 27.8 mmol) and DMAP (3.4 g, 27.8 mmol) were added to tetrahydrofuran (80 ml), and then added with Boc₂O (6.07 g, 27.8 mmol) under ice bath. After the addition was completed, the reaction solution was refluxed overnight (73°C), and the reaction was monitored to be completed by TLC. The reaction solution was cooled to room temperature, spin-dried, added with saturated citric acid solution (50 ml) and ethyl acetate (50 ml), and extracted. The organic phase was washed twice with citric acid aqueous solution (40 ml), washed with saturated sodium chloride aqueous solution (50 ml), and then dried over anhydrous sodium sulfate, filtered and spin-dried to obtain a crude product. The crude product was separated and purified by silica gel column chromatography (petroleum ether: ethyl acetate = 5:1 to 3:1) to obtain a yellow oily **compound 46-G** tert-butyl (3-bromo-4-methylphenyl)(2-((tert-butoxycarbonyl)(2-(4-nitro-1H-pyrazol-1-yl)ethyl)amino)ethy l)carbamate (1.2 g).

LC_MS: (ES⁺): m/z 570.10 [M+H] ⁺.

¹H NMR (400 MHz, CDCl₃): δ 8.08 (s, 2H), 7.34 (s, 1H), 7.16 (d, *J* = 8.0 Hz, 1H), 6.99-6.98 (m, 1H), 4.33-4.27 (m,2H), 3.66-3.59 (m, 4H), 3.26-3.14 (m, 2H), 2.36(s, 3H), 1.43 (s, 9H), 1.36 (s, 9H).

### Step 5: Compound 46-H tert-butyl(2-((tert-butoxycarbonyl)(2-(4-nitro-1H-pyrazol-1-yl)ethyl)amino)ethyl) (4-methyl-3-(4,4,5,5-1,3,2-dioxaborolan-2-yl)phenyl)carbamate tetramethyl ester

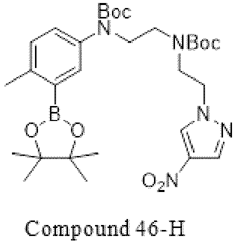

The synthesis of this step referred to the synthesis process of the sixth step of **compound 12 in Example 8.**

LC_MS: (ES⁺): m/z 616.15 [M+H] ⁺.

### Step 6: Compound 46-J tert-butyl (2-((tert-butyloxycarbonyl)(2-(4-nitro-1H-pyrazol-1-yl)ethyl)amino)ethyl)(3-(2,5-dichloropyrimi din-4-yl)-4-methylphenyl)carbamate

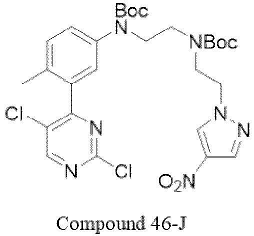

The synthesis of this step referred to the synthesis process of the seventh step of **compound 12 in Example 8.**

LC_MS: (ES⁺): m/z 636.20 [M+H] ⁺.

### Step 7: Compound 46-K tert-butyl (2-((2-(4-amino-1H-pyrazol-1-yl)ethyl)(tert-butoxycarbonyl)amino)ethyl)(3-(2,5-dichloropyrimid in-4-yl)-4-methylphenyl)carbamate

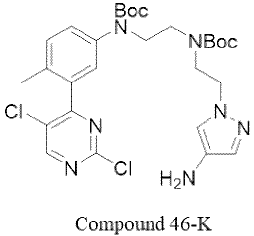

The synthesis of this step referred to the synthesis process of the fourth step of **compound 2 in Example 1.**

LC_MS: (ES⁺): m/z 606.20 [M+H] ⁺.

### Step 8: Compound 46-L di-tert-butyl (E)-2⁵-chloro-1⁶-methyll-4¹H-3,7,10-triaza-2(4,2)-pyrimidina-4(4,1)-pyrazola-1(1,3)-benzenacycl odecaphan-7,10-dicarboxylate

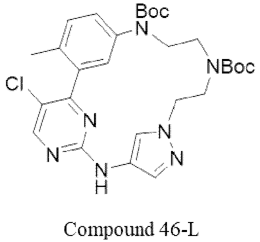

The synthesis of this step referred to the synthesis process of the eighth step of **compound 12 in Example 8.**

LC_MS: (ES⁺): m/z 570.60 [M+H] ⁺.

### Step 9: Compound 46 (E)-2⁵-chloro-1⁶-methyl-4¹H-3,7,10-triaza-2(4,2)-pyrimidina-4(4,1)-pyrazola-1(1,3)-benzenacycl odecaphane

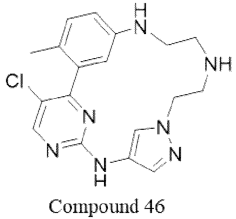

The synthesis of this step referred to the synthesis process of the sixth step of **compound 25 in Example 15.**

LC_MS: (ES⁺): m/z 370.15 [M+H] ⁺.

¹H NMR (400 MHz, DMSO-d6): δ 9.81 (s, 1H), 8.47 (s, 1H), 8.37 (s, 1H), 7.29 (s, 1H), 6.97 (d, *J* = 8.4 Hz, 1H), 6.84 (d, *J* = 2.4 Hz, 1H), 6.68-6.65 (m, 1H), 5.85-5.81 (m, 1H), 4.29-4.22 (m, 1H), 4.10-4.09 (m, 1H), 3.98-3.94 (m, 1H), 3.22-3.19 (m, 2H), 3.04-2.98 (m, 1H), 2.94-2.87 (m, 1H), 2.76-2.73 (m, 1H), 2.37-2.31 (m, 1H), 2.16 (s, 3H)..

### Example 36: Synthesis route of compound 47:

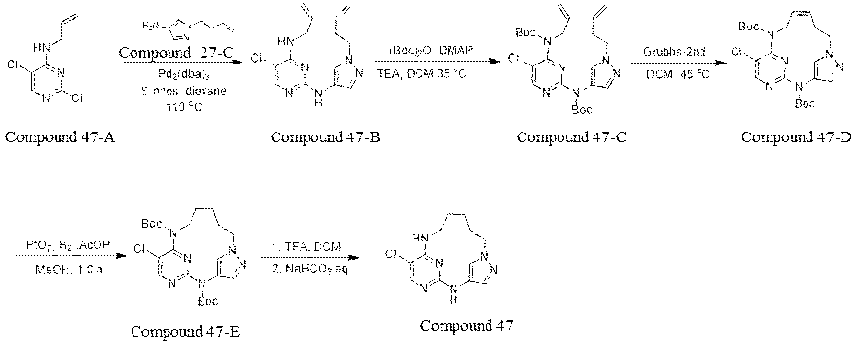

### Step 1: Compound 47-B tert-butyl N⁴-allyl-N²-(1-(but-3-en-1-yl)-1H-pyrazol-4-yl)-5-chloropyrimidin-2,4-diamine(3-bromo-4-meth oxyphenyl)carbamate

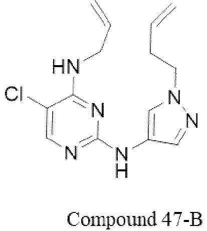

The synthesis of this step referred to the synthesis process of the third step of **compound 27 in Example 17.**

LC-MS: (ES⁺): m/z 305.1 [M+H] ⁺.

### Step 2: Compound 47-C tert-butyl allyl (2-((1-(but-3-en-1-yl)-1H-pyrazol-4-yl)(tert-butoxycarbonyl)amino)-5-chloropyrimidin-4-yl)carb amate

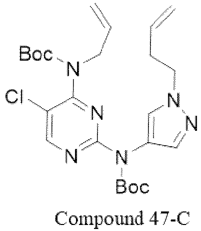

The synthesis of this step referred to the synthesis process of the third step of **compound 25 in Example 15.**

LC-MS: (ES⁺): m/z 505.1 [M+H] ⁺.

### Step 3: Compound 47-D di-tert-butyl (3⁴E,6Z)-1⁵-chloro-3¹H-2,9-diaza-1(2,4)-pyrimidina-3(4,1)-pyrazolacyclononaphan-6-en-2,9-dica rboxylate

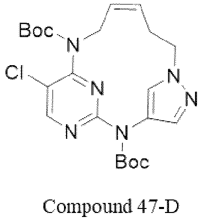

The synthesis of this step referred to the synthesis process of the fourth step of **compound 25 in Example 15.**

LC-MS: (ES⁺): m/z 477.1 [M+H] ⁺.

### Step 4: Compound 47-E di-tert-butyl (3⁴E,6Z)-1⁵-chloro-3¹H-2,9-diaza-1(2,4)-pyrimidina-3(4,1)-pyrazolecyclononane-2,9-dicarboxylate

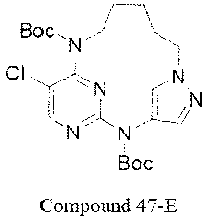

The synthesis of this step referred to the synthesis process of the fifth step of **compound 25 in Example 15.**

LC-MS: (ES⁺): m/z 479.1 [M+H] ⁺.

### Step 5: Compound 47 (E)-1⁵-chloro-3¹H-2,9-diaza-1(2,4)-pyrimidina-3(4,1)-pyrazolacyclononaphane

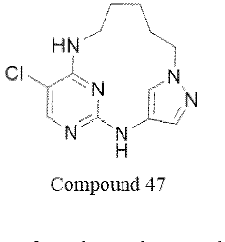

The synthesis of this step referred to the synthesis process of the sixth step of **compound 25 in Example 15.**

LC-MS: (ES⁺): m/z 279.0 [M+H] ⁺.

¹H NMR (400 MHz, DMSO-*d*₆) *δ*: 7.83 (s, 1H), 7.72 (s, 1H), 7.29 (s, 1H), 6.34 (s, 1H), 5.37 (s, 1H), 4.24 (t, *J =* 4.0 Hz, 2H), 3.19 - 3.12 (m, 2H), 1.84 - 1.80 (m, 2H), 1.53 - 1.39 (m, 4H).

### Example 37: Synthesis route of compound 48:

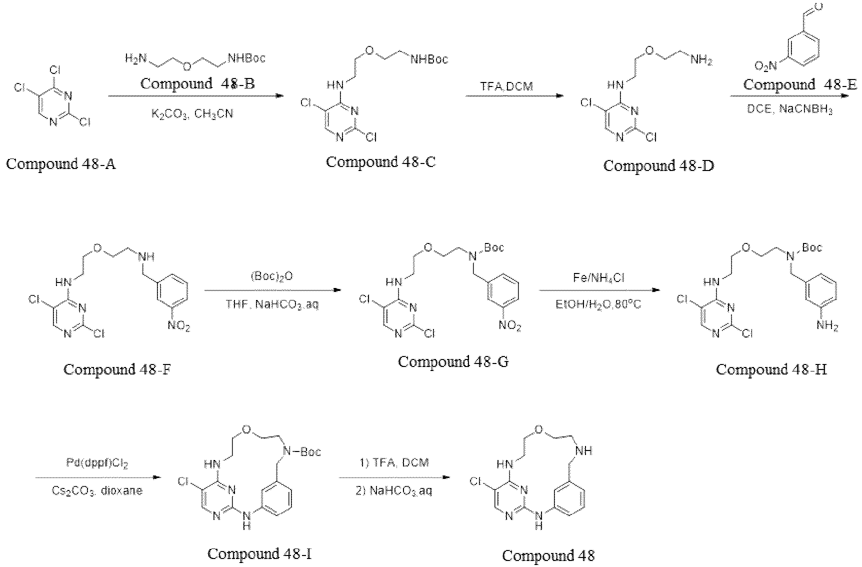

### Step 1: Compound 48-C tert-butyl 2-(2-(2,5-dichloropyrimidin-4-yl)aminoethoxy)ethylcarbamate

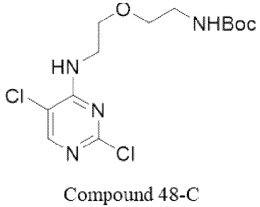

The synthesis of this step referred to the synthesis process of the fifth step of **compound 2 in Example 1.**

LC-MS: (ES⁺): m/z 351.1 [M+H] ⁺.

### Step 2: Compound 48-D 2-(2-aminoethoxy)ethyl-2,5-dichloropyrimidin-4-amine

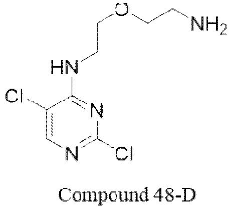

Trifluoroacetic acid (4 mL) was added to a solution of **compound 48-C** tert-butyl 2-(2-(2,5-dichloropyrimidin-4-yl)aminoethoxy)ethylcarbamate (340 mg, 0.97 mmol) in dichloromethane (4 mL) under stirring. The reaction solution was stirred at 25°C for 1.0 hour. When LCMS monitored the target mass spectrum and the reaction raw materials disappeared, the reaction solution was concentrated by rotary evaporation under reduced pressure and dried in vacuum to obtain a yellow solid product, **compound 48-D** 2-(2-aminoethoxy)ethyl-2,5-dichloropyrimidin-4-amine (350 mg, trifluoroacetate).

LC_MS: (ES⁺): m/z 251.1 [M+H] ⁺

### Step 3: Compound 48-F 2,5-dichloro-N-(2-(3-nitrobenzylamino)ethoxy)ethyl-4-aminopyrimidine

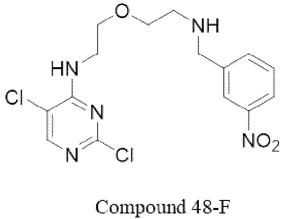

A reaction mixture of **compound 48-D** 2-(2-aminoethoxy)ethyl-2,5-dichloropyrimidin-4-amine trifluoroacetate (350 mg, 0.96 mmol), potassium acetate (470 mg, 4.79 mmol) and **compound 48-E** 3-nitrobenzaldehyde (145 mg, 0.96 mmol) in 1,2-dichloroethane (3.0 mL) was stirred at 25°C for 12 hours of reaction. The reaction was monitored by TLC to be completed. The reaction mixture was added with sodium cyanoborohydride (90 mg, 1.44 mmol) in batches and further stirred at 25°C for 3.5 hours. The reaction was monitored by LCMS to be completed and the target mass spectrum was detected. The solvent was evaporated by rotary evaporation, and the crude product **compound 48-F** 2,5-dichloro-N-(2-(3-nitrobenzylamino)ethoxy)ethyl-4-aminopyrimidine (369 mg, theoretical yield of crude product) was directly subjected to the next step of reaction.

LC_MS: (ES⁺): 385.9 [M+H] ⁺.

### Step 4: Compound 48-G tert-butyl 2-(2-(2,5-dichloropyrimidin-4-yl)aminoethoxy)ethyl(3-nitrobenzyl)carbamate

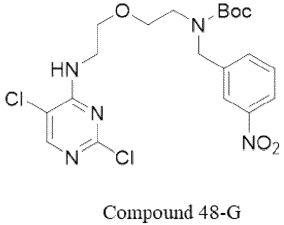

A saturated sodium bicarbonate solution (6 mL) was added to a suspension of crude product **compound 48-F** 2,5-dichloro-N-(2-(3-nitrobenzylamino)ethoxy)ethyl-4-aminopyrimidine (369 mg, 0.96 mmol) in tetrahydrofuran (6 mL).The reaction mixture was added with di-tert-butyl dicarbonate (251 mg, 1.15 mmol) under stirring, and stirred overnight at room temperature. The reaction was monitored by TLC to be completed. The reaction solution was concentrated, added with water (30 mL) and extracted with ethyl acetate (20 mL x 2). The organic phase was washed with saturated brine (30 mL), dried over anhydrous sodium sulfate, and filtered. The filtrate was concentrated to obtain a crude product, which was then eluted by silica gel column chromatography (using petroleum ether containing 15%-35% ethyl acetate) and concentrated to obtain a solid compound, which was further separated and purified (using 10%-80% acetonitrile solution in water) to obtain a light yellow solid product **compound 48-G** tert-butyl 2-(2-(2,5-dichloropyrimidin-4-yl)aminoethoxy)ethyl(3-nitrobenzyl)carbamate (200 mg).

LC_MS: (ES⁺): 486.1 [M+H] ⁺.

### Step 5: Compound 48-H tert-butyl (3-aminobenzyl)(2-(2,5-dichloropyrimidin-4-yl(amino)ethoxy)ethylcarbamate

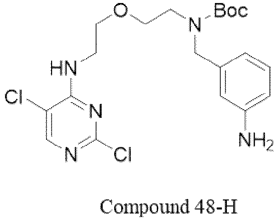

The synthesis of this step referred to the synthesis process of the fourth step of **compound 2 in Example 1.**

LC_MS: (ES⁺): 458.4 [M+2+H] ⁺.

### Step 6: Compound 48-I tert-butyl 1⁵-chloro-8-oxa-2,5,11-triaza-1(2,4)-pyrimidina-3(1,3)-benzenacycloundecaphan-5-carboxylate

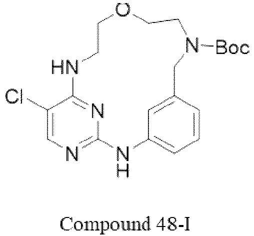

The synthesis of this step referred to the synthesis process of the eighth step of **compound 35 in Example 25.**

LC_MS: (ES⁺): 420.1 [M+H] ⁺.

### Step 7: Compound 48 1⁵-chloro-8-oxa-2,5,11-triaza-1(2,4)-pyrimidina-3(1,3)-benzenacycloundecaphane

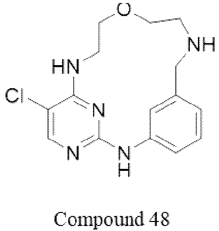

The synthesis of this step referred to the synthesis process of the sixth step of **compound 25 in Example 15.**

LC_MS: (ES⁺): 320.1 [M+H] ⁺.

¹H NMR (400 MHz, CDCl₃) δ: 8.42 (s, 1H), 7.91 (s, 1H), 7.55 (d, *J* = 8.0 Hz, 1H), 7.24 (t, *J* = 8.0 Hz, 1H), 7.13 (s, 1H), 6.90 (d, *J* = 8.0 Hz, 1H), 6.76 (d, *J* = 8.0 Hz, 1H), 5.54 (s, 1H), 3.97 (s, 2H), 3.73 (s, 6H), 2.84 (t, *J* = 4.0 Hz, 2H).

### Example 38: Synthesis route of compound 49:

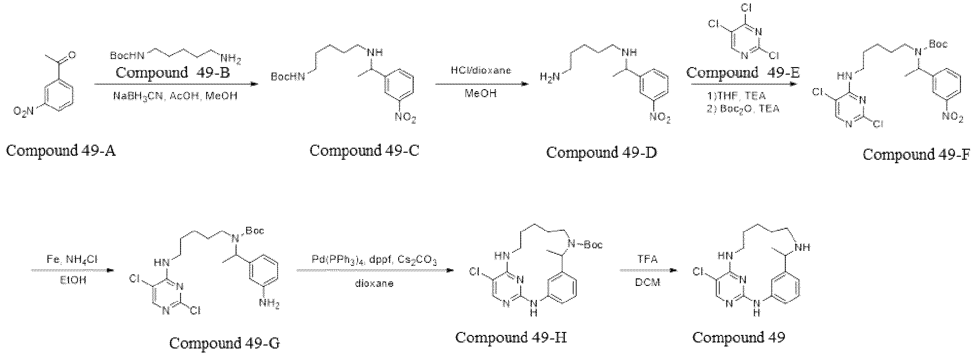

### Step 1: Compound 49-C tert-butyl (5-(1-(3-nitrophenyl)ethyl)amino)pentyl)carbamate

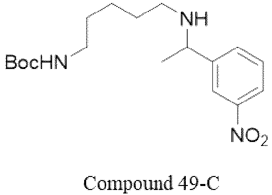

A solution of **compound 49-A** tert-butyl (5-aminopentyl)carbamate (500 mg, 2.47 mmol), compound 49-B 1-(3-nitrophenyl)ethanone (408 mg, 2.47 mmol), and acetic acid (300 mg, 4.94 mmol) in methanol (5 ml) was stirred at room temperature for 1 hour. Then the reaction solution was added with sodium cyanoborohydride (311 mg, 4.94 mmol) and stirred at 50°C for 16 hours. The reaction was monitored by TLC to be completed. The reaction solution was concentrated under reduced pressure. The residue was distributed between ethyl acetate (20 ml) and saturated sodium bicarbonate solution (20 ml). The organic layer was collected, washed with saturated brine (20 ml), dried over anhydrous sodium sulfate, and concentrated under reduced pressure. The obtained crude product was separated and purified by silica gel column chromatography (eluted with a dichloromethane solution containing 5% methanol) to obtain a yellow oily **compound 49-C** tert-butyl (5-(1-(3-nitrophenyl)ethyl)amino)pentyl)carbamate (264 mg, 30%).

LC_MS: (ES⁺): m/z 352.50 [M+H] ⁺.

¹H NMR (400 MHz, CDCl₃): δ 8.20 (t, *J* = 1.6 Hz, 1H), 8.11-8.08 (m, 1H), 4.52 (s, 1H), 3.90-3.85 (m, 1H), 3.12-3.07 (m, 2H), 2.55-2.34 (m, 2H), 1.48-1.43 (m, 13H), 1.37 (d, *J* = 6.4 Hz, 3H), 1.34-1.30(m, 2H).

### Step 2: Compound 49-D N¹-(1-(3-nitrophenyl)ethyl)pentyl-1,5-diamine

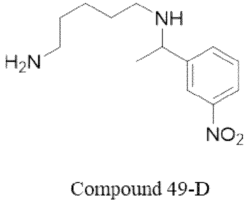

A solution of **compound 49-C** tert-butyl (5-(1-(3-nitrophenyl)ethyl)amino)pentyl)carbamate (260 mg) and hydrochloric acid-dioxane (4N, 2 ml) in methanol (2 ml) was stirred at room temperature for 2 hours. The reaction was monitored by TLC to be completed. The reaction solution was concentrated, and the residue was adjusted to pH=7-8 with saturated sodium bicarbonate solution and extracted with dichloromethane containing 10% methanol (10 ml x 3). The organic layers were combined, washed with saturated brine (20 ml), dried over anhydrous sodium sulfate, and concentrated under reduced pressure to obtain a light yellow oily **compound 49-D** N¹-(1-(3-nitrophenyl)ethyl)pentyl-1,5-diamine (180 mg, 96%).

LC_MS: (ES⁺): m/z 252.10 [M+H] ⁺.

### Step 3: Compound 49-F (tert-butyl (5)-(2,5-dichloropyrimidin-4-ylamino)pentyl)(1-(3-nitrophenyl)ethyl)carbamate

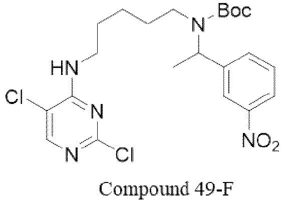

Triethylamine (84 mg, 0.83 mmol) was added to a solution of **compound 49-E** 2,4,5-trichloropyrimidine (138 mg, 0.75 mmol) in anhydrous tetrahydrofuran (5 ml) at -40°C, and then added with **compound 49-D** N¹-(1-(3-nitrophenyl)ethyl)pentyl-1,5-diamine (180 mg, 0.72 mmol) dropwise. The reaction solution was stirred at -40°C under nitrogen atmosphere for 3 hours. After the reaction was completed, the reaction mixture was heated to room temperature, added with triethylamine (84 mg, 0.83 mmol) and di-tert-butyl dicarbonate (197 mg, 0.905 mmol), and stirred at 50°C overnight. The reaction was monitored by TLC to be completed. The reaction mixture was distributed between ethyl acetate (20 ml × 2) and water (20 ml). The organic layer was collected, washed with saturated brine (20 ml), dried over anhydrous sodium sulfate, and concentrated under reduced pressure. The obtained crude product was separated and purified by silica gel column chromatography (eluted with a petroleum ether solution containing 10-20% ethyl acetate) to obtain a yellow oily **compound 49-F** (tert-butyl (5)-(2,5-dichloropyrimidin-4-ylamino)pentyl)(1-(3-nitrophenyl)ethyl)carbamate (350 mg, 98%).

LC_MS: (ES⁺): m/z 498.35 [M+H] ⁺.

¹H NMR (400 MHz, CDCl₃): δ 8.15-8.12 (m, 2H), 8.00 (s, 1H), 7.63 (d, *J* = 4.0 Hz, 1H), 7.51 (t, *J* = 8.0 Hz, 1H), 5.46 (s, 1H), 3.50-3.45 (m, 2H), 3.13-2.96 (m, 2H), 1.62 (d, *J* = 3.6 Hz, 2H), 1.54-1.52 (m, 2H), 1.44 (s, 9H), 1.30-1.25 (m, 4H).

### Step 4: Compound 49-G (tert-butyl (1)-3-aminophenylethyl)(5-((2,5-dichloropyrimidin-4-yl)amino)pentyl)carbamate

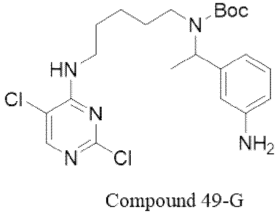

The synthesis of this step referred to the synthesis process of the fourth step of **compound 2 in Example 1.**

LC_MS: (ES⁺): m/z 468.20[M+H] ⁺.

¹H NMR (400 MHz, CDCl3): δ 8.00 (s, 1H), 7.10 (t, *J* = 8.0 Hz, 1H), 6.73-6.57 (m, 3H), 5.53-5.46 (m, 1H), 3.66 (brs, 2H), 3.47-3.38 (m, 2H), 3.10-2.81 (m, 2H), 1.53-1.41 (m, 16H), 1.23-1.14 (m, 2H).

### Step 5: Compound 49-H tert-butyl 1⁵-chloro-4-methyl-2,5,11-triaza-1(2,4)-pyrimidina-3(1,3)-benzenacycloundecaphan-5-carboxylat e

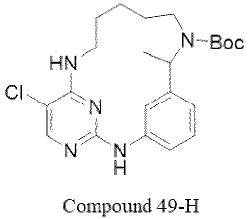

The synthesis of this step referred to the synthesis process of the eighth step of **compound 35 in Example 25.**

LC_MS: (ES⁺): m/z 432.25 [M+H] ⁺.

¹H NMR (400 MHz, DMSO-d6): δ 9.26 (s, 1H), 8.24 (s, 1H), 7.91 (s, 1H), 7.34 (t, J = 6.0 Hz, 1H), 7.21 (t, J = 7.6 Hz, 1H), 7.01 (d, J = 8.0 Hz, 1H), 6.82 (d, J = 7.2 Hz, 1H), 5.44-5.33 (m, 1H), 3.81-3.73 (m, 1H), 3.14-3.00 (m, 2H), 2.94-2.62 (m, 1H), 1.73-1.62 (m, 2H), 1.48-1.35 (m, 14H), 1.33-1.28 (m, 2H).

### Step 6: Compound 49 1⁵-chloro-4-methyl-2,5,11-triaza-1(2,4)-pyrimidina-3(1,3)-benzenacycloundecaphane

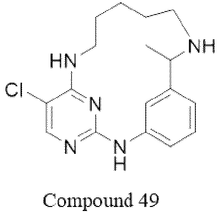

The synthesis of this step referred to the synthesis process of the sixth step of **compound 25 in Example 15.**

LC_MS: (ES⁺): m/z 332.20 [M+H] ⁺.

¹H NMR (400 MHz, DMSO-d6): δ 9.29 (s, 1H), 8.20 (s, 1H), 7.91 (s, 1H), 7.38-7.29 (m, 2H), 7.11-7.05 (m, 2H), 4.31-4.30 (m, 1H), 3.77-3.71 (m, 1H), 3.10-3.03 (m, 1H), 2.76-2.54 (m, 2H), 1.89-1.49 (m, 7H), 1.38-1.31 (m, 2H).

Biological activity test examples:
Biological test example-1: Experiment of inhibition on CDK9 kinase activity:

Compounds were tested at a concentration of 10 nM with detection in duplicate wells, or tested at 10 concentrations starting from 10 µM with three-fold serial dilutions, with detection in duplicate wells.

The inhibitory effect of the compounds on the activity of CDK9 protein kinase was detected by the caliper mobility shift assay method. 250 nL of the compound at 100-fold final concentration was transferred to a target 384-well-plate with a dispenser Echo 550. A kinase solution at 2.5-fold final concentration was prepared using 1× kinase buffer. 10 µL of the kinase solution at 2.5-fold final concentration was added to the compound well and positive control well respectively. 10 µL of 1× kinase buffer was added to the negative control well. The 384-well plate was centrifuged at 1000 rpm for 30 seconds and incubated at room temperature for 10 minutes. A mixed solution of ATP and kinase substrate at 5/3-fold final concentration was prepared with 1× kinase buffer, and to the 384-well plate was added with 15 µL per well of the mixed solution of ATP and substrate at 5/3-fold final concentration to start the reaction. The plate was centrifuged at 1000 rpm for 30 seconds, shaken to mix evenly, and incubated at room temperature for a corresponding time. 30 µL of detection stopping solution was added to terminate the kinase reaction. The 384-well plate was centrifuged at 1000 rpm for 30 seconds and shaken to mix evenly. The conversion rate was read with Caliper EZ Reader. The percent conversion inhibition rate = (% positive control conversion rate mean - % sample conversion rate / (% positive control conversion rate mean - % negative control conversion rate mean). Among them: negative control wells represent the conversion rate readings of kinase buffer background wells; positive control wells represent the conversion rate readings of wells without inhibition by compound. With the log value of concentration as the x-axis and the percentage inhibition rate as the y-axis, a dose-response curve was fitted using the log (inhibitor) vs. response -Variable slope of the analysis software GraphPad Prism 5 to obtain the IC50 value of each compound on enzyme activity. Calculation formula: y=Bottom + (Top-Bottom)/(1+10^((LogIC50-x) × hillslope)), x: logarithm of inhibitor concentration; y: % inhibition rate.

The following Table 1 provides the inhibition rates of some compounds at 10 nM on CDK9 kinase activity:

| (Inhibition rate range: A>50%; B: 25-49%) | |
|---|---|
| Compound | CDK9 |
| 5 | B |
| 8 | A |
| 10 | B |
| 12 | A |
| 15 | B |
| 18 | A |

Table 2 below provides the IC₅₀ ranges of some compounds in the CDK9 kinase activity assay:
(IC₅₀ range: A<0.1 µM; B: 0.1-0.5 µM; C: 0.5-1 µM):

| Compound | CDK9 |
|---|---|
| 5 | A |
| 6 | B |
| 8 | A |
| 10 | A |
| 12 | A |
| 15 | A |
| 18 | A |
| 21 | B |
| 22 | A |
| 24 | A |
| 25 | A |
| 27 | A |
| 28 | A |
| 29 | A |
| 30 | A |
| 31 | A |
| 32 | A |
| 33 | C |
| 34 | A |
| 35 | A |
| 36 | A |
| 37 | A |
| 38 | A |
| 39 | A |
| 40 | A |
| 41 | A |
| 43 | C |
| 44 | C |
| 45 | B |
| 46 | A |
| 47 | B |

According to the description of the above experiment on kinase activity inhibition, some compounds were tested for their selective inhibition rate against the kinases of CDK7, CDK9, JAK1, JAK2, JAK3, ITK, RET, TYK2, and LRRK2 at 10 nM. The results are shown in Table 3 below: (Inhibition rate range: A>50%; B: 25-49%; C: <25%):

| Compound | JAK1 | JAK2 | JAK3 | CDK7 | CDK9 | TYK2 | ITK | RET | LRRK2 |
|---|---|---|---|---|---|---|---|---|---|
| 8 | C | B | C | C | A | C | C | C | C |
| 12 | C | C | | | A | | | | C |
| 15 | C | C | | | B | | | | C |
| 18 | C | C | | | A | | | | |

### Biological test example-2:

The selectivity of the test compounds against CDK kinases was detected. The compounds were tested at a concentration of 0.1 µM, and the inhibition rate of CDK kinases by the compounds was detected in duplicate wells, mainly using HTRF and ADP-Glo detection methods.

The HTRF detection method was conducted as follows: 2-fold ATP/substrate solution and 2-fold kinase/metal ion solution were prepared respectively with a kinase reaction buffer, and 25 nL of diluted compound solution was transferred to a 384-well detection plate with Echo 655. After centrifugation, the plate was added with 2.5 µL of 2-fold kinase/metal ion solution and incubated at 25°C for 10 minutes. After 10 minutes, 2.5 µL of 2-fold substrate/ATP solution was added to the 384-well detection plate and incubated at 25°C for 60 minutes. 2 kinase detection reagent (XL665 and antibody) was prepared with detection buffer, and 5 µL of kinase detection reagent was added to the 384-well detection plate and incubated at 25°C for 60 minutes. The fluorescence signals at 620 nm (Cryptate) and 665 nm (XL665) were read by the microplate reader, and the ratio and the inhibition rate of kinase activity by the compound were calculated.

The ADP-Glo detection method was conducted as follows. 2-fold ATP/substrate solution and 2-fold kinase/metal ion solution were prepared respectively with a kinase reaction buffer. 20 nL of diluted compound solution was transferred to a 384-well detection plate by Echo 655. After centrifuged, the 384-well detection plate was added with 2 µL of 2 × kinase/metal ion solution, and incubated at 25°C for 10 minutes. After 10 minutes, the 384-well detection plate was added with 2 µL of 2 × substrate/ATP solution and incubated at 25°C for 60 minutes. 4 µL of ADP-Glo reagent was added to each well and incubated at room temperature for 40 minutes. 10 µL of ADP detection reagent was added to each well and incubated at room temperature for 40 minutes. The chemiluminescent signal was detected with a microplate reader and the inhibition rate of kinase activity was calculated.

**Table 4 below provides the inhibition rate of CDK kinases activity by compound 8 at 0.1 µM:**

| (Range: A>95%; B: 75-95%; C: 50-75%; D: <50%) | |
|---|---|
| Protein kinase | Compound 8 |
| CDK1/CycA2 | D |
| CDK1/CycB1 | D |
| CDK1/CycE1 | D |
| CDK12/CyclinK | C |
| CDK13/CyclinK | C |
| CDK16/CyclinY | C |
| CDK18/CyclinY | D |
| CDK19/CycC | D |
| CDK2/CyCA2 | D |
| CDK2/CycE1 | C |
| CDK3/CycE1 | C |
| CDK4/cycD1 | D |
| CDK4/CycD3 | C |
| CDK5/p25NCK | C |
| CDK5/p35NCK | D |
| CDK6/CycD 1 | D |
| CDK6/CycD3 | D |
| CDK7/CycH/MAT1 | C |
| CDK9/CycT1 | A |

### Biological test example-3:

In the CDK₉ cell activity assay, the cell line MV-4-11 was cultured in IMDM culture medium containing 10% fetal bovine serum (containing 100 U/mL penicillin and 0.1 g/L streptomycin); the cell line HCC1187 was cultured in RPMI-1640 culture medium containing 10% fetal bovine serum (containing 100 U/mL penicillin and 0.1 g/L streptomycin); the cell line Mia Paca-2 was cultured in DMEM containing 10% fetal bovine serum and 2.5% horse serum (containing 100 U/mL penicillin and 0.1 g/L streptomycin); and the cell line A375 was cultured in RPMI-1640 culture medium containing 10% fetal bovine serum (containing 100 U/mL penicillin and 0.1 g/L streptomycin). All cells were cultured in an incubator with saturated humidity at 37°C with 5% CO₂.

When MV-4-11 cells, HCC1187 cells, Mia Paca-2 cells, and A375 cells grew to 80%-90% confluence, they were digested with trypsin, resuspended, counted, and diluted to a certain cell density with complete medium. 100 µL of the cells was plated into each well of a 96-well plate, and the complete medium was used as a background control. The cells were cultured overnight in an incubator with saturated humidity at 37°C with 5% CO₂. The compounds were diluted with DMSO to a final concentration of 200×. 3 µL of the stock solution of the above compounds in DMSO was added to 197 µL of complete medium, and 50 µL of the compound diluted in the medium was added to the cells of each well. The cells were cultured in an incubator with saturated humidity at 37°C with 5% CO₂ for 72 hours. After 72 hours, the culture plates were placed at room temperature for equilibrium. 40 µL of CellTiter-Glo^{®} reagent was added to each well. The plate was mixed evenly on a shaker for 2 minutes to lyse the cells then incubated at room temperature for 60 minutes to stabilize the chemiluminescent signal, and the chemiluminescence was read using a PE EnVision microplate reader. The inhibition rate on cell growth was calculated as % Inh = (DMSO well - compound treatment well) / (DMSO well - background well) x 100, and fitting was performed using GraphPad Prism 5 to obtain the IC50 values against cell growth.

The IC₅₀ ranges of some compounds detected in cell activity assays are provided in Table 5 below:

| (IC₅₀ range: A < 0.25 µM; B: 0.25-1µM; C: 1-5µM; NT means not tested) | | | | |
|---|---|---|---|---|
| Compound | MV-4-11 | HCC1187 | Mia-Paca-2 | A375 |
| | IC₅₀ (nM) | | | |
| 8 | A | B | A | A |
| 10 | A | C | B | B |
| 12 | A | A | A | A |
| 18 | A | NT | NT | NT |
| 28 | A | NT | NT | NT |
| 31 | A | B | NT | NT |
| 35 | A | A | NT | NT |
| 38 | B | B | NT | NT |
| 39 | B | C | NT | NT |
| 41 | A | A | NT | NT |
| 46 | B | B | NT | NT |

## Claims

1. A compound represented by formula (I): 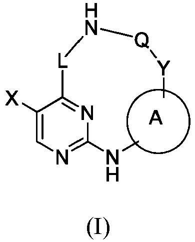
or a mesomer, a racemate, an enantiomer, a diastereoisomer or a pharmaceutically acceptable salt thereof,
wherein,
X is selected from the group consisting of H, C₁-C₃ alkyl, halogen, CF₃, carbamoyl, cyano and C₁-C₃ alkoxycarbonyl;
L is selected from the group consisting of a single bond, C₆-C₁₀ aryl, 5-12 membered heteroaryl, C₃-C₈ cycloalkyl and 3-8 membered heterocyclyl, wherein the C₆-C₁₀ aryl, 5-12 membered heteroaryl, C₃-C₈ cycloalkyl and 3-8 membered heterocyclyl are optionally further substituted by one or more R¹;
ring A is selected from the group consisting of C₆-C₁₀ aryl, 5-12 membered heteroaryl, C₃-C₈ cycloalkyl and 3-8 membered heterocyclyl, wherein the C₆-C₁₀ aryl, 5-12 membered heteroaryl, C₃-C₈ cycloalkyl and 3-8 membered heterocyclyl are optionally further substituted by one or more R²;
Y is selected from the group consisting of a single bond and -CH₂NR³-, wherein R³ is selected from the group consisting of H, C₁-C₃ alkyl, C₁-C₃ alkylacyl and Boc;
wherein at least one of L and Y is a single bond;
Q is selected from the group consisting of C₄-C₅ alkylene and C₄-C₅ alkenylene, wherein one -CH₂- unit in the above groups is optionally replaced by an O atom, a S atom or -NR³-; preferably, Q is selected from the group consisting of: 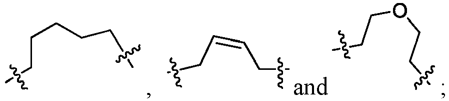 R¹ and R² are each independently selected from the group consisting of hydrogen, halogen, amino, C₁-C₃ alkylamino, cyano, oxo, hydroxyl, C₁-C₃ alkyl, C₁-C₃ alkoxy, 3-8 membered heterocyclyl and 3-8 membered heterocyclyl substituted by C₁-C₃ alkyl.

2. The compound represented by formula (I) or the mesomer, racemate, enantiomer, diastereomer or pharmaceutically acceptable salt thereof according to claim 1, wherein the compound of formula (I) has a structure represented by formula (II): 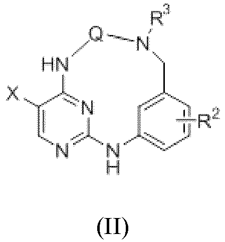 wherein,
X, Q, R² and R³ are as defined in claim 1.

3. The compound represented by formula (I) or the mesomer, racemate, enantiomer, diastereomer or pharmaceutically acceptable salt thereof according to claim 1, wherein the compound of formula (I) has a structure represented by formula (III): 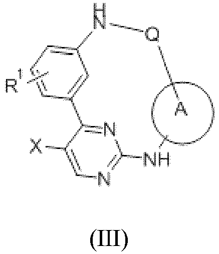 wherein,
X, Q, A and R¹ are as defined in claim 1.

4. The compound represented by formula (III) or the mesomer, racemate, enantiomer, diastereomer or pharmaceutically acceptable salt thereof according to claim 3, wherein ring A is selected from the group consisting of a benzene ring or a pyrazole ring.

5. The compound or the mesomer, racemate, enantiomer, diastereomer or pharmaceutically acceptable salt thereof according to any one of claims 1 to 4, wherein the compound is selected from the group consisting of:

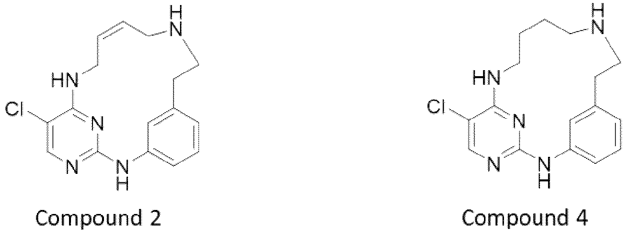

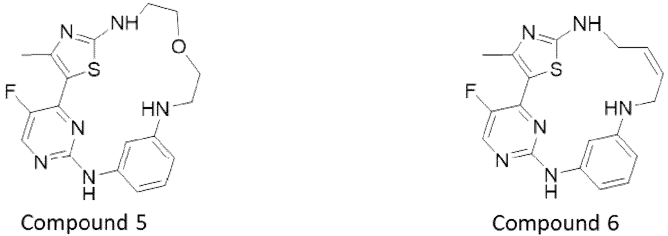

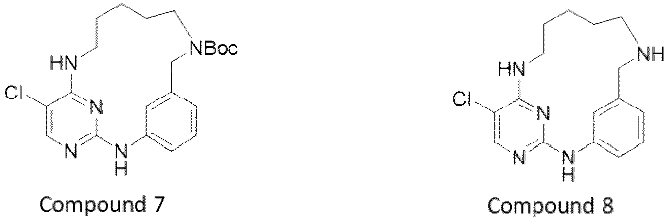

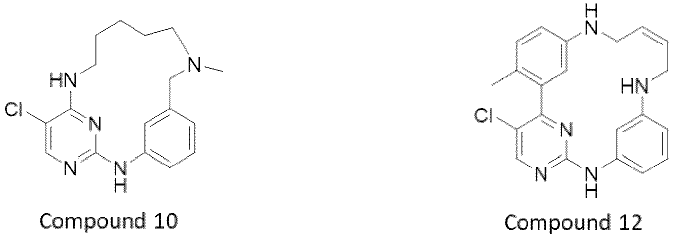

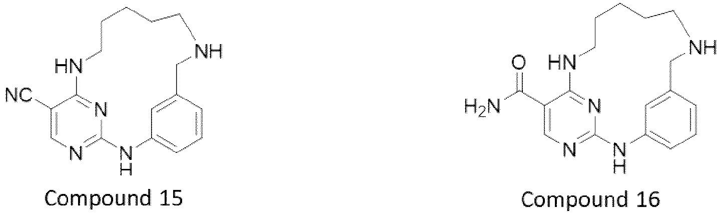

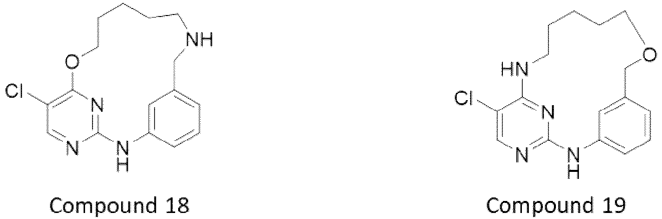

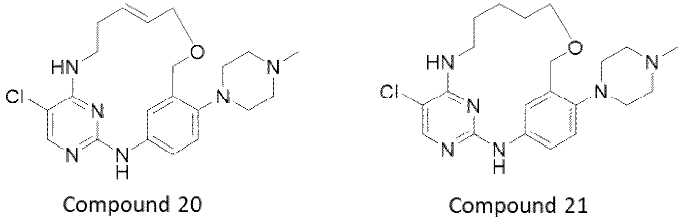

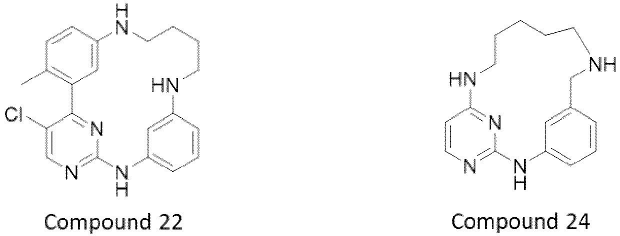

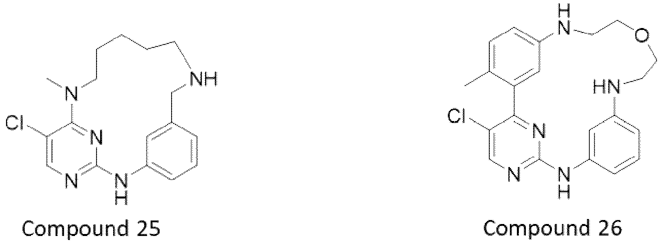

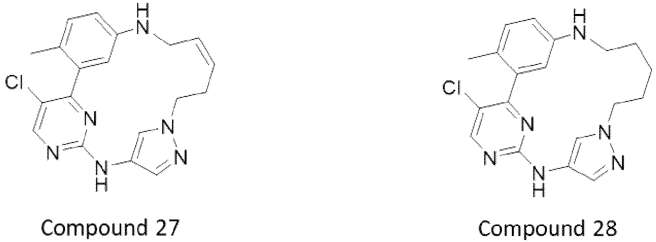

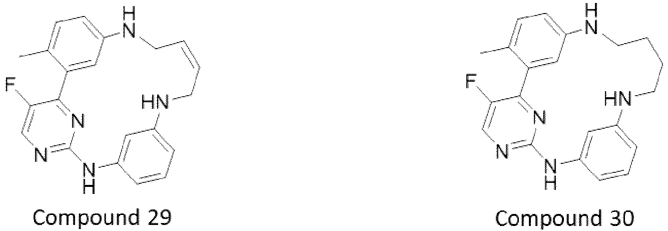

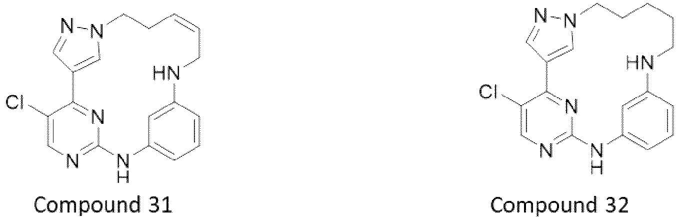

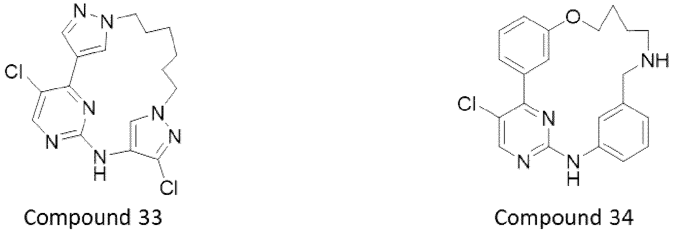

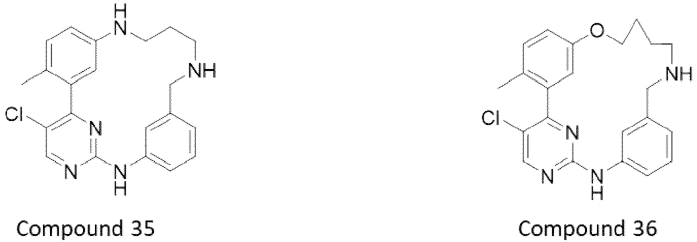

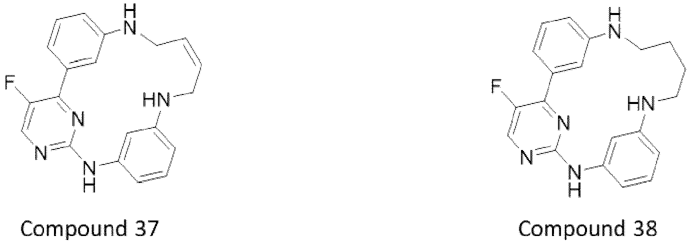

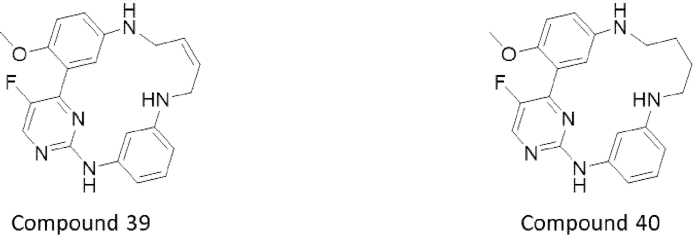

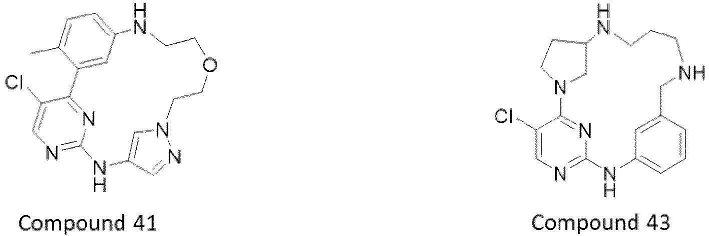

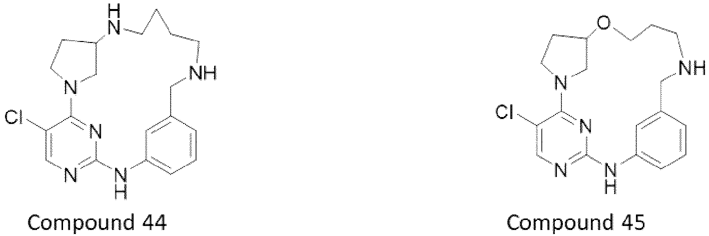

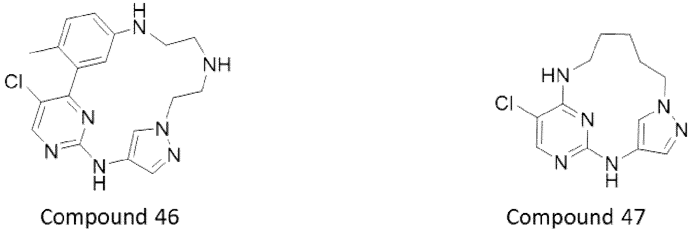

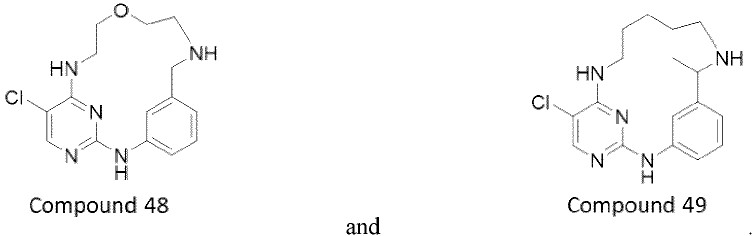

6. A pharmaceutical composition comprising the compound or the mesomer, racemate, enantiomer, diastereomer or pharmaceutically acceptable salt thereof according to any one of claims 1 to 5, and an optional pharmaceutically acceptable carrier.

7. Use of the compound or the mesomer, racemate, enantiomer, diastereomer or pharmaceutically acceptable salt thereof according to any one of claims 1 to 5 in the manufacture of a medicament for treating or preventing a disease or condition associated with CDK9 kinase activity disorder, wherein the disease or condition associated with CDK9 kinase activity disorder is preferably selected from the group consisting of a solid tumor and a hematological malignant tumor.

8. The use according to claim 7, wherein the disease or condition associated with CDK9 kinase activity disorder is selected from the group consisting of a solid tumor and cancer, preferably acute myeloid leukemia (AML), breast cancer, prostate cancer, hepatocellular carcinoma and pancreatic cancer.
